(19) Europäisches Patentamt
European Patent Office
Office européen des brevets

(11) EP 4 396 184 B1

(12) EUROPEAN PATENT SPECIFICATION

(45) Date of publication and mention of the grant of the patent:
**17.09.2025 Bulletin 2025/38**

(21) Application number: **22723730.2**

(22) Date of filing: **05.05.2022**

(51) International Patent Classification (IPC):
***C07D 413/14*** (2006.01) ***C08G 73/00*** (2006.01)

(52) Cooperative Patent Classification (CPC):
**C07D 413/14; C08G 73/0233; C08J 5/243; C08J 5/244; C08J 5/245;** C08J 2365/00

(86) International application number:
**PCT/GB2022/051146**

(87) International publication number:
**WO 2022/243655 (24.11.2022 Gazette 2022/47)**

(54) **DIFUNCTIONAL BIO-BENZOXAZINE COMPOUNDS, PREPARATION METHODS AND USES THEREOF**

DIFUNKTIONELLE BIOBENZOXAZINVERBINDUNGEN, HERSTELLUNGSVERFAHREN UND VERWENDUNGEN DAVON

COMPOSÉS DE BIO-BENZOXAZINE DIFONCTIONNELS, LEURS PROCÉDÉS DE PRÉPARATION ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.09.2021 GB 202112469**

(43) Date of publication of application:
**10.07.2024 Bulletin 2024/28**

(73) Proprietor: **Jones, Paul**
**Wrightington WN6 9PS (GB)**

(72) Inventor: **Jones, Paul**
**Wrightington WN6 9PS (GB)**

(74) Representative: **Stott, Michael John**
**Mathys & Squire**
**The Shard**
**32 London Bridge Street**
**London SE1 9SG (GB)**

(56) References cited:
**WO-A2-2019/040407**
**CN-A- 106 674 214**
**US-A1- 2019 212 650**

- **MACHADO IRLAINE ET AL: "A truly bio-based benzoxazine derived from three natural reactants obtained under environmentally friendly conditions and its polymer properties", GREEN CHEMISTRY, vol. 23, no. 11, 9 June 2021 (2021-06-09), GB, pages 4051 - 4064, XP055937014, ISSN: 1463-9262, DOI: 10.1039/D1GC00951F**
- **ANDRE ARNEBOLD ET AL: "Resorcinol-based benzoxazine with low polymerization temperature", JOURNAL OF POLYMER SCIENCE PART A: POLYMER CHEMISTRY, vol. 52, no. 12, 29 March 2014 (2014-03-29), US, pages 1693 - 1699, XP055251219, ISSN: 0887-624X, DOI: 10.1002/pola.27169**
- **DE OLIVEIRA DAVI RABELO ET AL: "Synthesis and Polymerization of Naphthoxazines Containing Furan Groups: An Approach to Novel Biobased and Flame-Resistant Thermosets", INTERNATIONAL JOURNAL OF POLYMER SCIENCE, vol. 2018, 25 November 2018 (2018-11-25), pages 1 - 13, XP055927512, ISSN: 1687-9422, Retrieved from the Internet <URL:https://downloads.hindawi.com/journals/ijps/2018/4201681.pdf> DOI: 10.1155/2018/4201681**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 396 184 B1

## Description

The invention relates to a class of benzoxazine compounds useful as a curative and having a high degree of biological based carbon content. In particular the invention is directed to a class of difunctional benzoxazine compounds comprising two benzoxazine rings, each di-substituted with furfuryl groups, a resin curative composition comprising the benzoxazine compound, processes for preparing the benzoxazines and uses thereof.

## BACKGROUND OF THE INVENTION

A benzoxazine is chemical compound comprising a bicyclic ring system containing an oxygen atom and a nitrogen atom within a heterocyclic oxazine ring that is directly fused to a benzene ring. There are several isomers of benzoxazine with the nitrogen and oxygen at differing positions that may be generically referred to as "benzoxazines", in addition to compounds having differing levels of saturation in the oxazine ring that may also go by the name "benzoxazine". In this application, the term "benzoxazine" refers to 3,4-dihydro-2*H*-1,3-benzoxazine, which is the benzoxazine monomer most useful in the production of benzoxazine based polymers, the structure of which is shown below.

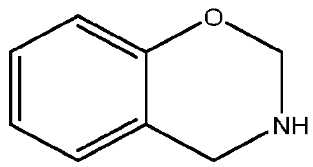

3,4-dihydro-2*H*-1,3-benzoxazine structure

Benzoxazines are capable of undergoing ring opening on heating without the need of catalysts and without emitting volatiles, leading to self-polymerisation, which is useful for both providing thermosets and also in curing to reinforce other materials. The benzoxazine polymers formed therefrom are the products of interest for providing suitable molecular weight structures that confer desirable protective properties once fully cured.

The synthesis of benzoxazine dates back over 60 years although the low molecular weight species at that time could not offer the performance characteristics now attributed to, and associated with, the polymeric benzoxazines that form the basis of the coating and matrix systems employed today. Commercially available benzoxazines are generally provided by reaction of i) a phenolic compound, which may be phenol, bisphenol, or a substituted derivative thereof, ii) formaldehyde which comes in different concentrations as an aqueous solution of the gas or in polymerized form as paraformaldehyde, and iii) a primary amine which contains one or multiple primary amine groupss, but may contain additional secondary or tertiary amine groups and/or other functional groups.

The process to synthesise the benzoxazine structure is a Mannich reaction between the primary amine and the aldehyde liberating water to form an iminium ion, followed by cyclisation with the phenolic compound and a second equivalent of aldehyde to form the oxazine ring. The Mannich reaction to form a benzoxazine ring, followed by self-polymerisation, is shown in Scheme 1 below.

Scheme 1: Mannich Synthesis and Self-Polymerisation of Generic Benzoxazine

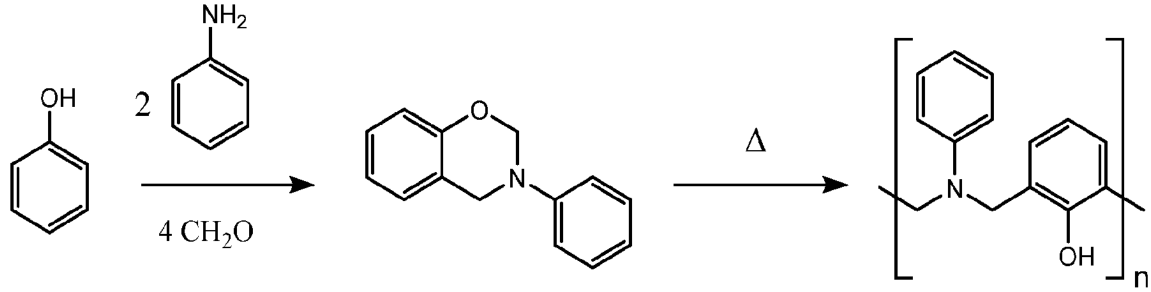


The most desirable benzoxazine polymers for coating and matrix applications are derived from difunctional benzoxazines. The resultant products have repeating units of a higher molecular weight and form polymers with a higher degree of cross-linkage. A difunctional benzoxazine may also comprise only one benzene ring that is fused to two separate oxazine rings, providing two benzoxazine moieties on a shared benzene ring.

For this reason, bisphenol A is a well-known choice of phenolic compound for this purpose as it comprises two

phenol groups capable of transformation into benzoxazines. Cardarez R111 (RTM), depicted below, is a known bisphenol A based benzoxazine useful for providing polymers with a high glass transition temperature (Tg), low water absorption, excellent physical electrical performance, along with excellent fire resistance properties. Like other conventional benzoxazines, Cardarez R111 (RTM) forms a solid resin when prepared.

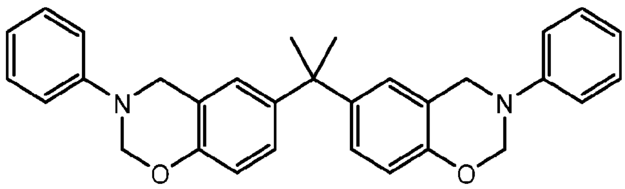

Cardarez R111 (RTM)

However, a disadvantage of conventional benzoxazines, is that the formaldehyde from which they are prepared is a highly toxic and carcinogenic chemical that is synthesised by energy intensive industrial processes. Furthermore, as mentioned above, benzoxazines formed by these methods tend to be solid resins. The process temperature and the melting point of the product also results in increased viscosity and subsequent difficulty in management or the process. In particular, increased viscosity can also generate problems when processing as a solvent free matrix or coating system. Furthermore, conventional benzoxazine resins often suffer from slow gel times, reducing the efficiency of processes such as the formation of matrixes or coatings.

The present invention is based on the surprising discovery of a new class of difunctional benzoxazine compounds that are liquids under standard conditions (e.g. at 25 °C and 100 KPa), but provide thermosets with comparable properties, such as viscosity and Tg, whilst also providing greatly increased bio-carbon content and obviating the need for synthesis from formaldehyde. Resin compositions formed from the new class of difunctional benzoxazine compounds of the present invention also present superior gel times, converting to a solid material more rapidly than conventional benzoxazine resins.

The bio-carbon content of the benzoxazines of the present invention is greatly increased by replacing the formaldehyde precursor with furfuraldehyde, also known as furfural. Furfural is an inexpensive, renewable and naturally occurring feedstock derived from the dehydration of sugars, and occurs in a variety of agricultural by-products, including corncobs, oats, wheat bran, and sawdust.

Furfural has been used in the synthesis of aryloxazines in the pharmaceutical industry, in particular, napthoxazines have been investigated for potential medicinal properties. See M. S. Al Ajely and A M Noori, Synthesis of New Oxazin Compounds Derived from Furfural. Chalcons and Schiff base, LOJ pharmacol., 2019, 1(3), 66-71. and S. A. Ozturkcan and T Zuhal, Synthesis and Charecterizations of New 1,3-Oxazine Derivatives, J. Chem. Soc. Pak., 2011, 33(6), 939-944.

D. R. de Oliveira, et al, Synthesis and Polymerization of Naphthoxazines Containing Furan Groups: An Approach to Novel Biobased and Flame-Resistant Thermosets, Int. J. Polym. Sci., 2018, 2018, 1-13, describe the use of solid furfuryl substituted napthoxazines, prepared from furfurylamine or furaldehyde precursors, as monomers for the production of polymeric materials. However, the polymer repeating unit of this disclosure is formed from a single napthoxazine ring.

WO 2019/040407 describes difunctional benzoxazine compounds for use as curatives for the preparation of polymers and methods of polymerizing the benzoxazines. These renewable benzoxazine monomers and polymers utilise the variety of building blocks found in renewable plant biomass. The benzoxazines of WO 2019/040407 are prepared using formaldehyde.

CN 106674214 describes difunctional benzoxazine resin and applications thereof as composite resin matrix. These renewable benzoxazine monomers and polymers utilise the variety of building blocks found in renewable plant biomass. The benzoxazines of CN 106674214 are prepared using formaldehyde.

The difunctional benzoxazine of the present invention is useful in forming a polymer comprising repeating units formed from a difunctional benzoxazine to provide a high degree of cross-linkage and a high bio-carbon based content. Surprisingly, the new class of furfural derived difunctional benzoxazines of the present invention have also been found to typically be in liquid form at standard conditions (e.g. 25 °C and 100 kPa) , which provides numerous advantages in handling, application and use in resin compositions over conventional solid benzoxazine resins. For example, liquid benzoxazines may be combined with a curing catalyst at low temperature (below which reaction of the benzoxazine can be expected) without the risk of unwanted or premature self-polymerisation. In contrast, a solid benzoxazine compound such as Cardarez R111 (RTM) must be melted before mixing with a catalyst is possible, thus risking undesired self-polymerisation due to the high temperature required for melting.

## SUMMARY OF THE INVENTION

In one aspect, the present invention provides a compound of formula (I):

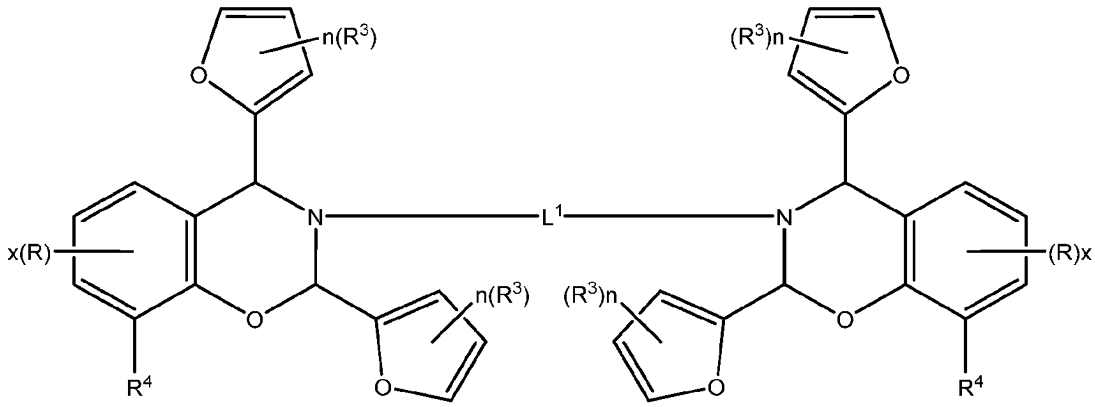

(I)

wherein each R group is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{20}$ cycloalkyl, $C_1$ to $C_{20}$ alkyl-$C_3$ to $C_{10}$ heterocycloalkyl, $C_1$ to $C_{20}$ alkyloxy, $C_1$ to $C_{20}$ alkylamino, -OH, $-OR^1$, $-NH_2$, $-NHR^1$, $-NR^1_2$, -C(O)OH, $-C(O)OR^1$, $-C(O)NH_2$, - $C(O)NHR^1$, $-C(O)NR^1_2$, -O(CO)H, $-O(CO)R^1$, -NH(CO)H, $-NH(CO)R^1$, $-NR^1(CO)H$, - $NR^1(CO)R^1$, -SH, $-SR^1$, $-SO_2H$, $-SO_2R^1$, $-SO_3R^1$, $-SO_3H$, $-SiR^1_3$, $-NO_2$, -CN, -F, -Cl, -Br, - I, $C_6$ to $C_{14}$ aryl optionally substituted with one or more $R^2$ group, and $C_2$ to $C_9$ heteroaryl optionally substituted with one or more $R^2$ group;

wherein each $R^1$ is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{10}$ cycloalkyl, $C_1$ to $C_{20}$ alkyl-$C_3$ to $C_{10}$ heterocycloalkyl, $C_1$ to $C_{20}$ alkoxy, $C_1$ to $C_{20}$ alkylamino, $C_6$ to $C_{14}$ aryl, and $C_2$ to $C_9$ heteroaryl;

wherein $R^2$ is independently selected from: $C_1$ to $C_{20}$ alkyl, $C_1$ to $C_{20}$ haloalkyl, $C_3$ to $C_{20}$ cycloalkyl, $C_2$ to $C_{20}$ alkenyl, $C_2$ to $C_{20}$ alkynyl, -OH, $-NH_2$, $-OC_1$ to $C_{20}$ alkyl, $-NHC_1$ to $C_{20}$ alkyl, $-N(C_1$ to $C_{20}$ alkyl$)_2$, $-NO_2$, -CN, -F, -Cl, -Br, and -I;

wherein $R^3$ is independently selected from: $C_1$ to $C_6$ alkyl, $C_1$ to $C_6$ alkenyl, $C_1$ to $C_6$ alkoxy, $C_1$ to $C_6$ alkyloxy, -C(O)OH, -C(O)O-$C_1$ to $C_6$ alkyl, $C_1$ to $C_6$ alkyl-(O)O-H, $C_1$ to $C_6$ alkyl-(O)O-$C_1$ to $C_6$ alkyl, and -OH;

wherein $R^4$ is hydrogen;

wherein $L^1$ is a divalent hydrocarbyl linker comprising 1 to 50 carbon atoms;

wherein x is independently 0, 1, 2 or 3; and

wherein n is independently 0, 1, 2 or 3.

In another aspect, the present invention provides a compound of formula (II):

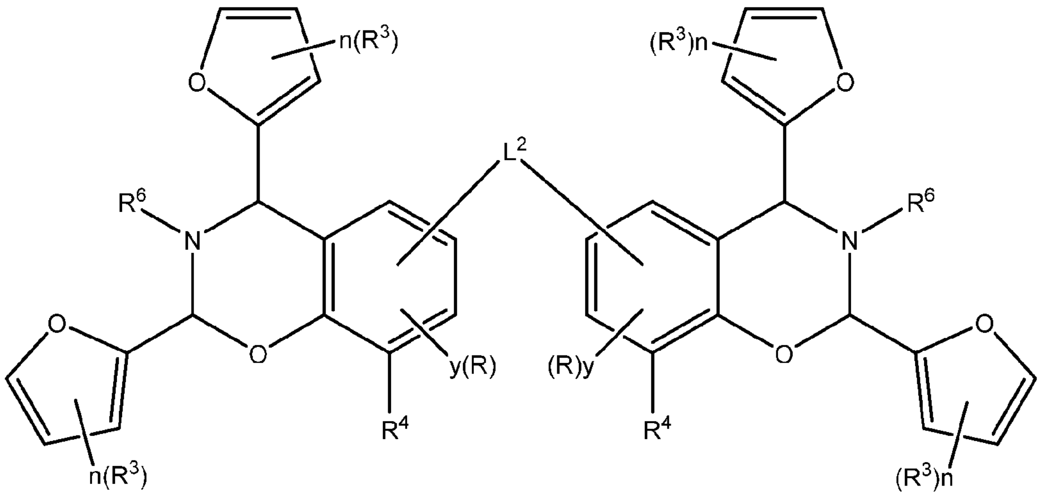

(II)

wherein each R group is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{20}$ cycloalkyl, $C_1$ to $C_{20}$ alkyl-$C_3$ to $C_{10}$ heterocycloalkyl, $C_1$ to $C_{20}$ alkyloxy, $C_1$ to $C_{20}$ alkylamino, -OH, $-OR^1$, $-NH_2$, $-NHR^1$, $-NR^1_2$, -C(O)OH, $-C(O)OR^1$, $-C(O)NH_2$, - $C(O)NHR^1$, $-C(O)NR^1_2$, -O(CO)H, $-O(CO)R^1$, -NH(CO)H, $-NH(CO)R^1$, $-NR^1(CO)H$, - $NR^1(CO)R^1$, -SH, $-SR^1$, $-SO_2H$, $-SO_2R^1$, $-SO_3R^1$, $-SO_3H$, $-SiR^1_3$, $-NO_2$, -CN, -F, -Cl, -Br, - I, $C_6$ to $C_{14}$ aryl optionally substituted with one or more $R^2$ group, and $C_2$ to $C_9$ heteroaryl optionally substituted with one or more $R^2$ group;

wherein each $R^1$ is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{10}$ cycloalkyl, $C_1$ to $C_{20}$ alkyl-$C_3$ to $C_{10}$ heterocycloalkyl, $C_1$ to $C_{20}$ alkoxy, $C_1$ to $C_{20}$ alkylamino, $C_6$ to $C_{14}$ aryl, and $C_2$ to $C_9$ heteroaryl;

wherein $R^2$ is independently selected from: $C_1$ to $C_{20}$ alkyl, $C_1$ to $C_{20}$ haloalkyl, $C_3$ to $C_{20}$ cycloalkyl, $C_2$ to $C_{20}$ alkenyl, $C_2$ to $C_{20}$ alkynyl, -OH, $-NH_2$, $-OC_1$ to $C_{20}$ alkyl, $-NHC_1$ to $C_{20}$ alkyl, $-N(C_1$ to $C_{20}$ alkyl$)_2$, $-NO_2$, -CN, -F, -Cl, -Br, and -I;

wherein $R^3$ is independently selected from: $C_1$ to $C_6$ alkyl, $C_1$ to $C_6$ alkenyl, $C_1$ to $C_6$ alkoxy, $C_1$ to $C_6$ alkyloxy, -C(O)OH, -C(O)O-$C_1$ to $C_6$ alkyl, $C_1$ to $C_6$ alkyl-(O)O-H, $C_1$ to $C_6$ alkyl-(O)O-$C_1$ to $C_6$ alkyl, and -OH;

wherein $R^4$ is hydrogen;

wherein $R^6$ is independently a monovalent group selected from -H, -OH, or a hydrocarbyl group comprising 1 to 50 carbon atoms;

wherein $L^2$ is a direct bond, a divalent group selected from: C(O), O, S, S(O), $S(O)_2$, or a hydrocarbyl linker comprising 1 to 50 carbon atoms;

wherein y is independently 0, 1, or 2; and

wherein n is independently 0, 1, 2 or 3.

In yet another aspect, the present invention provides a compound of either formula (IIIa) or formula (IIIb):

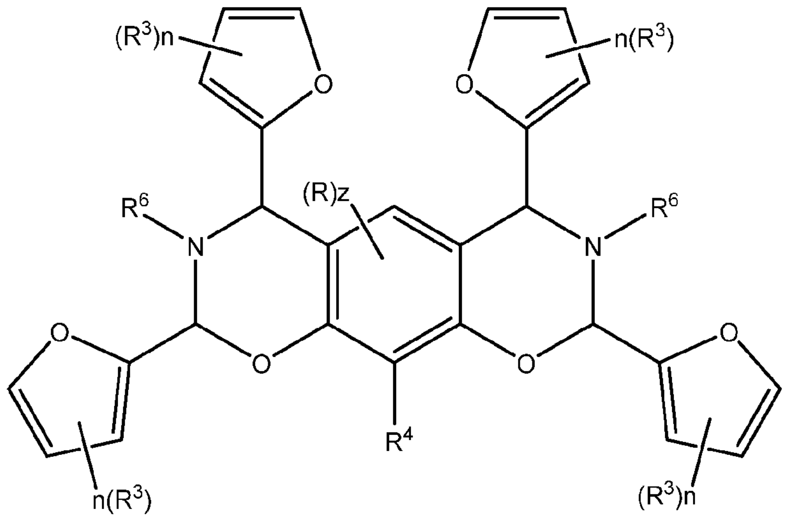

(IIIa)

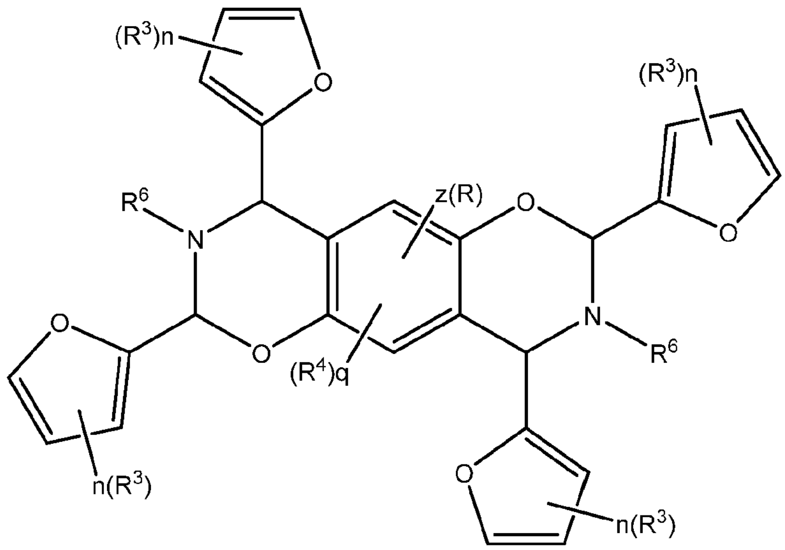

(IIIb)

wherein each R group is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{20}$ cycloalkyl, $C_1$ to $C_{20}$ alkyl-$C_3$ to $C_{10}$ heterocycloalkyl, $C_1$ to $C_{20}$ alkyloxy, $C_1$ to $C_{20}$ alkylamino, -OH, $-OR^1$, $-NH_2$, $-NHR^1$, $-NR^1_2$, -C(O)OH, $-C(O)OR^1$, $-C(O)NH_2$, - $C(O)NHR^1$, $-C(O)NR^1_2$, -O(CO)H, $-O(CO)R^1$, -NH(CO)H, $-NH(CO)R^1$, $-NR^1(CO)H$, - $NR^1(CO)R^1$, -SH, $-SR^1$, $-SO_2H$, $-SO_2R^1$, $-SO_3R^1$, $-SO_3H$, $-SiR^1_3$, $-NO_2$, -CN, -F, -Cl, -Br, - I, $C_6$ to $C_{14}$ aryl optionally substituted with one or more $R^2$ group, and $C_2$ to $C_9$ heteroaryl optionally substituted with one or more $R^2$ group;

wherein each $R^1$ is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{10}$ cycloalkyl, $C_1$ to $C_{20}$ alkyl-$C_3$ to $C_{10}$ heterocycloalkyl, $C_1$ to $C_{20}$ alkoxy, $C_1$ to $C_{20}$ alkylamino, $C_6$ to $C_{14}$ aryl, and $C_2$ to $C_9$ heteroaryl;

wherein $R^2$ is independently selected from: $C_1$ to $C_{20}$ alkyl, $C_1$ to $C_{20}$ haloalkyl, $C_3$ to $C_{20}$ cycloalkyl, $C_2$ to $C_{20}$ alkenyl, $C_2$ to $C_{20}$ alkynyl, -OH, $-NH_2$, $-OC_1$ to $C_{20}$ alkyl, $-NHC_1$ to $C_{20}$ alkyl, $-N(C_1$ to $C_{20}$ alkyl$)_2$, $-NO_2$, -CN, -F, -Cl, -Br, and -I;

wherein $R^3$ is independently selected from: $C_1$ to $C_6$ alkyl, $C_1$ to $C_6$ alkenyl, $C_1$ to $C_6$ alkoxy, $C_1$ to $C_6$ alkyloxy, -C(O)OH, -C(O)O-$C_1$ to $C_6$ alkyl, $C_1$ to $C_6$ alkyl-(O)O-H, $C_1$ to $C_6$ alkyl-(O)O-$C_1$ to $C_6$ alkyl, and -OH;

wherein $R^4$ is hydrogen;

wherein $R^6$ is independently a monovalent group selected from -H, -OH, or a hydrocarbyl group comprising 1 to 50 carbon atoms;

wherein z is 0 or 1;
wherein q = 2 - z; and
wherein n is independently 0, 1, 2 or 3.

In still another aspect, the present invention provides a process for preparing a compound of formula (I) as described herein, said method comprising:

i) providing a diamine $NH_2$-$L^1$-$NH_2$ wherein $L^1$ is as described herein;
ii) adding at least one furfuraldehyde substituted with n $R^3$ groups, and at least one phenolic compound substituted with x R groups, wherein R, $R^3$, n and x are as described herein, with the proviso that both positions ortho to the phenolic OH group are unsubstituted on the at least one phenolic compound; and
iii) forming a compound of formula (I).

In a further aspect, the present invention provides a process for preparing a compound of formula (II), (IIIa), or (IIIb) as described herein, said method comprising:

i) providing: A) a bisphenol compound comprising a linker $L^2$ and substituted with y R groups on each benzene ring of the bisphenol compound; B) a benzene-1,4-diol compound substituted with z R groups; or C) a benzene-1,3-diol optionally substituted with 1 R group at the 5-position; wherein y, z, R and $L^2$ are as described herein;
ii) contacting the compound from step i) with at least one furfuraldehyde substituted with n $R^3$ groups, and at least one primary amine substituted with one $R^6$ group, wherein n, $R^3$ and $R^6$ are as described herein; and
iii) forming a compound of formula (II), (IIIa), or (IIIb).

In a yet further aspect, the present invention comprises a resin composition comprising a benzoxazine compound of formula (I), (II), (IIIa), or (IIIb), as described herein.
In a still further aspect, the present invention provides the use of a benzoxazine compound of formula (I), (II), (IIIa), or (IIIb), or a resin composition thereof, as described herein, to reinforce a material.
In another aspect, the present invention provides a method for preparing a reinforced material, said method comprising:

a) contacting a material to be reinforced with a compound of formula (I), (II), (IIIa), or (IIIb), or a resin composition thereof; and
b) allowing said compound to self-polymerise to form a reinforced material.

In another aspect, the present invention provides a reinforced material prepared, or preparable, by the methods described herein.

## BRIEF DESCRIPTION OF THE FIGURES

Figure 1 demonstrates the viscosity vs temperature of the comparative Cardarez R111 (RTM) benzoxazine;

Figure 2 demonstrates the viscosity development over time of comparative Cardarez R111 (RTM) benzoxazine at 100 °C, 120 °C and 140 °C;

Figure 3 demonstrates the gel-time reactivity vs temperature of comparative Cardarez R111 (RTM);

Figure 4 demonstrates the gel-time reactivity vs temperature of Compound A;

Figure 5 demonstrates the Tg development of thermosets cured at 180 °C vs time for a compound A thermoset and a comparative Cardarez R111 (RTM) thermoset;

Figure 6 demonstrates the Tg development of Compound A during curing at 140 °C over 1 hour with 3%, 4% or 5% of a ferric chloride catalyst;

Figure 7 demonstrates the Compound A viscosity build vs time at 60 °C and 100 °C, with 5% ferric chloride catalyst; and

Figure 8 demonstrates the gel times of Compound A in combination with concentrations of 1, 1.5 and 2 PHR of $BCl_3$ octyl dimethylamine complex at 160 °C.

## DETAILED DESCRIPTION

Definition of terms

For the purposes of the present invention, the following terms as used herein shall, unless otherwise indicated, be understood to have the following meanings. Other terms that are not as defined below are to be understood as their normal meaning in the art.

The term "hydrocarbyl" as used herein, refers to a monovalent or divalent group, comprising hydrogen and carbon atoms, such as a major proportion (i.e., more than 50 %) of hydrogen and carbon atoms, preferably consisting exclusively of hydrogen and carbon atoms. The hydrocarbyl group may be aromatic, saturated aliphatic or unsaturated aliphatic. The hydrocarbyl group may be entirely aliphatic or a combination of aliphatic and aromatic portions. In some examples, the hydrocarbyl group includes a branched aliphatic chain which is substituted by one or more aromatic groups. Examples of hydrocarbyl groups therefore include acyclic groups, as well as groups that combine one or more acyclic portions and one or more cyclic portions, which may be selected from carbocyclic, aryl and heterocyclyl groups. The hydrocarbyl group includes monovalent groups and polyvalent groups as specified and may, for example, include one or more groups selected from alkyl, alkenyl, alkynyl, carbocyclyl (e.g. cycloalkyl or cycloalkenyl), aryl and heterocyclyl. The hydrocarbyl group may contain one or more heteroatoms, such as oxygen, nitrogen, sulphur, silicon or halogen which may be part of a functional group such as an alcohol, ether, carbonyl, ester, carboxylic acid, carbonate, amide, amine, carbamate, urea, thiol, thioether, thioester, thioacid, thioamide, silane organic halide or heterocycle, the hydrocarbyl linker may contain any combination of the above insofar as it is chemically stable. Furthermore, in some embodiments, halogens may entirely replace the hydrogen component of the hydrocarbyl group (i.e. the carbon-bonded hydrogens) to give the corresponding halo-substituted analogue.

The term "alkyl" as used herein refers to a monovalent straight- or branched-chain alkyl moiety. Unless specifically indicated otherwise, the term "alkyl" does not include optional substituents. The term "haloalkyl" as used herein refers to an alkyl group substituted with one or more halogen atoms. The term "halogen" as used herein refers to any of fluorine, chlorine, bromine, or iodine.

The term "alkyloxy" as used herein refers to an alkyl group substituted with one or more hydroxy groups. The term "alkylamino" as used herein refers to an alkyl group substituted with one or more primary, secondary, or tertiary amine groups.

The term "cycloalkyl" as used herein refers to a monovalent saturated aliphatic hydrocarbyl moiety containing at least one ring, wherein said ring has at least 3 ring carbon atoms. The cycloalkyl groups mentioned herein may optionally have alkyl groups attached thereto. Examples of cycloalkyl groups include groups that are monocyclic, polycyclic (e.g., bicyclic) or bridged ring system. Examples of cycloalkyl groups include cyclopropyl, cyclobutyl, cyclopentyl, cyclohexyl and the like. The term "heterocycloalkyl" as used herein refers to a cycloalkyl group wherein the ring contains at least one heteroatom selected from oxygen, nitrogen, and sulphur. Examples of heterocycloalkyl groups include morpholine, piperidine, piperazine and the like.

The term "alkenyl" as used herein refers to a monovalent straight- or branched-chain alkyl group containing at least one carbon-carbon double bond, of either E or Z configuration unless specified. The term "alkynyl" as used herein refers to a monovalent straight- or branched-chain alkyl group containing at least one carbon-carbon triple bond. Examples of alkenyl groups include ethenyl, 2-propenyl, 1-butenyl, 2-butenyl, 3-butenyl, 1-pentenyl, 2-pentenyl, 3-pentenyl, 1-hexenyl, 2-hexenyl, 3-hexenyl and the like.

The term "aryl" as used herein refers to an aromatic carbocyclic ring system. An example of an aryl group includes a group that is a monocyclic aromatic ring system or a polycyclic ring system containing two or more rings, at least one of which is aromatic. Examples of aryl groups include aryl groups that comprise from 1 to 6 exocyclic carbon atoms in addition to ring carbon atoms. Examples of aryl groups include aryl groups that are monovalent or polyvalent as appropriate. Examples of monovalent aryl groups include phenyl, benzyl, naphthyl, fluorenyl, azulenyl, indenyl, anthryl and the like. An example of a divalent aryl group is 1,4-phenylene.

The term "heteroaryl" as used herein refers to an aromatic heterocyclic ring system wherein said ring atoms include at least one ring carbon atom and at least one ring heteroatom selected from nitrogen, oxygen and sulphur. Examples of heteroaryl groups include heteroaryl groups that are a monocyclic ring system or a polycyclic (e.g. bicyclic) ring system, containing two or more rings, at least one of which is aromatic. Examples of heteroaryl groups include those that, in addition to ring carbon atoms, comprise from 1 to 6 exocyclic carbon atoms. Examples of heteroaryl groups include those that are monovalent or polyvalent as appropriate. Examples of heteroaryl groups include pyridyl, pyrimidyl, thiopheneyl, isoxazolyl and benzo[b]furanyl groups.

The terms "furfural", "furfuraldehyde", "furfuryl" and "furan" are herein intended to optionally include any of the

possible $R^3$ substituents defined herein, unless explicitly stated otherwise. In the present invention, furfural is the aldehyde used in the Mannich reaction to form the benzoxazine rings. The resulting benzoxazine rings are substituted with furan at the 2- and 4-positions of the oxazine ring. Nevertheless, in the present invention a substituted furfural may be used instead of, or in combination with, unsubstituted furfural. In formulae (I), (II), (IIIa), and (IIIb) the optional furfural substituents are defined by $R^3$.

The term "phenolic compound" as used herein refers to phenol, optionally substituted with one or more R groups at one or more of the 3-, 4-, and/or 5- positions relative to the phenol oxygen, wherein the R groups are as defined herein.

The term "bisphenol compound" as used herein refers to an organic compound comprising phenol moieties conjoined by a linker attached at any one of the 3-, 4-, or 5-positions, independently, relative to the phenol oxygen, wherein the linker is $L^2$ as defined herein. The phenol moieties are optionally substituted with one or more R groups at one or more of the 3-, 4-, and/or 5- positions relative to the phenol oxygen that are not occupied by $L^2$, wherein the R groups are as defined herein.

The present invention relates to a hitherto unknown class of difunctional benzoxazine compounds comprising two benzoxazine rings di-substituted with furfuryl groups. Two benzoxazine groups may be conjoined by a linker attached to the oxazine nitrogen atoms, or attached to the benzoxazine benzene rings, or the two benzoxazine groups may share a single benzene ring.

The difunctional benzoxazine compounds comprise at least one unsubstituted position on the benzene ring ortho to a benzoxazine oxygen atom, as this position must be unsubstituted to allow for self-polymerisation of the difunctional benzoxazine compound. As will be appreciated, "unsubstituted" herein refers to substitution by a hydrogen atom. The self-polymerisation takes place by thermal ring-opening polymerisation with or without catalyst or co-reactant. Whilst a wide range of Lewis acid catalysts (for example, including titanium isopropoxide, aluminium trichloride, Dichloro(methyl) aluminium, aluminium tribromide, iron tribromide, titanium tetrachloride, tin tetrachloride, yttrium trichloride, boron trifluoride (optionally in the form of boron trifluoride complexes, such as $BF_3$ etherate, $BF_3$ THF complex, or $BF_3$ amine complexes such as $BF_3$ methyl ethyl amine, $BF_3$ ethyl amine, $BF_3$ triethyl amine, or $BF_3$ dimethyl octyl amine, preferably $BF_3$ methyl ethyl amine), boron trichloride (optionally in the form of boron trichloride complexes, such as $BCl_3$ methyl ethyl amine, $BCl_3$ etherate, $BCl_3$ THF complex, or $BCl_3$ amine complexes such as $BCl_3$ ethyl amine, $BCl_3$ triethyl amine, or $BCl_3$ dimethyl octyl amine, preferably $BCl_3$ dimethyl octyl amine), zinc dichloride and trimethyl borane) may be used to catalyse self-polymerisation, it has been found that ferric chloride is particularly suitable for catalysing the self-polymerisation reaction. The result of the self-polymerisation is a high molecular weight thermoset polymer matrix with a high degree of cross-linkage. Resins comprising the difunctional benzoxazine compounds of the present invention are therefore useful precursors to poly-benzoxazine polymers. Advantageously, whilst the difunctional benzoxazine compounds of the present invention are liquids at standard conditions, it may not be necessary to include a solvent or diluent in such a resin. Nevertheless, solvents or diluents may be used to adjust the properties of the resin as is required.

The class of benzoxazines of the present invention may be synthesised by a Mannich reaction between a phenolic compound, a primary amine, and furfural. As would be appreciated, two equivalents of furfural are required for every one equivalent of phenolic compound and primary amine. As the present class of benzoxazines are difunctional, two equivalents of phenolic compound and two equivalents of primary amine must be reacted with four equivalents of furfural to provide one equivalent of difunctional benzoxazine. However, two equivalents of the phenolic compound or two equivalents of the primary amine may be comprised within a single difunctional molecule. For example, one equivalent of a difunctional phenolic compound or of a difunctional primary amine, may be reacted with two equivalents of a primary amine or phenolic compound, respectively, as well as four equivalents of furfural. The resulting benzoxazine will be difunctional as two benzoxazine rings are formed within the same single molecule. Therefore, the class of difunctional benzoxazine compounds of the present invention all share the feature of two benzoxazine rings, each substituted with two furan groups.

In one aspect, the benzoxazine compounds of the present invention have the general formula (I), shown below.

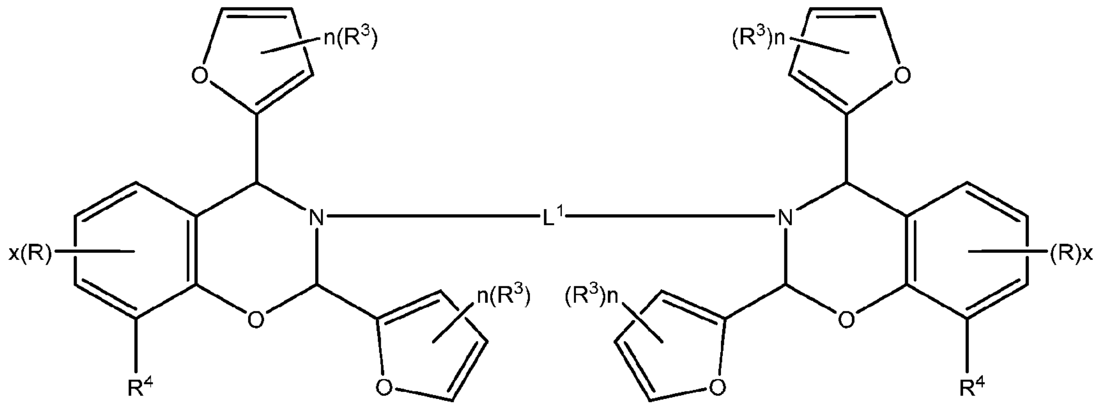

(I)

wherein each R group is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{20}$ cycloalkyl, $C_1$ to $C_{20}$ alkyl-$C_3$ to $C_{10}$ heterocycloalkyl, $C_1$ to $C_{20}$ alkyloxy, $C_1$ to $C_{20}$ alkylamino, -OH, $-OR^1$, $-NH_2$, $-NHR^1$, $-NR^1{}_2$, -C(O)OH, $-C(O)OR^1$, $-C(O)NH_2$, - $C(O)NHR^1$, $-C(O)NR^1{}_2$, -O(CO)H, $-O(CO)R^1$, -NH(CO)H, $-NH(CO)R^1$, $-NR^1(CO)H$, - $NR^1(CO)R^1$, -SH, $-SR^1$, $-SO_2H$, $-SO_2R^1$, $-SO_3R^1$, $-SO_3H$, $-SiR^1{}_3$, $-NO_2$, -CN, -F, -Cl, -Br, - I, $C_6$ to $C_{14}$ aryl optionally substituted with one or more $R^2$ group, and $C_2$ to $C_9$ heteroaryl optionally substituted with one or more $R^2$ group;

wherein each $R^1$ is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{10}$ cycloalkyl, $C_1$ to $C_{20}$ alkyl-$C_3$ to $C_{10}$ heterocycloalkyl, $C_1$ to $C_{20}$ alkoxy, $C_1$ to $C_{20}$ alkylamino, $C_6$ to $C_{14}$ aryl, and $C_2$ to $C_9$ heteroaryl;

wherein $R^2$ is independently selected from: $C_1$ to $C_{20}$ alkyl, $C_1$ to $C_{20}$ haloalkyl, $C_3$ to $C_{20}$ cycloalkyl, $C_2$ to $C_{20}$ alkenyl, $C_2$ to $C_{20}$ alkynyl, -OH, $-NH_2$, $-OC_1$ to $C_{20}$ alkyl, $-NHC_1$ to $C_{20}$ alkyl, $-N(C_1$ to $C_{20}$ alkyl$)_2$, $-NO_2$, -CN, -F, -Cl, -Br, and -I;

wherein $R^3$ is independently selected from: $C_1$ to $C_6$ alkyl, $C_1$ to $C_6$ alkenyl, $C_1$ to $C_6$ alkoxy, $C_1$ to $C_6$ alkyloxy, -C(O)OH, -C(O)O-$C_1$ to $C_6$ alkyl, $C_1$ to $C_6$ alkyl-(O)O-H, $C_1$ to $C_6$ alkyl-(O)O-$C_1$ to $C_6$ alkyl, and -OH;

wherein $R^4$ is hydrogen;

wherein $L^1$ is a divalent hydrocarbyl linker comprising 1 to 50 carbon atoms;

wherein x is independently 0, 1, 2 or 3; and

wherein n is independently 0, 1, 2 or 3.

In some embodiments one or both benzene rings of the benzoxazines are substituted with R groups at the 3-position and/or the 5-position, relative to the oxygen of the benzoxazine ring.

In some embodiments both x values ≤ 2, preferably wherein both x values ≤ 1, and preferably wherein both x values are the same, and/or wherein both benzene rings of the benzoxazines are substituted with the same R group(s), more preferably wherein both x values = 0.

Formula (I) comprises two benzoxazine moieties attached at the benzoxazine nitrogen atoms though a divalent hydrocarbyl linker group $L^1$. The difunctional benzoxazine may be derived from a difunctional primary amine, comprising the linker group L', wherein the difunctional primary amine undergoes a Mannich reaction with four equivalents of the furfural and two equivalents of a phenolic compound to form the compound of formula (I).

In some embodiments $L^1$ comprises 2 to 50 carbon atoms, 3 to 50 carbon atoms, 5 to 50 carbon atoms, 2 to 40 carbon atoms, 3 to 40 carbon atoms, 5 to 40 carbon atoms, 2 to 30 carbon atoms, 3 to 30 carbon atoms, 5 to 30 carbon atoms, 2 to 20 carbon atoms, 3 to 20 carbon atoms, or 5 to 20 carbon atoms.

Preferably, $L^1$ comprises at least one of: i) a heteroatom selected from nitrogen, oxygen, sulphur and silicon; ii) a double or triple carbon-carbon bond; or iii) an aromatic ring, heteroaromatic ring, and/or non-aromatic ring.

A list of preferred $L^1$ groups is provided in Table 1 below, along with their corresponding difunctional primary amines.

**Table 1. Exemplary list diamines useful in the present invention and their corresponding $L^1$ linkers.**

| Diamine Name | Diamine Structure | Corresponding $L^1$ Structure |
|---|---|---|
| ethylene diamine | 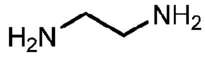 | 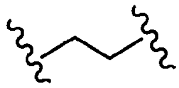 |
| diethylene triamine | 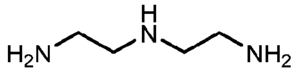 | 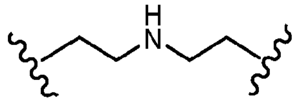 |
| triethylene tetramine | 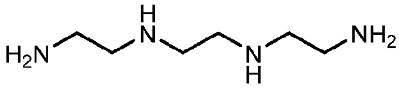 | 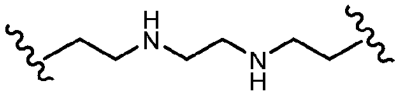 |
| hexamethylene diamine | 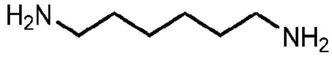 | 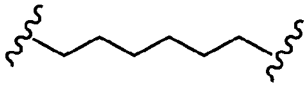 |
| 2,2,4-trimethyl hexamethylene diamine | 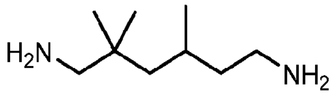 | 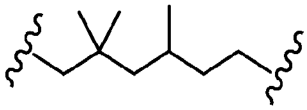 |
| 2,4,4-trimethyl hexamethylene diamine | 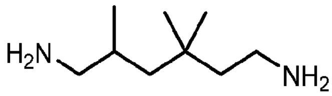 | 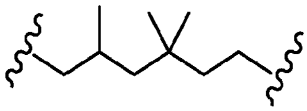 |
| Jeffamine | 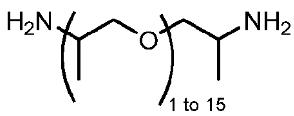 | 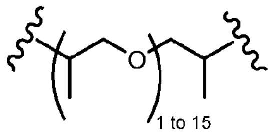 |
| metaxylylene diamine | 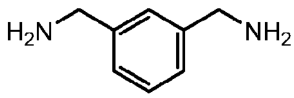 | 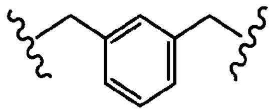 |
| 1,3-bis(aminomethyl) cyclohexane | 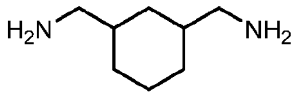 | 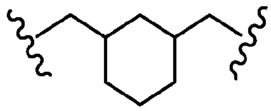 |
| benzene-1,4-diamine | 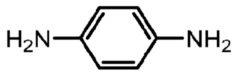 | 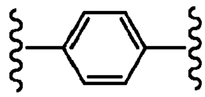 |
| isophorone diamine | 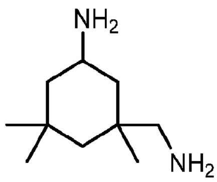 | 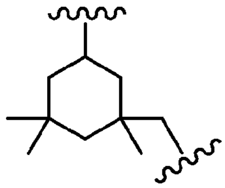 |
| diaminocyclohexane | 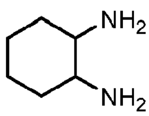 | 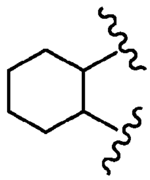 |

(continued)

| Diamine Name | Diamine Structure | Corresponding $L^1$ Structure |
|---|---|---|
| 4-Cyclohexene-1,2-diamine | 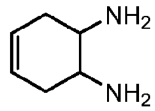 | 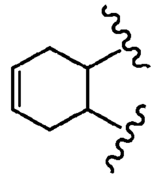 |
| 3-Hexyne-1,6-diamine | 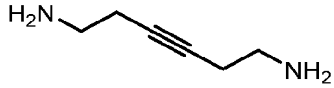 | 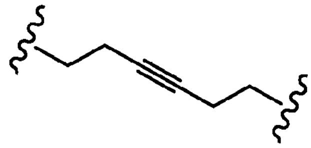 |
| bis-(p-aminocyclohexyl) methane | 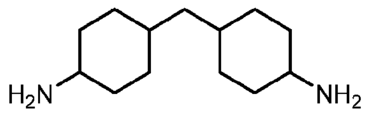 | 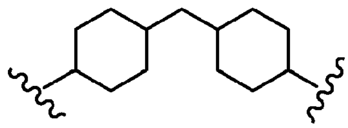 |
| methylene dianiline | 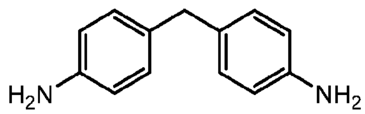 | 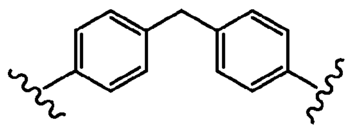 |

Bis-(p-aminocyclohexyl) methane and methylene dianiline, as well as derivatives thereof are particularly preferred difunctional amines. In some embodiments, $L^1$ is a divalent hydrocarbyl linker comprising two rings conjoined by a divalent $C_1$ to $C_{10}$ alkyl group. The two rings may independently be carbocycles or heterocycles, but are both preferably carbocycles. The two rings may independently be 3- to 14-membered, but are preferably both 6-membered. The two rings may independently be aromatic, saturated or partially saturated, but are preferably saturated. Therefore, the two rings are most preferably cyclohexane.

In a preferred embodiment, the compound of formula (I) has the general formula (Ia), shown below.

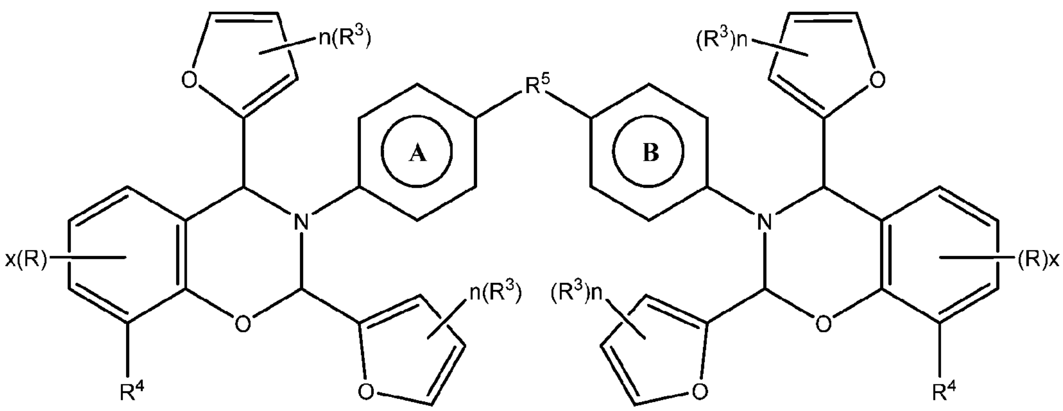

(Ia)

wherein ring A and ring B are independently either aromatic or saturated 6 membered carbon rings, preferably wherein rings A and B are both saturated rings (e.g. both a cyclohexane group);
wherein $R^5$ is a divalent $C_1$ to $C_{10}$ alkyl group, preferably wherein $R^5$ is a methylene group; and
wherein R, $R^3$, x, and n are independently as defined hereinabove, preferably where both x values = 0 and/or wherein all n values = 0.

As can be seen from formula (I), substituted phenols may be used to provide the corresponding substituted benzoxazine, or unsubstituted phenols may be used to provide an unsubstituted benzoxazine. In formula (I) the optional benzoxazine substituents are defined by group R.

However, both positions ortho to the phenolic -OH group, *i.e.* the 2-position and 6-position, must be unsubstituted( *i.e.* substituted by hydrogen). This is because a first unsubstituted ortho position, is required for the Mannich reaction to take place to form the benzoxazine ring, and the second ortho position is required for the benzoxazine self-polymerisation to take place. Thus, the phenolic precursor must be 2,6-unsubsituted, in relation to the phenolic group, in order to provide a benzoxazine that is 6-unsubsituted in relation to the benzoxazine oxygen. In formula (I), the 6-unsubsituted position is defined by $R^4$, wherein $R^4$ is hydrogen. This leaves 3 positions available for substitution with an R group, such that 0 to 3 R groups may exist on each benzoxazine ring. The number of possible R groups on each benzoxazine ring is shown in formula (I) as x. Two identical phenolic compounds may be used to form a symmetrical difunctional benzoxazine, or two different phenolic compounds may be used to form an asymmetrical difunctional benzoxazine, as long as both phenolic compounds are 2,6-unsubsituted, in relation to the phenolic oxygen.

Preferably, naturally derived phenolic compounds may be used in order to further increase the content of bio-carbon in the resulting benzoxazine. One such example of bio derivable phenolic compound is cardanol. Cardanol is a mixture of straight chain 3-($C_{15}$)alkyl and 3-($C_{15}$)alkenyl phenols and is obtained from anacardic acid, the main component of cashew nutshell liquid, a by-product of cashew nut processing. Preferably cardanol is used as the phenolic compound giving a compound of formula (I) wherein one R group is present at each benzene 3-position and is selected from a $-C_{15}H_{31}$ straight chain alkyl group, $-C_{15}H_{29}$ straight chain alkenyl group, $-C_{15}H_{27}$ straight chain alkenyl group, and $-C_{15}H_{25}$ straight chain alkenyl group. As would be appreciated, a mixture of symmetrical and asymmetrical products would be obtained, all of which are within the scope of formula (I).

In another aspect, the benzoxazine compounds of the present invention have the general formula (II), shown below.

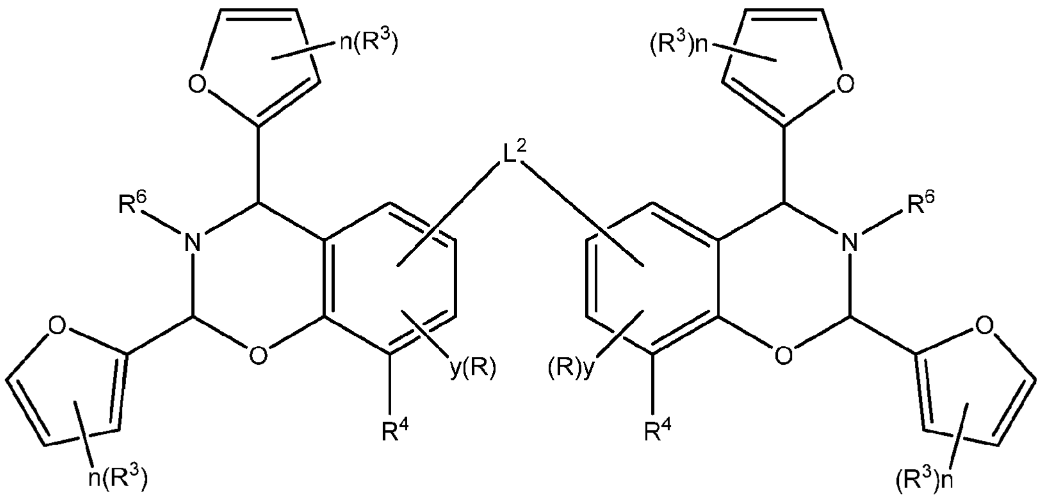

(II)

wherein each R group is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{20}$ cycloalkyl, $C_1$ to $C_{20}$ alkyl-$C_3$ to $C_{10}$ heterocycloalkyl, $C_1$ to $C_{20}$ alkyloxy, $C_1$ to $C_{20}$ alkylamino, -OH, $-OR^1$, $-NH_2$, $-NHR^1$, $-NR^1_2$, -C(O)OH, $-C(O)OR^1$, $-C(O)NH_2$, - $C(O)NHR^1$, $-C(O)NR^1_2$, -O(CO)H, $-O(CO)R^1$, -NH(CO)H, $-NH(CO)R^1$, $-NR^1(CO)H$, - $NR^1(CO)R^1$, -SH, $-SR^1$, $-SO_2H$, $-SO_2R^1$, $-SO_3R^1$, $-SO_3H$, $-SiR^1_3$, $-NO_2$, -CN, -F, -Cl, -Br, - I, $C_6$ to $C_{14}$ aryl optionally substituted with one or more $R^2$ group, and $C_2$ to $C_9$ heteroaryl optionally substituted with one or more $R^2$ group;

wherein each $R^1$ is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{10}$ cycloalkyl, $C_1$ to $C_{20}$ alkyl-$C_3$ to $C_{10}$ heterocycloalkyl, $C_1$ to $C_{20}$ alkoxy, $C_1$ to $C_{20}$ alkylamino, $C_6$ to $C_{14}$ aryl, and $C_2$ to $C_9$ heteroaryl;

wherein $R^2$ is independently selected from: $C_1$ to $C_{20}$ alkyl, $C_1$ to $C_{20}$ haloalkyl, $C_3$ to $C_{20}$ cycloalkyl, $C_2$ to $C_{20}$ alkenyl, $C_2$ to $C_{20}$ alkynyl, -OH, $-NH_2$, $-OC_1$ to $C_{20}$ alkyl, $-NHC_1$ to $C_{20}$ alkyl, $-N(C_1$ to $C_{20}$ alkyl$)_2$, $-NO_2$, -CN, -F, -Cl, -Br, and -I;

wherein $R^3$ is independently selected from: $C_1$ to $C_6$ alkyl, $C_1$ to $C_6$ alkenyl, $C_1$ to $C_6$ alkoxy, $C_1$ to $C_6$ alkyloxy, -C(O)OH, $-C(O)O-C_1$ to $C_6$ alkyl, $C_1$ to $C_6$ alkyl-(O)O-H, $C_1$ to $C_6$ alkyl-(O)O-$C_1$ to $C_6$ alkyl, and -OH;

wherein $R^4$ is hydrogen;

wherein $R^6$ is independently a monovalent group selected from -H, -OH, or a hydrocarbyl group comprising 1 to 50 carbon atoms;

wherein $L^2$ is a direct bond, a divalent group selected from: C(O), O, S, S(O), $S(O)_2$, or a hydrocarbyl linker comprising

1 to 50 carbon atoms;
wherein y is independently 0, 1, or 2; and

wherein n is independently 0, 1, 2 or 3.

In some embodiments both y values ≤ 1, and preferably wherein both y values are the same, and/or wherein both benzene rings of the benzoxazine are substituted with the same R group(s), more preferably wherein both y values = 0.

Formula (II) comprises two benzoxazine moieties linked by a linker $L^2$ attached to the benzene ring of each benzoxazine. As will be appreciated, this difunctional benzoxazine may be derived from a bisphenol compound. For the purpose of the present invention, the linker $L^2$ may be independently attached to each benzene ring at any of the 3-, 4-, or 5-positions relative to the phenolic oxygen. The ortho (*i.e.* 2- and 6-positions) must remain unsubstituted, *i.e.* substituted by hydrogen in order for the Mannich reaction to proceed to form the benzoxazine ring, and for the self-polymerisation of the benzoxazine to occur.

In formula (II), $L^2$ is a direct bond, or a divalent group selected from C(O), O, S, S(O), $S(O)_2$ or a hydrocarbyl linker comprising 1 to 50 carbon atoms. In some embodiments $L^2$ is attached at the 4-position, relative to the benzoxazine oxygen. In some embodiments $L^2$ is selected from $S(O)_2$, $CH_2$, $C(CH_3)_2$, or a divalent benzene ring.

In some embodiments, $L^2$ comprises 2 to 50 carbon atoms, 3 to 50 carbon atoms, 5 to 50 carbon atoms, 2 to 40 carbon atoms, 3 to 40 carbon atoms, 5 to 40 carbon atoms, 2 to 30 carbon atoms, 3 to 30 carbon atoms, 5 to 30 carbon atoms, 2 to 20 carbon atoms, 3 to 20 carbon atoms, or 5 to 20 carbon atoms.

The linker $L^2$ may be attached to the resulting benzoxazine benzene ring at any available position, and not at the ortho position, which is occupied by $R^4$ in formula (II), wherein $R^4$ is hydrogen. It is preferred that $L^2$ is attached at the at the 4-position, relative to the benzoxazine oxygen. Many widely commercially available bisphenol compounds comprise linkers $L^2$ attached at both of the 4-positions relative to the phenolic oxygens, these are generally known as 4,4-bisphenols. Examples of 4,4-bisphenols suitable for use in the present invention are listed in Table 2 below.

**Table 2. Exemplary list bisphenols useful in the present invention and their corresponding $L^2$ linkers.**

| Bisphenol compound | Bisphenol structure | $L^2$ structure |
|---|---|---|
| Bisphenol A | 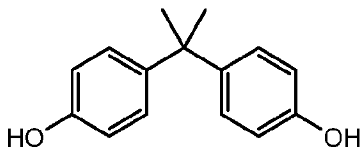 | $-C(CH_3)_2-$ |
| Bisphenol AF | 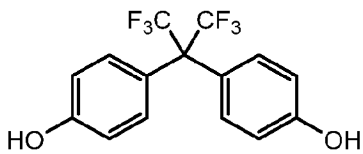 | $-C(CF_3)_2-$ |
| Bisphenol AP | 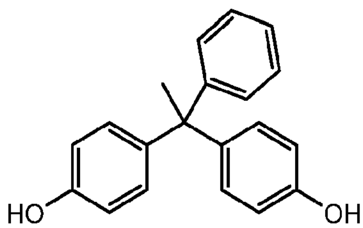 | $-C(CH_3)Ph-$ |
| Bisphenol B | 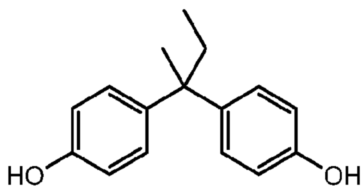 | $-C(CH_3)(CH_2CH_3)-$ |
| Bisphenol BP | 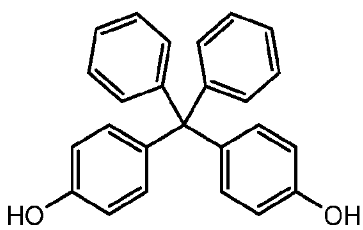 | $-CPh_2-$ |

(continued)

| Bisphenol compound | Bisphenol structure | $L^2$ structure |
|---|---|---|
| Bisphenol C | 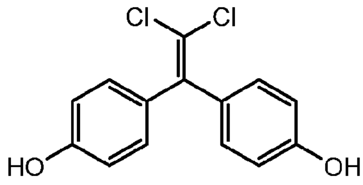 | $-C=CCl_2-$ |
| Bisphenol CP | 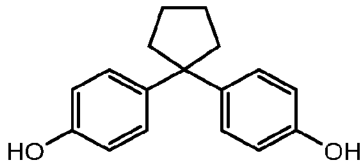 | 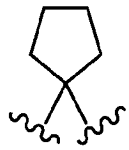 |
| Bisphenol E | 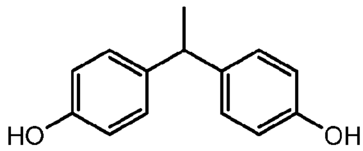 | $-C(CH_3)H-$ |
| Bisphenol F | 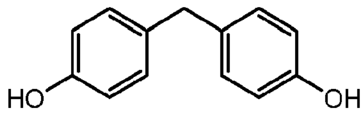 | $-CH_2-$ |
| Bisphenol FL | 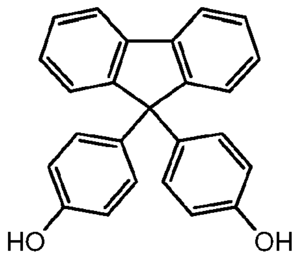 | 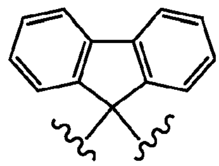 |
| Bisphenol M | 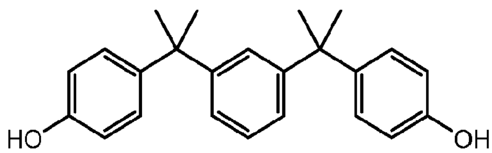 | 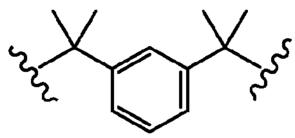 |
| Bisphenol P | 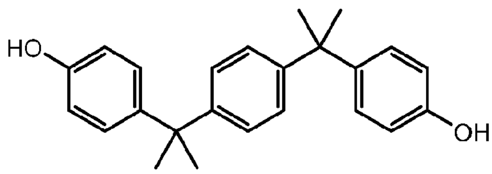 | 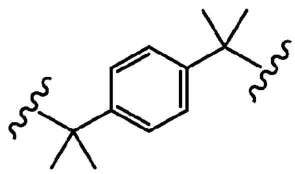 |
| Bisphenol S | 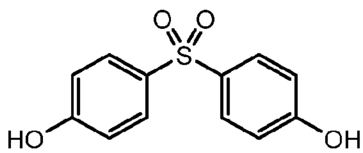 | $-S(O)_2-$ |
| Bisphenol TMC | 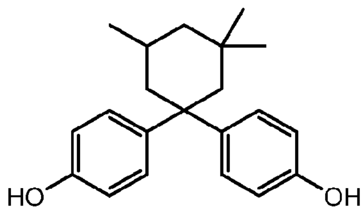 | 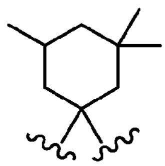 |

(continued)

| Bisphenol compound | Bisphenol structure | L2 structure |
|---|---|---|
| Bisphenol Z | 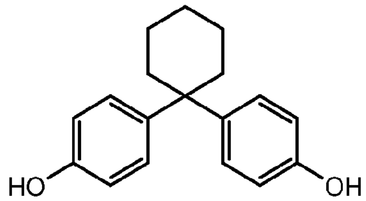 | 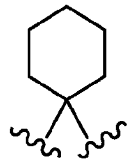 |
| 4,4'-Thiodiphenol | 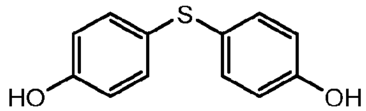 | -S- |
| 4,4'-Dihydroxydiphenyl ether | 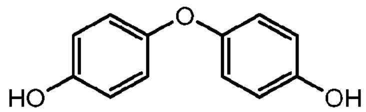 | -O- |
| 4,4'-Dihydroxybenzophenone | 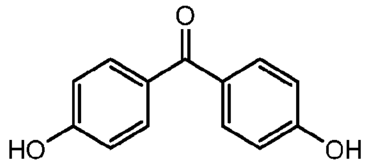 | -C(O) - |
| Phenolphthalein | 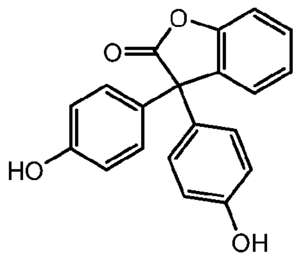 | 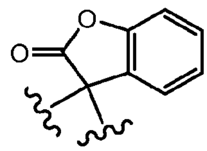 |
| dicyclopentadiene bisphenol | 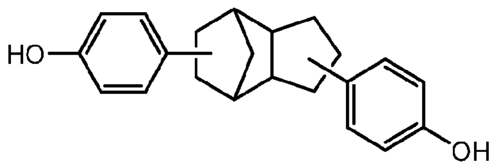 | 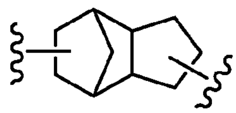 |
| 1,1':4',1"-Terphenyl-4,4"-diol | 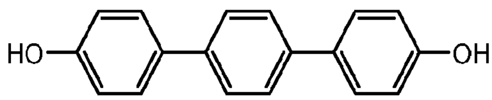 | 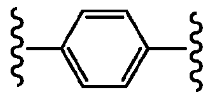 |
| 1,4-Bis(4-hydroxybenzyl)benzol | 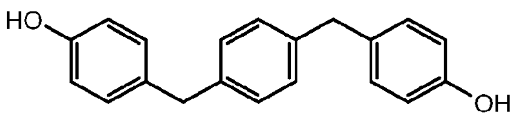 | 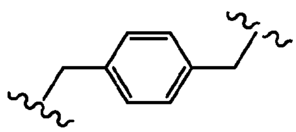 |
| 4,4'-Biphenol | 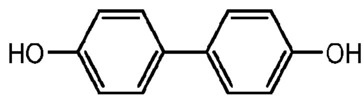 | Direct bond |

As would be appreciated, the $L^2$ groups would be conserved during a Mannich reaction and would thus be present in the compound of formula (II) when derived from the corresponding bisphenol compound precursor. Whilst the bisphenol compounds listed in Table 2 are all 4,4-bisphenols, the scope of the invention also includes any isomers of these bisphenol compounds wherein the $L^2$ linker is attached at any combination of the 3-, 4- and 5-positions.

This leaves two positions available for substitution with an R group, such that 0 to 2 R groups may exist on each benzoxazine ring. The number of possible R groups on each benzoxazine ring is shown in formula (II) as y. A symmetrical bisphenol compound, with the same or no substituents on each phenol group may be used to form a symmetrical difunctional benzoxazine, or an asymmetrical bisphenol compound, with differing substituents on each phenol group may

be used may be used to form an asymmetrical difunctional benzoxazine, as long as both phenol groups are 2,6-unsubsituted, in relation to the phenolic oxygen, in both cases the total number of R groups is y + y.

In another aspect, the benzoxazine compounds of the present invention have the general formula (IIIa), shown below.

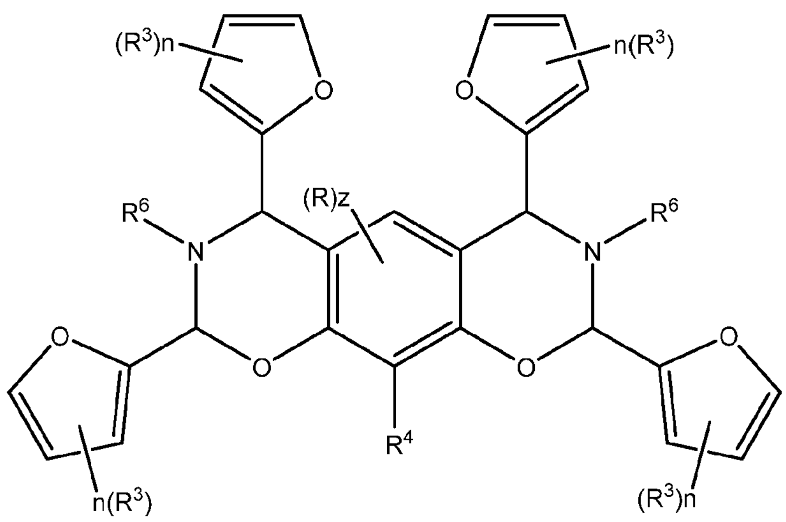

(IIIa)

wherein each R group is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{20}$ cycloalkyl, $C_1$ to $C_{20}$ alkyl-$C_3$ to $C_{10}$ heterocycloalkyl, $C_1$ to $C_{20}$ alkyloxy, $C_1$ to $C_{20}$ alkylamino, -OH, $-OR^1$, $-NH_2$, $-NHR^1$, $-NR^1_2$, -C(O)OH, $-C(O)OR^1$, $-C(O)NH_2$, - $C(O)NHR^1$, $-C(O)NR^1_2$, -O(CO)H, $-O(CO)R^1$, -NH(CO)H, $-NH(CO)R^1$, $-NR^1(CO)H$, - $NR^1(CO)R^1$, -SH, $-SR^1$, $-SO_2H$, $-SO_2R^1$, $-SO_3R^1$, $-SO_3H$, $-SiR^1_3$, $-NO_2$, -CN, -F, -Cl, -Br, - I, $C_6$ to $C_{14}$ aryl optionally substituted with one or more $R^2$ group, and $C_2$ to $C_9$ heteroaryl optionally substituted with one or more $R^2$ group;

wherein each $R^1$ is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{10}$ cycloalkyl, $C_1$ to $C_{20}$ alkyl-$C_3$ to $C_{10}$ heterocycloalkyl, $C_1$ to $C_{20}$ alkoxy, $C_1$ to $C_{20}$ alkylamino, $C_6$ to $C_{14}$ aryl, and $C_2$ to $C_9$ heteroaryl;

wherein $R^2$ is independently selected from: $C_1$ to $C_{20}$ alkyl, $C_1$ to $C_{20}$ haloalkyl, $C_3$ to $C_{20}$ cycloalkyl, $C_2$ to $C_{20}$ alkenyl, $C_2$ to $C_{20}$ alkynyl, -OH, $-NH_2$, $-OC_1$ to $C_{20}$ alkyl, $-NHC_1$ to $C_{20}$ alkyl, $-N(C_1$ to $C_{20}$ $alkyl)_2$, $-NO_2$, -CN, -F, -Cl, -Br, and -I;

wherein $R^3$ is independently selected from: $C_1$ to $C_6$ alkyl, $C_1$ to $C_6$ alkenyl, $C_1$ to $C_6$ alkoxy, $C_1$ to $C_6$ alkyloxy, -C(O)OH, $-C(O)O-C_1$ to $C_6$ alkyl, $C_4$ to $C_6$ alkyl-(O)O-H, $C_1$ to $C_6$ alkyl-(O)O-$C_1$ to $C_6$ alkyl, and -OH;

wherein $R^4$ is hydrogen;

wherein $R^6$ is independently a monovalent group selected from -H, -OH, or a hydrocarbyl group comprising 1 to 50 carbon atoms;

wherein z is 0 or 1; and

wherein n is independently 0, 1, 2 or 3.

In this aspect, the difunctional benzoxazine may be derived from a 1,3-benzene-diol. Two oxazine rings are formed on the shared benzene ring, giving a difunctional benzoxazine with a shared benzene ring. The compound of formula (IIIa) may or may not be substituted with an R group at the 5-position of the benzene ring relative to the benzoxazine oxygens which are considered to occupy the 1- and 3-positions. The corresponding 4- and 6-positions of the benzene ring are occupied carbon atoms that form part of the benzoxazine ring. The compound of formula (IIIa)must be unsubstituted at the 2-position, relative to the benzoxazine oxygens which are considered to occupy the 1- and 3-positions, so that self-polymerisation of the resulting benzoxazine may take place at this position. The 2-position is occupied by $R^4$ in formula (IIIa), wherein $R^4$ is hydrogen. Therefore, the 1,3-benzene-diol precursor must be unsubstituted at the 4- and 6-positions in order to allow the oxazine rings to be formed at these positions, and the 2-position must be unsubstituted to allow for self-polymerisation of the resulting benzoxazine compound.

This leaves only one position available for optional substitution on the benzoxazine ring. The number of possible

R groups on the benzoxazine ring is shown in formula (IIIa) as z. Thus, z is either 1 or 0. In a preferred embodiment of the compound of formula (IIIa), z = 0 and so the compound of formula (IIIa) is unsubstituted at the 5-position. The corresponding precursor without substitution at the 5-position, *i.e.* 1,3-benzene-diol, is known as resorcinol.

In another embodiment, the compound of formula (IIIa) is substituted with a $C_1$ to $C_{20}$ alkyl group or a $C_2$ to $C_{20}$ alkenyl group at the 5-position. The corresponding precursor 1,3-benzene-diols, *i.e.* 5-alkylresorcinols and 5-alkeneylresorcinols are readily available from natural sources, one such example is 5-Pentadecenylresorcinol, also known as bilobol, which is found in Ginkgo biloba fruits.

In a related aspect, the benzoxazine compounds of the present invention have the general formula (IIIb), shown below.

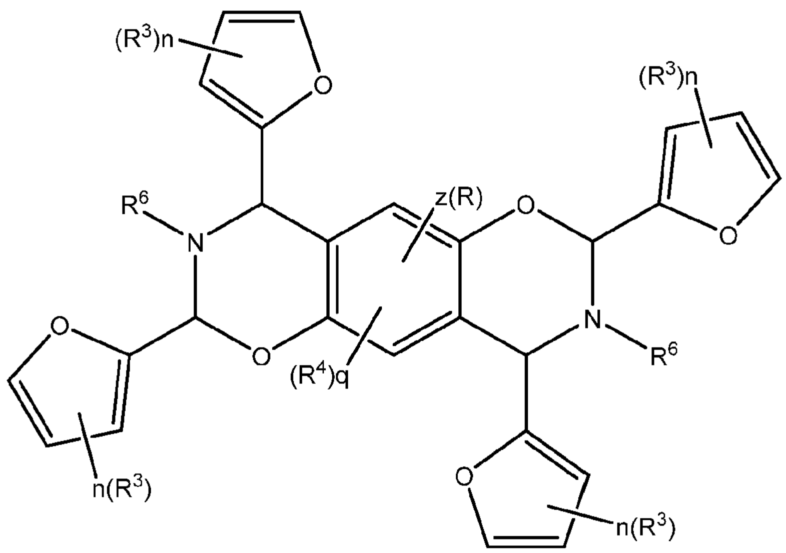

(IIIb)

wherein each R group is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{20}$ cycloalkyl, $C_1$ to $C_{20}$ alkyl-$C_3$ to $C_{10}$ heterocycloalkyl, $C_1$ to $C_{20}$ alkyloxy, $C_1$ to $C_{20}$ alkylamino, -OH, $-OR^1$, $-NH_2$, $-NHR^1$, $-NR^1_2$, -C(O)OH, $-C(O)OR^1$, $-C(O)NH_2$, - $C(O)NHR^1$, $-C(O)NR^1_2$, -O(CO)H, $-O(CO)R^1$, -NH(CO)H, $-NH(CO)R^1$, $-NR^1(CO)H$, - $NR^1(CO)R^1$, -SH, $-SR^1$, $-SO_2H$, $-SO_2R^1$, $-SO_3R^1$, $-SO_3H$, $-SiR^1_3$, $-NO_2$, -CN, -F, -Cl, -Br, - I, $C_6$ to $C_{14}$ aryl optionally substituted with one or more $R^2$ group, and $C_2$ to $C_9$ heteroaryl optionally substituted with one or more $R^2$ group;

wherein each $R^1$ is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{10}$ cycloalkyl, $C_1$ to $C_{20}$ alkyl-$C_3$ to $C_{10}$ heterocycloalkyl, $C_1$ to $C_{20}$ alkoxy, $C_1$ to $C_{20}$ alkylamino, $C_6$ to $C_{14}$ aryl, and $C_2$ to $C_9$ heteroaryl;

wherein $R^2$ is independently selected from: $C_1$ to $C_{20}$ alkyl, $C_1$ to $C_{20}$ haloalkyl, $C_3$ to $C_{20}$ cycloalkyl, $C_2$ to $C_{20}$ alkenyl, $C_2$ to $C_{20}$ alkynyl, -OH, $-NH_2$, $-OC_1$ to $C_{20}$ alkyl, $-NHC_1$ to $C_{20}$ alkyl, $-N(C_1$ to $C_{20}$ alkyl$)_2$, $-NO_2$, -CN, -F, -Cl, -Br, and -I;

wherein $R^3$ is independently selected from: $C_1$ to $C_6$ alkyl, $C_1$ to $C_6$ alkenyl, $C_1$ to $C_6$ alkoxy, $C_1$ to $C_6$ alkyloxy, -C(O)OH, -C(O)O-$C_4$ to $C_6$ alkyl, $C_1$ to $C_6$ alkyl-(O)O-H, $C_1$ to $C_6$ alkyl-(O)O-$C_1$ to $C_6$ alkyl, and -OH;

wherein $R^4$ is hydrogen;

wherein $R^6$ is independently a monovalent group selected from -H, -OH, or a hydrocarbyl group comprising 1 to 50 carbon atoms;

wherein z is 0 or 1;

wherein q = 2 - z; and

wherein n is independently 0, 1, 2 or 3.

In this aspect, the difunctional benzoxazine may be derived from a 1,4-benzene-diol. Two oxazine rings are formed on the shared benzene ring, giving a difunctional benzoxazine with a shared benzene ring. The compound of formula (IIIb) may or may not be substituted with an R group at one of the two positions on the benzene ring ortho to the benzoxazine oxygens that is not occupied by a carbon atom of the benzoxazine ring. At least one of, preferably both of, the positions on the benzene ring ortho to the benzoxazine oxygens that is not occupied by a carbon atom of the benzoxazine

ring, must remain unsubstituted so that self-polymerisation of the benzoxazine of formula (IIIb) may take place.

At least three positions on the 1,4-benzene-diol precursor must be unsubstituted, two of which must be unsubstituted in order to allow the oxazine rings to be formed at these positions, and a further one, preferably two must be, so that self-polymerisation of the resulting benzoxazine may take place.

The number of possible R groups on the benzoxazine ring is shown in formula (IIIb) as z. z is either 1 or 0. If one position ortho to a benzoxazine oxygen that is not occupied by a carbon atom of the benzoxazine ring is substituted with an R group, then the other position ortho to the other benzoxazine oxygen that is not occupied by a carbon atom of the other benzoxazine ring must be substituted with $R^4$, wherein $R^4$ is hydrogen. If neither of the positions ortho to the benzoxazine oxygens that are not occupied by carbon atoms of the benzoxazine rings are substituted with an R group, then both of these positions must be substituted with $R^4$, wherein $R^4$ is hydrogen. Therefore, the number of $R^4$ groups on the benzoxazine ring is shown in formula (IIIb) is q, wherein q = 2 - z.

In some embodiments, the compounds of any one of Formulae (I), (II), (IIIa) and (IIIb) are selected from those wherein R is independently selected from: $C_1$ to $C_{20}$ alkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{10}$ cycloalkyl, $C_1$ to $C_{20}$ alkyl-$C_3$ to $C_{10}$ heterocycloalkyl, $C_1$ to $C_{20}$ alkyloxy, $C_1$ to $C_{20}$ alkylamino, -OH, -$OR^1$, -$NH_2$, -$NHR^1$, -$NR^1_2$, -O(CO)H, -O(CO)$R^1$, -NH(CO)H, - NH(CO)$R^1$, -$NR^1$(CO)H, -$NR^1$(CO)$R^1$, -SH, -$SR^1$, -$SiR^1_3$, -F, -Cl, -Br, -I, $C_6$ to $C_{14}$ aryl optionally substituted with one or more $R^2$ group, and $C_2$ to $C_9$ heteroaryl optionally substituted with one or more $R^2$ group;

wherein $R^1$ is independently selected from: $C_1$ to $C_{20}$ alkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{20}$ cycloalkyl, $C_1$ to $C_{20}$ alkyl-$C_3$ to $C_{10}$, heterocycloalkyl, $C_1$ to $C_{20}$ alkoxy, $C_1$ to $C_{20}$ alkylamino, $C_6$ to $C_{14}$ aryl, and $C_2$ to $C_9$ heteroaryl; and

wherein $R^2$ is independently selected from: $C_1$ to $C_{20}$ alkyl, $C_3$ to $C_{20}$ cycloalkyl, $C_2$ to $C_{20}$ alkenyl, $C_2$ to $C_{20}$ alkynyl, -OH, -$NH_2$, -$OC_1$ to $C_{20}$ alkyl, -$NHC_1$ to $C_{20}$ alkyl, -N($C_1$ to $C_{20}$ alkyl)$_2$, -F, -Cl, -Br, or -I.

In some embodiments, the compounds of any one of Formulae (I), (II), (IIIa) and (IIIb) are selected from those wherein R is independently selected from: $C_1$ to $C_{20}$ alkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyloxy, $C_1$ to $C_{20}$ alkylamino, -OH, -$OR^1$, -$NH_2$, -$NHR^1$, -$NR^1_2$, $C_6$ to $C_{14}$ aryl optionally substituted with one or more $R^2$ group, or $C_2$ to $C_9$ heteroaryl optionally substituted with one or more $R^2$ group; preferably -OH;

wherein $R^1$ is independently selected from: $C_1$ to $C_{20}$ alkyl, $C_3$ to $C_{20}$ cycloalkyl or $C_2$ to $C_{20}$ alkenyl;

wherein $R^2$ is independently selected from: $C_1$ to $C_{20}$ alkyl, -OH, -$NH_2$, -$OC_1$ to $C_{20}$ alkyl, -$NHC_1$ to $C_{20}$ alkyl or -N($C_1$ to $C_{20}$ alkyl)$_2$, preferably -OH

In some embodiments, the compounds of any one of Formulae (I), (II), (IIIa) and (IIIb) are selected from those wherein R is independently selected from: $C_1$ to $C_{10}$ alkyl, $C_1$ to $C_{10}$ haloalkyl, $C_3$ to $C_{10}$ cycloalkyl, $C_3$ to $C_{40}$ heterocycloalkyl, $C_2$ to $C_{10}$ alkenyl, $C_2$ to $C_{10}$ alkynyl, $C_1$ to $C_{10}$ alkyl-$C_6$ to $C_{14}$ aryl, $C_1$ to $C_{10}$ alkyl-$C_2$ to $C_9$ heteroaryl, $C_1$ to $C_{10}$ alkyl-$C_3$ to $C_{10}$ cycloalkyl, $C_1$ to $C_{10}$ alkyl-$C_3$ to $C_{10}$ heterocycloalkyl, $C_1$ to $C_{10}$ alkyloxy, $C_1$ to $C_{10}$ alkylamino, -OH, -$OR^1$, -$NH_2$, -$NHR^1$, -$NR^1_2$, -C(O)OH, -C(O)$OR^1$, -C(O)$NH_2$, - C(O)$NHR^1$, -C(O)$NR^1_2$, -O(CO)H, -O(CO)$R^1$, -NH(CO)H, -NH(CO)$R^1$, -$NR^1$(CO)H, - $NR^1$(CO)$R^1$, -SH, -$SR^1$, -$SO_2$H, -$SO_2R^1$, -$SO_3R^1$, -$SO_3$H, -$SiR^1_3$, -$NO_2$, -CN, -F, -Cl, -Br, - I, $C_6$ to $C_{14}$ aryl optionally substituted with one or more $R^2$ group, and $C_2$ to $C_9$ heteroaryl optionally substituted with one or more $R^2$ group;

wherein each $R^1$ is independently selected from: $C_1$ to $C_{10}$ alkyl, $C_1$ to $C_{10}$ haloalkyl, $C_3$ to $C_{10}$ cycloalkyl, $C_3$ to $C_{10}$ heterocycloalkyl, $C_2$ to $C_{10}$ alkenyl, $C_2$ to $C_{10}$ alkynyl, $C_1$ to $C_{10}$ alkyl-$C_6$ to $C_{14}$ aryl, $C_1$ to $C_{10}$ alkyl-$C_2$ to $C_9$ heteroaryl, $C_1$ to $C_{10}$ alkyl-$C_3$ to $C_{10}$ cycloalkyl, $C_1$ to $C_{10}$ alkyl-$C_3$ to $C_{10}$ heterocycloalkyl, $C_1$ to $C_{10}$ alkoxy, $C_1$ to $C_{10}$ alkylamino, $C_6$ to $C_{14}$ aryl, and $C_2$ to $C_9$ heteroaryl;

wherein $R^2$ is independently selected from: $C_1$ to $C_{10}$ alkyl, $C_1$ to $C_{10}$ haloalkyl, $C_3$ to $C_{10}$ cycloalkyl, $C_2$ to $C_{10}$ alkenyl, $C_2$ to $C_{10}$ alkynyl, -OH, -$NH_2$, -$OC_1$ to $C_{10}$ alkyl, -$NHC_1$ to $C_{10}$ alkyl, -N($C_1$ to $C_{10}$ alkyl)$_2$, -$NO_2$, -CN, -F, -Cl, -Br, and -I.

In some embodiments, the compounds of any one of Formulae (I), (II), (IIIa) and (IIIb) are selected from those wherein R is independently selected from: $C_1$ to $C_{10}$ alkyl, $C_3$ to $C_{10}$ cycloalkyl, $C_3$ to $C_{10}$ heterocycloalkyl, $C_2$ to $C_{10}$ alkenyl, $C_2$ to $C_{10}$ alkynyl, $C_1$ to $C_{10}$ alkyl-$C_6$ to $C_{14}$ aryl, $C_1$ to $C_{10}$ alkyl-$C_2$ to $C_9$ heteroaryl, $C_1$ to $C_{10}$ alkyl-$C_3$ to $C_{10}$ cycloalkyl, $C_1$ to $C_{10}$ alkyl-$C_3$ to $C_{10}$ heterocycloalkyl, $C_1$ to $C_{10}$ alkyloxy, $C_1$ to $C_{10}$ alkylamino, -OH, -$OR^1$, -$NH_2$, -$NHR^1$, -$NR^1_2$, -O(CO)H, -O(CO)$R^1$, -NH(CO)H, - NH(CO)$R^1$, -$NR^1$(CO)H, -$NR^1$(CO)$R^1$, -SH, -$SR^1$, -$SiR^1_3$, -F, -Cl, -Br, -I, $C_6$ to $C_{14}$ aryl optionally substituted with one or more $R^2$ group, and $C_2$ to $C_9$ heteroaryl optionally substituted with one or more $R^2$

group;

wherein $R^1$ is independently selected from: $C_1$ to $C_{10}$ alkyl, $C_3$ to $C_{10}$ cycloalkyl, $C_3$ to $C_{10}$ heterocycloalkyl, $C_2$ to $C_{10}$ alkenyl, $C_2$ to $C_{10}$ alkynyl, $C_1$ to $C_{10}$ alkyl-$C_6$ to $C_{14}$ aryl, $C_1$ to $C_{10}$ alkyl-$C_2$ to $C_9$ heteroaryl, $C_1$ to $C_{10}$ alkyl-$C_3$ to $C_{10}$ cycloalkyl, $C_1$ to $C_{10}$ alkyl-$C_3$ to $C_{10}$, heterocycloalkyl, $C_1$ to $C_{10}$ alkoxy, $C_1$ to $C_{10}$ alkylamino, $C_6$ to $C_{14}$ aryl, and $C_2$ to $C_9$ heteroaryl; and
wherein $R^2$ is independently selected from: $C_1$ to $C_{10}$ alkyl, $C_3$ to $C_{10}$ cycloalkyl, $C_2$ to $C_{10}$ alkenyl, $C_2$ to $C_{10}$ alkynyl, -OH, $-NH_2$, $-OC_1$ to $C_{10}$ alkyl, $-NHC_1$ to $C_{10}$ alkyl, $-N(C_1$ to $C_{10}$ alkyl$)_2$, -F, -Cl, -Br, or -I.

In some embodiments, the compounds of any one of Formulae (I), (II), (IIIa) and (IIIb) are selected from those wherein R is independently selected from: $C_1$ to $C_{10}$ alkyl, $C_3$ to $C_{10}$ cycloalkyl, $C_3$ to $C_{10}$ heterocycloalkyl, $C_2$ to $C_{10}$ alkenyl, $C_2$ to $C_{10}$ alkynyl, $C_1$ to $C_{10}$ alkyloxy, $C_1$ to $C_{10}$ alkylamino, -OH, $-OR^1$, $-NH_2$, $-NHR^1$, $-NR^1_2$, $C_6$ to $C_{14}$ aryl optionally substituted with one or more $R^2$ group, or $C_2$ to $C_9$ heteroaryl optionally substituted with one or more $R^2$ group; preferably -OH;

wherein $R^1$ is independently selected from: $C_1$ to $C_{10}$ alkyl, $C_3$ to $C_{10}$ cycloalkyl or $C_2$ to $C_{10}$ alkenyl;
wherein $R^2$ is independently selected from: $C_1$ to $C_{10}$ alkyl, -OH, $-NH_2$, $-OC_1$ to $C_{10}$ alkyl, $-NHC_1$ to $C_{10}$ alkyl or $-N(C_1$ to $C_{10}$ alkyl$)_2$, preferably -OH.

It is preferred that the R group(s) present on the benzoxazine ring(s) of compounds of any of Formulae (I), (II), (IIIa), and/or (IIIb) are activating groups. The activating properties of an activating group derive from their ability to donate electrons into an aromatic system, thus enhancing its affinity towards electrophiles, *i.e.* activating groups are electron donating groups. The degree of electron donation of an electron donating group is proportional to its strength as an activating group. These properties can be determined in relation to a specific substituent using the Hammett equation:

$$\sigma = \log K_X - \log K_H$$

$K_H$ is the ionisation constant for benzoic acid in water at 25 °C and $K_X$ is the ionisation constant in water at 25 °C for a benzoic acid substituted with the electron donating group in question. σ is the Hammett substituent constant, the more negative the Hammett substituent constant is, the more electron donating the substituent is, and therefore the stronger an activating group the substituent is. The Hammett substituent constant can thus be used to measure the strength of an activating group.

The Hammett substituent constant of a substituent will differ depending on its position on the benzoic acid (e.g. *-para, -meta or -ortho*), the *-para, -meta and -ortho* Hammett substituent constants thus represent the activating effect the substituent has on the position *-para, -meta or -ortho* to the substituent, respectively. Hammett substituent constants for substituents at the *-ortho* position cannot be accurately experimentally calculated due to the interference of steric effects. However, *-ortho* Hammett substituent constants can be estimated using density functional theory and are found to closely correspond to the *-para* Hammett substituent constants. Hammett substituent constants of an electron donating group at the *-meta* position tend to be significantly smaller than those at the *-para* and *-ortho* positions, which gives rise to the preferentially *-ortho* and - *para* regioselective directing effects of activating groups. *-Meta* Hammett substituent constants are thus of little relevance to electrophilic aromatic substitution.

The Hammett substituent constant (σ) herein refers to the Hammett substituent constant of the substituent in question at the *-para* position, which is approximately equivalent to the Hammett substituent constant at the *-ortho* position and corresponds to the increase in reactivity of the aromatic ring with regard to electrophilic aromatic substitution at both the *-para* and *-ortho* positions. As would be appreciated, a more negative Hammett substituent constant corresponds to an increase in reactivity.

Literature values of Hammett substituent constants for common substituents would be known to the skilled person and can be found by reference to textbooks, for example, Hansch. C, Leo. A, Substituent constants for correlation analysis in chemistry and biology, New York NY: Wiley, 1979. Hammett substituent constants could also be easily measured by the skilled person by methods known in the art, see Hammett. P, The Effect of Structure upon the Reactions of Organic Compounds. Benzene Derivatives, J. Am. Chem. Soc., 1937, 59, 1, 96-103 and Hansch. C et al A Survey of Hammett Substituent Constants and Resonance and Field Parameters, Chem. Rev., 1991, 91, 165-195.

Table 3 provides examples of Hammett substituent constants, as reported by Hammett. P, The Effect of Structure upon the Reactions of Organic Compounds. Benzene Derivatives, J. Am. Chem. Soc., 1937, 59, 1, 96-103 and Hansch. C et al A Survey of Hammett Substituent Constants and Resonance and Field Parameters, Chem. Rev., 1991, 91, 165-195, as well as Hansch. C, Leo. A, Substituent constants for correlation analysis in chemistry and biology, New York NY: Wiley, 1979. As described hereinabove, Hammett Substituent constants reported in Table 3 are the para Hammett Substituent Constants.

**Table 3. Hammett substituent constants**

| Group | Structure | Hammett Substituent Constant (σ) |
|---|---|---|
| Dimethyl amine | $-NMe_2$ | -0.830 |
| Primary amine | $-NH_2$ | -0.660 |
| Hydroxy | -OH | -0.370 |
| Methoxy | -OMe | -0.268 |
| Ethoxy | -OEt | -0.250 |
| Methyl | $-CH_3$ | -0.170 |
| Benzyl | $-CH_2C_6H_5$ | -0.090 |
| Trimethyl silyl | $-SiMe_3$ | -0.070 |
| Phenyl | $-C_6H_6$ | -0.010 |

In some embodiments, the compounds of any one of Formulae (I), (II), (IIIa) and (IIIb) are selected from those wherein R is selected from a substituent having a Hammett substituent constant (σ) of less than zero, more preferably less than -0.050, even more preferably less than -0.100, most preferably less than -0.200.

Unsubstituted furfural is a particularly desirable feedstock due to its low toxicity and abundant availability from natural sources. Related furan 2-carbaldehydes are also derivable from natural sources and thus also desirable chemical feedstocks, in particular 5-(Hydroxymethyl)-2-furaldehyde, 5-(methoxymethyl)-2-furaldehyde, and 5-methy-2-furfuraldehyde are preferred furfuraldehydes useful in the present invention. Unsubstituted furfural is the most preferred.

In some embodiments, the compounds of any one of Formulae (I), (II), (IIIa) and (IIIb) are selected from those wherein $R^3$ is independently selected from: $C_1$ to $C_6$ alkyl, $C_1$ to $C_6$ alkoxy, $C_1$ to $C_6$ alkyloxy, and -OH, preferably $-CH_3$, $-CH_2OH$, or $-CH_2OCH_3$.

In some embodiments, the compounds of any one of Formulae (I), (II), (IIIa) and (IIIb) are selected from those wherein all n values ≤ 1, and preferably wherein all n values are the same, and/or wherein all $R^3$ groups are the same, and/or wherein all $R^3$ groups are attached at the position ortho to the furan oxygen; more preferably wherein all n values = 0.

In some embodiments, each $R^6$ group of compounds (II), (IIIa), and/or (IIIb) is independently selected from: -H, -OH, $C_1$ to $C_{20}$ alkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyloxy, $C_1$ to $C_{20}$ alkylamino, $C_6$ to $C_{14}$ aryl, and $C_2$ to $C_9$ heteroaryl; wherein $R^6$ is optionally substituted with one or more $R^7$ groups; wherein $R^7$ is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyloxy, $C_1$ to $C_{20}$ alkylamino, -OH, $-OR^1$, $-NH_2$, $-NHR^1$, $-NR^1_2$, - C(O)OH, $-C(O)OR^1$, $-C(O)NH_2$, $-C(O)NHR^1$, $-C(O)NR^1_2$, -O(CO)H, $-O(CO)R^1$, -NH(CO)H, $-NH(CO)R^1$, $-NR^1(CO)H$, $-NR^1(CO)R^1$, -SH, $-SR^1$, $-SO_2H$, $-SO_2R^1$, $-SO_3R^1$, $-SO_3H$, $-SiR^1_3$, $-NO_2$, -CN, -F, -Cl, -Br, -I, $C_6$ to $C_{14}$ aryl, and $C_2$ to $C_9$ heteroaryl.

In some embodiments, the compounds of any one of Formulae (II), (IIIa) and (IIIb) are selected from those wherein $R^6$ is independently selected from: -H, -OH, $C_1$ to $C_{20}$ alkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyloxy, $C_1$ to $C_{20}$ alkylamino, $C_6$ to $C_{14}$ aryl, and $C_2$ to $C_9$ heteroaryl; wherein $R^6$ is optionally substituted with one or more $R^7$ groups; wherein $R^7$ is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyloxy, $C_1$ to $C_{20}$ alkylamino, -OH, $-OR^1$, $-NH_2$, $-NHR^1$, $-NR^1_2$, -C(O)OH, $-C(O)OR^1$, $-C(O)NH_2$, $-C(O)NHR^1$, $-C(O)NR^1_2$, -O(CO)H, $-O(CO)R^1$, -NH(CO)H, $-NH(CO)R^1$, $-NR^1(CO)H$, $-NR^1(CO)R^1$, -SH, $-SR^1$, $-SO_2H$, $-SO_2R^1$, $-SO_3R^1$, $-SO_3H$, $-SiR^1_3$, $-NO_2$, -CN, -F, -Cl, -Br, -I, $C_6$ to $C_{14}$ aryl, and $C_2$ to $C_9$ heteroaryl.

In some embodiments, the compounds of any one of Formulae (II), (IIIa) and (IIIb) are selected from those wherein $R^6$ is independently selected from: H, -OH, $C_1$ to $C_{10}$ alkyl, $C_3$ to $C_{10}$ cycloalkyl, $C_3$ to $C_{10}$ heterocycloalkyl, $C_2$ to $C_{10}$ alkenyl, $C_2$ to $C_{10}$ alkynyl, $C_1$ to $C_{10}$ alkyloxy, $C_1$ to $C_{10}$ alkylamino, $C_6$ to $C_{14}$ aryl, and $C_2$ to $C_9$ heteroaryl; wherein $R^6$ is optionally substituted with one or more $R^7$ groups; wherein $R^7$ is independently selected from: $C_1$ to $C_{10}$ alkyl, $C_1$ to $C_{10}$ haloalkyl, $C_3$ to $C_{10}$ cycloalkyl, $C_3$ to $C_{10}$ heterocycloalkyl, $C_2$ to $C_{10}$ alkenyl, $C_2$ to $C_{10}$ alkynyl, $C_1$ to $C_{10}$ alkyloxy, $C_1$ to $C_{10}$ alkylamino, -OH, $-OR^1$, $-NH_2$, $-NHR^1$, $-NR^1_2$, -C(O)OH, $-C(O)OR^1$, $-C(O)NH_2$, $-C(O)NHR^1$, $-C(O)NR^1_2$, -O(CO)H, $-O(CO)R^1$, -NH(CO)H, $-NH(CO)R^1$, $-NR^1(CO)H$, $-NR^1(CO)R^1$, -SH, $-SR^1$, $-SO_2H$, $-SO_2R^1$, $-SO_3R^1$, $-SO_3H$, $-SiR^1_3$, $-NO_2$, -CN, -F, -Cl, -Br, -I, $C_6$ to $C_{14}$ aryl, and $C_2$ to $C_9$ heteroaryl.

In some embodiments, the compounds of any one of Formulae (II), (IIIa) and (IIIb) are selected from those wherein $R^6$ is independently selected from: H, -OH, $C_1$ to $C_{20}$ alkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyloxy, $C_6$ to $C_{14}$ aryl, and $C_2$ to $C_9$ heteroaryl; wherein $R^6$ is optionally substituted with one or more $R^7$ groups; wherein $R^7$ is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyloxy, -OH, $-OR^1$, $-NR^1_2$, $-C(O)OR^1$, $-C(O)NH_2$, $-C(O)NHR^1$, - $C(O)NR^1_2$, $-O(CO)R^1$, -NH(CO)H, $-NH(CO)R^1$, $-NR^1(CO)H$, $-NR^1(CO)R^1$, -SH, $-SR^1$, - $SiR^1_3$, $-NO_2$, -CN, -F, -Cl, -Br, -I, $C_6$ to $C_{14}$ aryl, and $C_2$ to $C_9$ heteroaryl.

In some embodiments, the compounds of any one of Formulae (II), (IIIa) and (IIIb) are selected from those wherein $R^6$ is independently selected from: -H, -OH, $C_1$ to $C_{10}$ alkyl, $C_3$ to $C_{10}$ cycloalkyl, $C_3$ to $C_{10}$ heterocycloalkyl, $C_2$ to $C_{10}$ alkenyl, $C_2$ to $C_{10}$ alkynyl, $C_1$ to $C_{10}$ alkyloxy, $C_6$ to $C_{14}$ aryl, and $C_2$ to $C_9$ heteroaryl; wherein $R^6$ is optionally substituted with one or more $R^7$ groups; wherein $R^7$ is independently selected from: $C_1$ to $C_{10}$ alkyl, $C_1$ to $C_{10}$ haloalkyl, $C_3$ to $C_{10}$ cycloalkyl, $C_3$ to $C_{10}$ heterocycloalkyl, $C_2$ to $C_{10}$ alkenyl, $C_2$ to $C_{10}$ alkynyl, $C_1$ to $C_{10}$ alkyloxy, -OH, $-OR^1$, $-NR^1_2$, $-C(O)OR^1$, $-C(O)NH_2$, $-C(O)NHR^1$, - $C(O)NR^1_2$, $-O(CO)R^1$, -NH(CO)H, $-NH(CO)R^1$, $-NR^1(CO)H$, $-NR^1(CO)R^1$, -SH, $-SR^1$, - $SiR^1_3$, $-NO_2$, -CN, -F, -Cl, -Br, -I, $C_6$ to $C_{14}$ aryl, and $C_2$ to $C_9$ heteroaryl.

In some embodiments, the compounds of any one of Formulae (II), (IIIa) and (IIIb) are selected from those wherein $R^6$ is independently selected from: H, -OH, $C_1$ to $C_{20}$ alkyl, $C_6$ to $C_{14}$ aryl, and $C_2$ to $C_9$ heteroaryl; wherein $R^6$ is optionally substituted with one or more $R^7$ groups; wherein $R^7$ is independently selected from: $C_1$ to $C_{20}$ alkyl, -OH, $-OR^1$, -$NR^1_2$, $-NO_2$, -CN, -F, -Cl, -Br, and -I.

In some embodiments, the compounds of any one of Formulae (II), (IIIa) and (IIIb) are selected from those wherein $R^6$ is independently selected from: -H, $C_1$ to $C_{10}$ alkyl, $C_6$ to $C_{14}$ aryl, and $C_2$ to $C_9$ heteroaryl; wherein $R^6$ is optionally substituted with one or more $R^7$ groups; wherein $R^7$ is independently selected from: $C_1$ to $C_{10}$ alkyl, -OH, $-OR^1$, $-NR^1_2$, - $NO_2$, -CN, -F, -Cl, -Br, and -I. In preferred embodiments, the compounds of any one of Formulae (II), (IIIa) and (IIIb) are selected from those wherein, $R^6$ is a phenyl group.

In some embodiments, the compounds of any one of Formulae (II), (IIIa) and (IIIb) are selected from those wherein, both $R^6$ groups are the same. In other embodiments, the compounds of any one of Formulae (II), (IIIa) and (IIIb) are selected from those wherein, both $R^6$ groups are the different.

The present invention also provides processes for synthesising the compounds of formulae (I), (II), (IIIa) and (IIIb).

In one aspect the present invention also provides a process for preparing a compound of formula (I) comprising the steps of:

i) providing a diamine $NH_2$-$L^1$-$NH_2$ wherein $L^1$ is as defined herein;
ii) adding at least one furfuraldehyde substituted with n $R^3$ groups, and at least one phenolic compound substituted with x R groups, wherein R, $R^3$, n and x are as defined herein, with the proviso that both positions ortho to the phenolic OH group are unsubstituted on the at least one phenolic compound; and
iii) forming a compound of formula (I).

Whilst the exact ratio of diamine to phenolic compound to furfuraldehyde used in this process is 1:2:4, differing molar ratios of these precursors may be utilised in some embodiments. Optionally, the molar ratio of the diamine to the at least one furfuraldehyde is from 1:2 to 1:20, preferably from 1:3 to 1:10, more preferably from 1:4 to 1:8; and/or wherein the molar ratio of the diamine to the at least one phenolic compound is from 1:1 to 1:5, preferably from 1:1.5 to 1:4, more preferably from 1:2 to 1:3.

There exists a wide range of orders of addition that fall within the scope of the processes for synthesising the compound of formula (I). Different orders of addition of the reactants may be used to favour specific products.

In one embodiment the diamine is contacted with the at least one furfuraldehyde prior to the addition of the at least one phenolic compound.

In one embodiment the diamine is contacted with a portion of the at least one furfuraldehyde prior to the addition of the at least one phenolic compound, wherein the remaining furfuraldehyde is added after and/or simultaneously to addition of the at least one phenolic compound.

In one embodiment the diamine is contacted with the at least one phenolic compound and the at least one furfuraldehyde simultaneously.

In one embodiment, two different phenolic compounds are added separately. This embodiment is suited to the preparation of asymmetrical benzoxazines.

In one embodiment, only one phenolic compound is added. This embodiment is suited to the preparation of symmetrical benzoxazines.

In another aspect, the present invention provides a process for preparing a compound of formula (II), (IIIa) or (IIIb) comprising the steps of:

i) providing A) a bisphenol compound comprising a linker $L^2$ and substituted with y R groups on each benzene ring of the bisphenol compound; B) a benzene-1,4-diol compound substituted with z R groups; or C) a benzene-1,3-diol optionally substituted with 1 R group at the 5-position; wherein y, z, R and $L^2$ are as defined herein;
ii) contacting the compound from step i) with at least one furfuraldehyde substituted with n $R^3$ groups, and at least one primary amine substituted with one $R^6$ group, wherein n, $R^3$ and $R^6$ are as defined herein; and
iii) forming a compound of formula (II), (IIIa), or (IIIb).

As described above, this process yields a compound of formula (II) when a bisphenol compound precursor is used, a compound of formula (IIIa) when a 1,3-benzene-diol is used, or a compound of formula (IIIb) when a 1,4-benzene-diol is used. In the aspects relating to formula (II), (IIIa) and (IIIb), two equivalents of primary amine are used in the preparation of the benzoxazine compound, the primary amine has one substituent defined herein as $R^6$. The primary amine becomes incorporated into the benzoxazine ring to provide the benzoxazine nitrogen substituted with $R^6$. A single primary amine may be used so that both $R^6$ groups are the same, or multiple primary amines may be used so that both $R^6$ groups are different from each other.

Whilst the exact ratio of phenolic compound or benzene-diol, to primary amine to furfuraldehyde used in this process is 1:2:4, differing molar ratios of these precursors may be utilised in some embodiments. Optionally, the molar ratio of the bisphenol compound or benzenediol to the at least one furfuraldehyde is from 1:2 to 1:20, preferably from 1:3 to 1:10, more preferably from 1:4 to 1:8; and/or wherein the molar ratio of the bisphenol compound or benzenediol to the at least one primary amine is from 1:1 to 1:5, preferably from 1:1.5 to 1:4, more preferably from 1:2 to 1:3.

There exists a wide range of orders of addition that fall within the scope of the processes for synthesising the compounds of formulae (II), (IIIa) and (IIIb). Different orders of addition of the reactants may be used to favour specific products.

In one embodiment the at least one primary amine, is contacted with the at least one furfuraldehyde prior to the addition of the bisphenol compound or benzenediol.

In one embodiment the at least one primary amine, is contacted with a portion of the at least one furfuraldehyde prior to the addition of the bisphenol compound or benzenediol, wherein the remaining furfuraldehyde is added after and/or simultaneously to addition of the bisphenol compound or benzenediol, respectively.

In one embodiment the at least one primary amine, is contacted with the bisphenol compound or benzenediol, and the at least one furfuraldehyde simultaneously.

In one embodiment two different primary amines are added separately. This embodiment is suited to the preparation of asymmetrical benzoxazines.

In one embodiment only one primary amine is added. This embodiment is suited to the preparation of symmetrical benzoxazines.

The compounds of the present invention may be prepared over a range of different temperatures that the skilled person would be familiar with from conventional Mannich chemistry and conventional benzoxazine preparations. The compounds of the present invention may, for instance, be prepared at room temperature and the process may be accelerated by heating and/or the use of a catalyst. Preferably, the temperature of the reaction is between 25 °C and 150 °C, more preferably the temperature of the reaction is between 40 °C and 100 °C. The reaction may optionally be catalysed wherein either: the catalyst is i) an acid catalyst, for example an acid catalyst selected from HCl, trifluoroacetic acid, methane sulphonic acid, p-toluenesulphonic acid, trifluoromethanesulphonic acid, benzoic acid and mixtures thereof; or ii) a basic catalyst, for example a basic catalyst selected from NaOH, $Na_2CO_3$, triethylamine, triethanolamine and mixtures thereof. A broad range of organic solvents are suitable for use in the reaction, for example MeCN, benzene, methanol, ethanol, IPA, butanol, chloroform, DCM, diethyl ether, DMF, dioxane, ethyl acetate, petroleum ether, kerosine, pentane, hexane, heptane, MTBE, NMP, THF, toluene, xylene and mixtures thereof. Toluene is the most preferred solvent for use in the reaction.

As would be appreciated, the Mannich reaction and benzoxazine cyclisation reaction produce water. The skilled person is capable of employing methods of removing water produced during the reaction along with any solvent used, in order to yield the final product, such as boiling, distillation, vacuum distillation, azeotroping, dean stark, precipitation etc.

In a yet further aspect, the present invention comprises a resin composition comprising a benzoxazine compound of formula (I), (II), (IIIa), or (IIIb), as described herein. Any suitable organic solvent may be used as a solvent or diluent in a resin composition of the present invention, for example MeCN, benzene, methanol, ethanol, IPA, butanol, chloroform, DCM, diethyl ether, DMF, dioxane, ethyl acetate, petroleum ether, kerosine, pentane, hexane, heptane, MTBE, NMP, THF, toluene, xylene and mixtures thereof. Toluene is a particularly suitable solvent or diluent for difunctional benzoxazine compounds and resins thereof of the present invention. As would be appreciated, methods of forming and handling a resin composition from a given monomeric compound would be known by the skilled person.

## Reinforced materials

The benzoxazine compounds of the present invention, or resins thereof are particularly useful in the manufacture of reinforced materials. In particular, thermoset polymer matrixes, such as fibre-reinforced plastic, comprising fibrous materials, for example aramid fibre, basalt, wood fibre, glass fibre, carbon fibre or flax. The material to be reinforced is contacted with a difunctional benzoxazine compound of the present invention, or resin thereof, to form a pre-preg, followed by curing of the difunctional benzoxazine compound by self-polymerisation to form cross-linkages, to thereby reinforce the material. A coating may be formed on a material by contacting the surface of the material with a difunctional benzoxazine compound of the present invention, or resin thereof, followed by allowing self-polymerisation of the difunctional

benzoxazine compound. Self-polymerisation of the difunctional benzoxazine compound can occur at ambient temperature or elevated temperature, with or without a catalyst or co-reactant.

Self-polymerisation of the difunctional benzoxazine also allows the compounds of the invention to be used for the purpose of liquid moulding, film adhesives, composite construction, material bonding and repair, as in the case of conventional benzoxazines. As the skilled person will appreciate from the present disclosure, the properties of the difunctional benzoxazine compounds also find particular utility in resin formation, resin polymerisation, and formation of reinforced materials such as fibre-reinforced plastic.

The polymer formed by self-polymerisation of the difunctional benzoxazine compounds of the present invention has exposed phenol groups, due to the benzoxazine ring opening reaction. This means that further cross-linkage or curing can be achieved using epoxy resin, the epoxide groups of which are capable of reacting with the exposed phenol groups. An epoxy-based resin may be applied to, or incorporated into, a difunctional benzoxazine compound of the present invention, or resin composition thereof, or a pre-preg or reinforced material formed therefrom, in order to provide further curing, hardening, cross-linkage and reinforcement. Hybridisation with epoxy-based resins may be used to increase the Tg of a benzoxazine thermoset, or material comprising or reinforced with the same.

Examples of epoxy-based resins suitable for use in the present invention include polyglycidyl ethers of polyhydric phenols, epoxidised novolacs or similar glycidated polyphenolic resins, glycidated bisphenols, such as glycidated bisphenol A or F, or halogenated (e.g. chlorinated or fluorinated) analogues thereof; polyglycidyl ethers of alcohols, glycols or polyglycols, and polyglycidyl esters of polycarboxylic acids. Preferred examples of epoxy resins are polyglycidyl ethers of a polyhydric phenol. Polyglycidyl ethers of polyhydric phenols can be produced, for example, by reacting an epihalohydrin with a polyhydric phenol in the presence of an alkali. Examples of suitable polyhydric phenols include: 2,2-bis (4-hydroxyphenyl) propane (bisphenol-A); 2,2-bis(4-hydroxy-3-tert-butylphenyl)propane; 1,1-bis(4-hydroxyphenyl) ethane; 1,1-bis(4-hydroxyphenyl) isobutane; bis(2-hydroxy-1-naphthyl) methane; 1,5-dihydroxynaphthalene; 1,1-bis(4-hydroxy-3-alkylphenyl) ethane and the like. Commercial examples of preferred epoxy resins that may be used include EPILOK 60-600.

The reinforced material may be prepared by applying a layer of a resin as defined herein, onto a bottom film carrier layer. Glass fibres, carbon fibres, flax, or any other fibrous material as the reinforcement may then then be applied to the upper surface of the resin on the film carrier. A further layer of the resin is applied to sandwich the fibrous material between the layers. A top film may be applied to the upper layer. The resulting layered composition may subsequently be compressed as part of forming the reinforced material.

As will be appreciated, in some embodiments, the reinforced material of the present invention may be formed from a sheet-molding compound (SMC) comprising a compound according to formula (I), (II), (IIIa), or (IIIb), or resin thereof, and fibrous material as defined above. Methods of preparing such SMC materials are known in the art and can be readily adapted for use in preparing reinforced materials as defined herein. The SMC material may further include additives selected from hardeners, accelerators, fillers, pigments, fire retardants and/or any other components, as required. For example, the sheet-form polymeric material may also include melamine, which is useful as a fire retardant. The SMC may include a further thermoplastic material.

In some embodiments, the reinforced material of the present invention may comprise a number of layers, the layers may be joined together in a variety of ways. For instance, where air-tight sealing coating material comprising an elastomer is used, the same coating material may be used to bond the first insulating layer to one or more adjacent layers of the layered composite material panel. Alternatively, or in addition, SMC material comprising a compound according to formula (I), (II), (IIIa), or (IIIb) may be bonded to one or more adjacent layers during curing of the reinforced material, for instance using heat and/or pressure. In addition, a variety of known adhesives may be used to bond the individual layers of the layered composite material panel together. Preferably, pressure is applied to the layered composite material during the bonding step so as to ensure good adhesion of the layers. As noted above, where one or more layers comprises a compound according to formula (I), (II), (IIIa), or (IIIb), or resin thereof, (e.g. as part of an SMC material) the application of pressure may also assist in the curing of the compound according to formula (I), (II), (IIIa), or (IIIb), or resin thereof.

The invention will now be described by reference to the following non-limiting Examples and the Figures.

## EXAMPLES

### EXAMPLE 1 - Preparation of Compound A according to Formula (I)

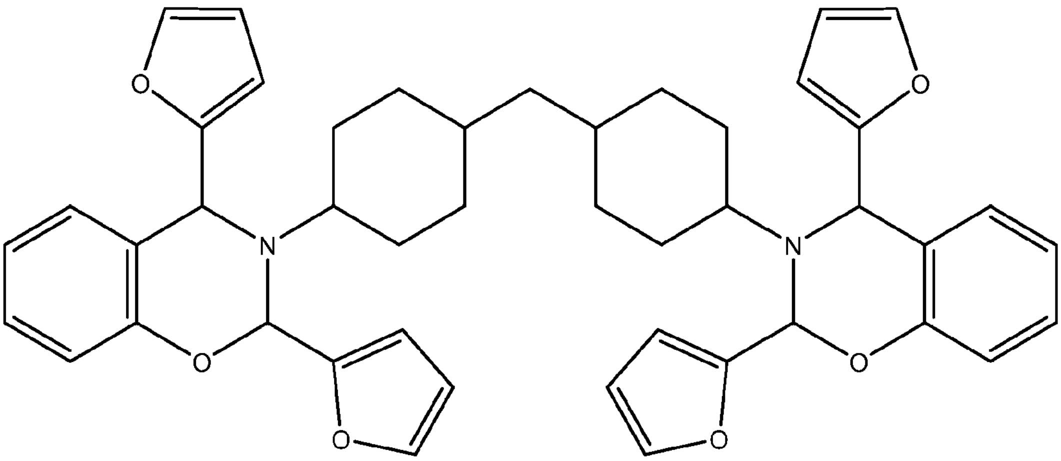

Compound A

184 grams of toluene was added to a 1 litre, round-bottomed flask equipped with a condenser, mechanical stirrer, addition funnel and thermometer. 384 grams [4 Mols] of unsubstituted furfuraldehyde was added and the agitator started. 210 grams [1 Mol] of 4,4-Diaminodicyclohexylmethane, was added to the flask over a 60-minute period controlling the temperature below 60 °C throughout the addition.

188 grams of phenol [2 Mols] was added in 8 aliquots maintaining the temperature below 50 °C throughout the 30-minute period. The mixture was held for a further 30 minutes before being heated to azeotrope up to 120 °C removing approximately 72 grams of water formed during the reaction.

The flask was then reconfigured for vacuum distillation and distilled under vacuum to 120 °C recovering the toluene [182 grams recovered] to yield Compound A in a liquid form.

**EXAMPLE 2 - Preparation of Compound B according to Formula (II)**

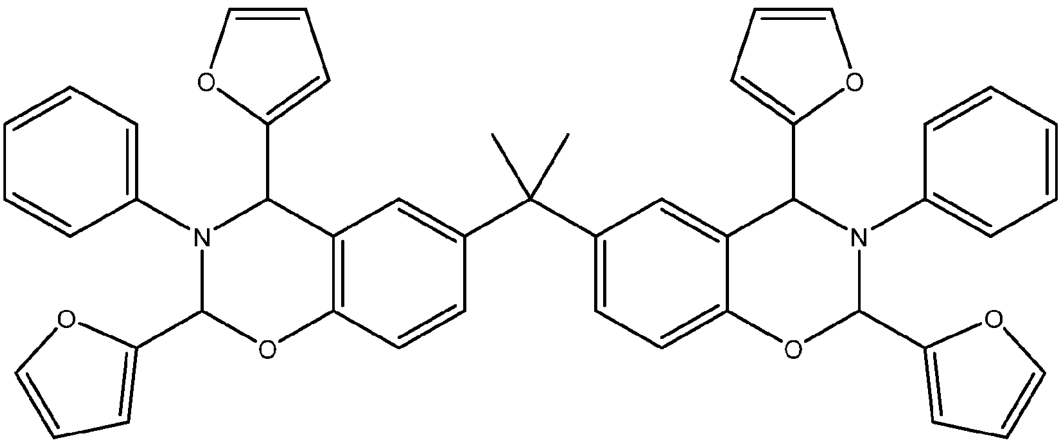

Compound B

184 grams toluene was added to a 1 litre, round-bottomed flask equipped with a dean and stark separator, condenser, mechanical stirrer, addition funnel and thermometer. 228 grams of bisphenol A [1 Mole] was added to the flask along with 384 grams [4 Mols] of unsubstituted furfuraldehyde and the agitator started. A nitrogen atmosphere was applied and aniline 186 grams [2 Moles] added in 4 aliquots over a period of 60 minutes. The temperature was maintained between 43 - 47 °C throughout the addition.

Following the addition of the aniline the flask was heated to 85 °C and held 1 hour. It was then azeotroped up to 110 °C removing approximately 71 grams water formed during the reaction. The Flask was then reconfigured for vacuum distillation and distilled under vacuum to 120 °C recovering the toluene [181 grams recovered] to yield Compound B in a

liquid form.

**EXAMPLE 3 - Preparation of Compound C according to Formula (IIIa)**

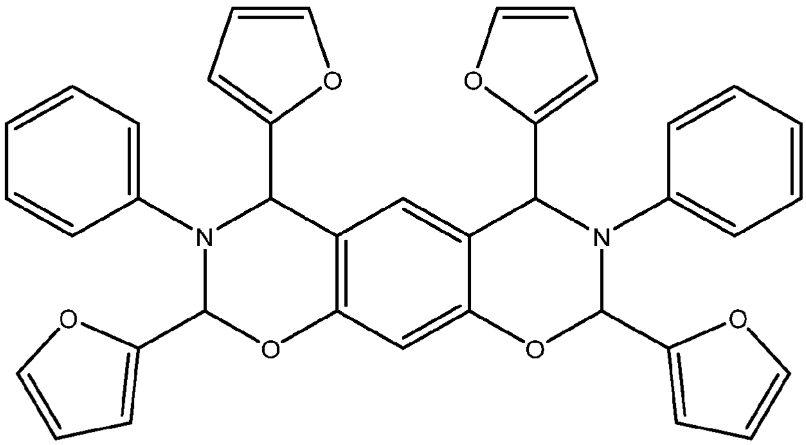

Compound C

184 grams of toluene was added to a 1 litre, round-bottomed flask equipped with a dean and stark separator, condenser, mechanical stirrer, addition funnel and thermometer. 110 grams of resorcinol [1 Mole] was added to the flask along with 384 grams [4 Mols] of unsubstituted furfuraldehyde and the agitator started. A nitrogen atmosphere was applied and aniline 186 grams [2 Moles] added in 4 aliquots over a period of 60 minutes. The temperature was maintained between 45 - 50 °C throughout the addition.

Following the addition of the aniline the flask was heated to 85 °C and held 1 hour. It was then azeotroped up to 110 °C removing approximately 72 grams water formed during the reaction. The flask was then reconfigured for vacuum distillation and distilled under vacuum to 120 °C recovering the toluene [182 grams recovered] to yield Compound C in a liquid form.

**EXAMPLE 4 - Preparation of Compound D according to Formula (I)**

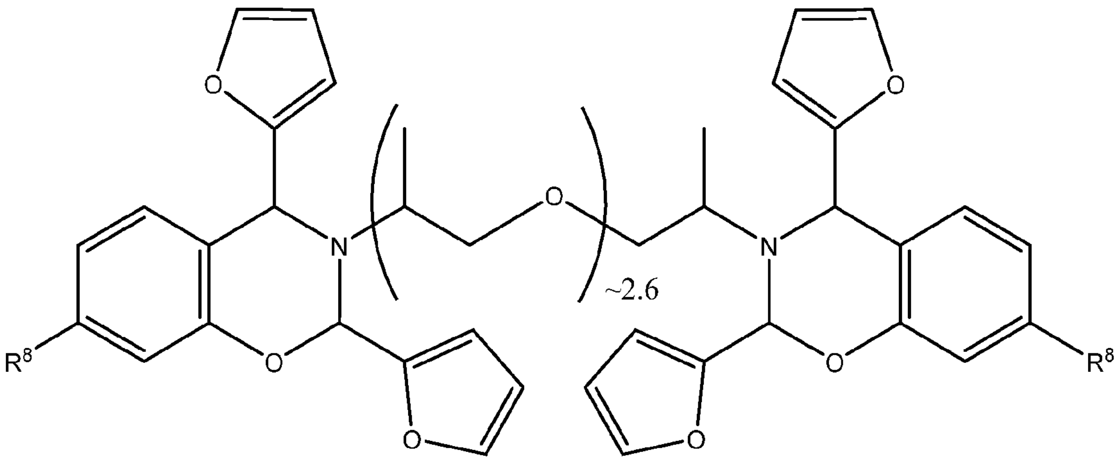

Compound D

Wherein $R^8$ is independently selected from a $-C_{15}H_{31}$ straight chain alkyl group, $-C_{15}H_{29}$ straight chain alkenyl group, $-C_{15}H_{27}$ straight chain alkenyl group, and $-C_{15}H_{25}$ straight chain alkenyl group.

184 grams of toluene was added to a 1 litre, round-bottomed flask equipped with a dean and stark separator, condenser, mechanical stirrer, addition funnel and thermometer. 230 grams of Jeffamine D230 (RTM) [1 Mole] was added

to the flask along with 384 grams [4 Mols] of unsubstituted furfuraldehyde and the agitator started. A nitrogen atmosphere was applied and 660 grams [2 Moles] of cardanol added. The temperature was maintained <75 °C throughout the additions.

Following the addition of the cardanol the flask was heated to 85 °C and held 1 hour. It was then azeotroped up to 110 °C removing approximately 71 grams of water formed during the reaction. The flask was then reconfigured for vacuum distillation and distilled under vacuum to 120 °C recovering the toluene [180 grams recovered] to yield Compound D in a liquid form.

**Example 5 - Comparison of physical properties against known benzoxazines**

**Table 4. Comparison of the properties of Compounds A to D with those of a convention benzoxazine (Cardarez R111)**

| Compound | Colour (ASTM D1544-04(2018)) | Plate gel at 200 °C / minutes (ASTM D3056-14(2018)) | Viscosity at 25 °C / Pa·S (ASTM D2556-14(2018)) | Tg / °C (cured at 180 °C for 2 hours, and then 200 °C for 4 hours) | Bio-carbon content / % |
|---|---|---|---|---|---|
| Compound A | 18 (black) | 6:55 | 115 | 190 | 44 |
| Compound B | 18 (black) | 7:22 | 215.7 | 128 | 43 |
| Compound C | 18 (black) | 7:18 | 345.2 | 114 | 53 |
| Compound D | 18 (black) | 9:18 | 11.8 | 123 | ~85 |
| Cardarez R111 (RTM) | 18 (black) | 12:00 | 3* | 160 | 0 |

* Solution viscosity is undertaken as a 50% weight/weight solution in toluene as Cardarez R111 (RTM) is a solid.

The results in Table 4 demonstrate the advantages of the benzoxazine compounds of the invention. The benzoxazines of the present invention provide a significant bio-carbon contents, exceeding 40% in each case, meaning that the compounds can help meet environmental targets in the applications in which they are deployed and are produced using renewal feedstocks.

The benzoxazines of the present invention also have advantageously low viscosities which provides for easier handling during preparation, storge and use, as well as reducing the need for diluents or solvents in a resin composition. Conventional benzoxazine, Cardarez R111 (RTM), is a solid, and as such no directly comparable viscosity measurement can be taken at 25 °C. However, Figure 1 demonstrates the viscosity (mPa. s) v temperature (°C) of Cardarez R111 (RTM), using ASTM D4287-00(2019). Due to the solid nature of Cardarez R111 (RTM) viscosity measurements must be taken at high temperatures in order to melt the Cardarez R111 (RTM). It can be seen from Figure 1 that temperatures as high as 120 °C to 140 °C are required before the viscosity of Cardarez R111 (RTM) is at a comparable order of magnitude to the viscosities of compounds A to D at 25 °C.

Figure 2 demonstrates viscosity (mPa. s) development over time (hours) of Cardarez R111 (RTM) at 100 °C, 120 °C and 140°C. Cardarez R111 (RTM) is a thermoset and reactivity rate [devoid of catalysis or co-polymer] is temperature dependent and depicted in Figure 2. Cardarez R111 (RTM) stability evaluation undertaken with a 50 gram mass in all cases. Viscosity figures quoted are evaluated and detailed at the temperature that the material was held. Viscosity evaluation was undertaken using a cone and plate viscometer [CPV] and the test temperature for viscosity evaluation is the same as the designated stability test temperature[s].

As will be appreciated, each of the compounds of the invention exhibits a significantly shorter gel time (as measured by ASTM D3056-14(2018)) in comparison to conventional benzoxazine, Cardarez R111. Relatively slow curing times are a known deficiency associated with conventional benzoxazines. The benzoxazines of the present invention provide surprising improvements in gel time, indicating that the benzoxazines of the present invention exhibit greater reactivity, leading to improvements in process efficiency in applications where benzoxazines are employed.

Figure 3 and Figure 4 compare the gel-time reactivity (gel time / minutes) undertaken with a 1 gram mass of Cardarez R111 (RTM) and Compound A, respectively. Cure determination by plate gel at specified temperatures (°C). The

gel time is representative of the progress of curing by self-polymerisation. No catalyst or co-reactant was used. Figures 3 and 4 are in contrast to the plate gel data (ASTM D3056-14(2018)) shown in Table 4. The ASTM D3056-14(2018) method used in Table 4 uses a catalyst, whereas the experiments shown in Figures 3 and 4 were performed in the absence of a catalyst. As can be seen, Cardarez R111 (RTM) is less amenable to catalysis as compared to the compounds of the invention which are particularly well suited to catalysed curing. The addition of the catalyst to the compounds of the present invention provides for a much greater reduction in gel time. Without being bound to any particular theory, it is expected that the furan groups on the benzoxazine ring assist in co-ordinating the catalyst to bring it into closer proximity with the benzoxazine ring to stimulate ring opening.

The results in Table 4 also show that glass transition temperatures compare favourably with the conventional benzoxazine (Cardarez R111) or, in the case of Compound A, are improved over the conventional benzoxazine.

Figure 5 demonstrates the Tg development (°C) of thermosets cured at 180 °C vs time (minutes) for a Compound A thermoset and a comparative Cardarez R111 (RTM) thermoset. As can be seen, Compound A provides superior Tg development over time and thus is able to produce thermosets of a higher Tg once sufficient cure time is applied. As would be appreciated, cure times of 90 minutes or greater are commonly used in the art for various applications.

It has also been demonstrated that the benzoxazine compounds of the present invention are particularly well suited to curing with a ferric chloride catalyst, although any suitable Lewis acid catalyst may also be used. Figure 6 demonstrates the Tg development (°C) of Compound A during curing at 140 °C over 1 hour with 3%, 4% or 5% by weight of a ferric chloride catalyst. Increased concentration of catalyst can clearly be seen to accelerate curing in order to rapidly provide thermosets of higher Tg.

Similarly, Figure 7 demonstrates the Compound A viscosity build (Pa· s) vs time (minutes) at 60 °C and 100 °C, with 5% ferric chloride catalyst. The 5% ferric chloride allows for curing to take place at significantly lower temperatures. Cardarez R111 (RTM) is a solid and so must be heated to a high temperature and melted before a catalyst may be mixed with the Cardarez R111 (RTM). This means that the catalysis of a solid benzoxazine such as Cardarez R111 (RTM) requires addition of the catalyst at high temperature followed by mixing before it can be applied in the desired use. This risks premature curing and causes difficulty in handling and storage. The benzoxazine compounds of the present invention, however, are liquids at room temperature (e.g. 25 °C) and so may be mixed with a catalyst at comparatively low temperature without the risk of premature curing.

As can be seen from Figure 7, the viscosity build is very slow at 60 °C, which demonstrates the utility for storage and handling of Compound A pre-mixed with catalyst at low temperature. Additionally, rapid curing can nevertheless be achieved, once desired, by heating to a higher temperature, *e.g.* 100 °C. This is not possible for a solid benzoxazine which cannot be pre-mixed with a catalyst prior to melting at high temperature.

**Example 6 - Preparation of reinforced material**

A prepreg composite material was provided, in which a reinforcement fibre is preimpregnated with a resin composition comprising Compound A. Compound A is a viscous liquid and can be commercially processed via hot-melt or solvated and processed through conventional methods.

The material was used to make a composite part produced by a standard prepreg stage and pressing at a consolidation pressure 80 bar/$cm^2$. Each composite part was generated using glass fabric with fibre density - binder ratio of 60:40 by weight: E-glass fabric, Style 7628 Finish phenolic compatible.

The cure schedule employed was heating at a rate of 8 °C/minute from RT up to 180 °C, followed by holding at 180 °C for 3 hours under a consolidation pressure of 80 bar/$cm^2$.

The resultant composite panel had excellent surface appearance, free from defects such as porosity. The resultant product is suitable for use with a variety of reinforcements finding application in numerous areas including but not limited to, aircraft interiors and flooring, aerospace components, cargo liners, automotive parts, ballistic components, electrical laminates, fire resistant laminates and panels, sporting goods, train components, and tooling.

**Example 7 - Sample laminate properties**

The physical properties, and chemical resistance properties of a reinforced material according to Example 6 were measured and compared to a comparative reinforced material produced by the same method of Example 6 using a known benzoxazine (Cardarez R111 (RTM))

**Table 5 - Reinforced material physical properties comparison**

| Physical Property | Cardarez R111 (RTM) | Compound A |
|---|---|---|
| Tg / °C | 160 | 190 |
| Flexural strength / MPa | 580 | 550 |

(continued)

| Physical Property | Cardarez R111 (RTM) | Compound A |
|---|---|---|
| Flexural modulus / GPa | 27 | 25 |
| Impact strength / $KJM^2$ | 48 | 50 |
| UL 94 evaluation | - | V0 |

Chemical resistance of the reinforced material of the invention vs a comparative reinforced material was determined by weight change following submersion of the materials. The samples were submerged in the given chemical for 4 weeks at 25 °C.

**Table 6 - Reinforced material chemical resistance comparison**

| Chemical submersion | Cardarez R111 (RTM) Weight gain/loss / % | Compound A Weight gain/loss / % |
|---|---|---|
| Acetone | -7.00 | +4.58 |
| Butyl acetate | -1.23 | +3.36 |
| Ethylene diamine | Destroyed | Destroyed |
| Methanol | -0.64 | +5.61 |
| Nitric acid 20 % | -0.74 | +0.47 |
| Sulphuric acid 50 % | -3.08 | +1.24 |
| Xylene | -0.42 | +1.39 |

As can be seen from Table 5, the reinforced material made with compound A, has comparable strength and flexibility to that of a reinforced material produced with Cardarez R111 (RTM). The reinforced material made with Compound A is also shown to have a high degree of fire resistance. As can be seen from Table 6, the reinforced material made with Compound A, also has comparable chemical resistance to that of a reinforced material produced with Cardarez R111 (RTM). A very similar degree of weight change (indicative of chemical degradation) is generally observed as a result of chemical exposure.

The present invention thus allows for the provision of reinforced materials comprising a high degree of bio-carbon from renewable stocks without negatively impacting the physical properties or chemical resistance.

**Example 8 - Effects of catalysis**

*Effect of Catalyst on Gel Time*

The effects of catalysis on gel time were investigated by measuring the gel time of a solution of 90% Compound A and 10% acetone with various concentrations of a curing catalyst at 160 °C. The catalyst used was $BCl_3$ octyl dimethylamine complex and its relative concentration was measured in parts per hundred (PHR) with respect to Compound A.

A solution of $BCl_3$ octyl dimethylamine complex in ethanol was added to the solution of Compound A and mixed for 5 minutes with a slow rate agitator to form a homogenous solution without introducing air. The combined material had a viscosity of 1180 mPa·s at 25 °C. The gel times of Compound A in combination with concentrations of 1, 1.5 and 2 PHR of $BCl_3$ octyl dimethylamine complex at 160 °C were found to be approximately 320 seconds, 180 seconds, and 60 seconds, respectively. As would be expected, increasing the concentration of catalyst decreases the gel time, and these results are shown graphically in Figure 8.

*Preparation of Reinforced Material Using a Catalyst*

A combined solution of 90% Compound A and 10% acetone combined with 1 PHR of $BCl_3$ octyl dimethylamine complex was used to prepare a prepreg material. The combined solution was applied via a conventional process of impregnation of an E-glass fabric cloth (Style 7628 - finish phenolic compatible) having a fibre density of 70 % by weight, with the solution followed by driving off the solvent and partially advancing the resin to form a partially cured state or state of advancement/cure that allows the sheets to have sufficient strength so that they can be laid up as a laminate (prepreg layer stack), but not so advanced that they are rigid or fail to flow when pressed under heat and pressure. The sheets were cut and then aligned so they can be consolidated, and the cure schedule employed was heating at a rate of 8 °C/minute up to 170 °C, followed by heating at 170 °C for two hours, followed by heating at a rate of 8 °C/minute up to 190 °C under a consolidation pressure of 80 bar/$cm^2$. The resulting laminate had the following properties:

**Table 7 - Catalysed reinforced material physical properties**

| Physical Property | Compound A (1 PHR Catalyst) | Testing Method |
|---|---|---|
| Density / g/cm$^3$ | 1.95 | ISO 1183 |
| Flexural strength / MPa | 500 | ISO 178 |
| E-modulus (flexural strength) / GPa | 35 | ISO 178 |
| Tensile strength / MPa | 400 | ISO 527 |
| Compression strength perpendicular to lamination / MPa | 500 | ISO 604 |
| Breakdown voltage / KV (d=25mm)* | 75 | IEC 60243-1 |
| Flammability 5mm | V0** | UL 94 |

* (d=25mm) denotes that both electrodes have a 25 mm diameter.
** V0 = Burning stops within 10 seconds after two applications of ten seconds each of a flame/vertical burn to a test bar. No flaming drips are observed.

Thus, the present invention is highly compatible with curing catalysts and may readily provide reinforced materials having desirable properties derived from the compounds described herein, prepared with the assistance of a curing catalyst.

## Claims

**1.** A compound of formula (I):

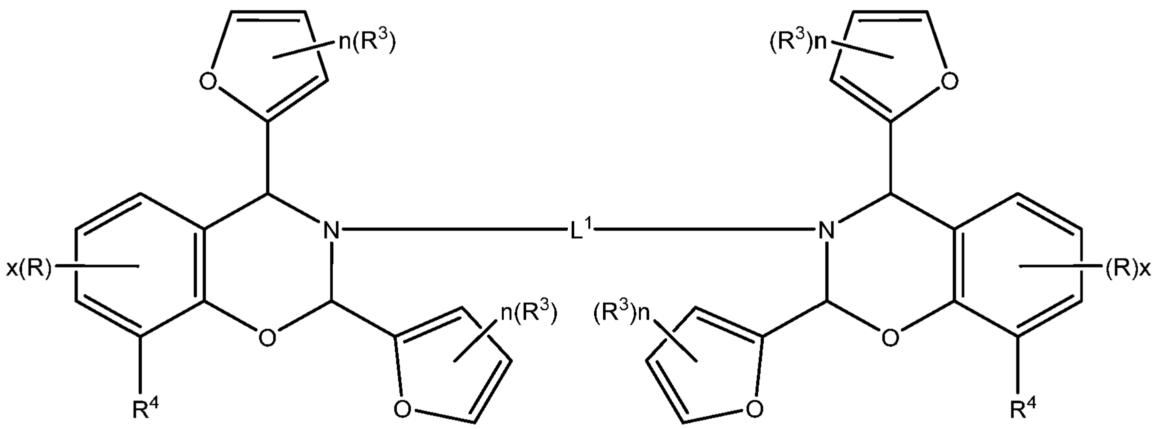

(I)

wherein each R group is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{10}$ cycloalkyl, $C_1$ to $C_{20}$ alkyl-$C_3$ to $C_{10}$ heterocycloalkyl, $C_1$ to $C_{20}$ alkyloxy, $C_1$ to $C_{20}$ alkylamino, -OH, -OR$^1$, -NH$_2$, -NHR$^1$, -NR$^1_2$, -C(O)OH, -C(O)OR$^1$, -C(O)NH$_2$, - C(O)NHR$^1$, -C(O)NR$^1_2$, -O(CO)H, -O(CO)R$^1$, -NH(CO)H, -NH(CO)R$^1$, -NR$^1$(CO)H, - NR$^1$(CO)R$^1$, -SH, -SR$^1$, -SO$_2$H, -SO$_2$R$^1$, -SO$_3$R$^1$, -SO$_3$H, -SiR$^1_3$, -NO$_2$, -CN, -F, -Cl, -Br, - I, $C_6$ to $C_{14}$ aryl optionally substituted with one or more R$^2$ group, and $C_2$ to $C_9$ heteroaryl optionally substituted with one or more R$^2$ group;
wherein each R$^1$ is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{10}$ cycloalkyl, $C_1$ to $C_{20}$ alkyl-$C_3$ to $C_{10}$ heterocycloalkyl, $C_1$ to $C_{20}$ alkoxy, $C_1$ to $C_{20}$ alkylamino, $C_6$ to $C_{14}$ aryl, and $C_2$ to $C_9$ heteroaryl;
wherein R$^2$ is independently selected from: $C_1$ to $C_{20}$ alkyl, $C_1$ to $C_{20}$ haloalkyl, $C_3$ to $C_{20}$ cycloalkyl, $C_2$ to $C_{20}$ alkenyl, $C_2$ to $C_{20}$ alkynyl, -OH, -NH$_2$, -OC$_1$ to $C_{20}$ alkyl, -NHC$_1$ to $C_{20}$ alkyl, -N($C_1$ to $C_{20}$ alkyl)$_2$, -NO$_2$, -CN, -F, -Cl, -Br, and -I;
wherein R$^3$ is independently selected from: $C_1$ to $C_6$ alkyl, $C_1$ to $C_6$ alkenyl, $C_1$ to $C_6$ alkoxy, $C_1$ to $C_6$ alkyloxy, -C(O)OH, -C(O)O-$C_1$ to $C_6$ alkyl, $C_1$ to $C_6$ alkyl-(O)O-H, $C_1$ to $C_6$ alkyl-(O)O-$C_1$ to $C_6$ alkyl, and -OH;
wherein R$^4$ is hydrogen;

wherein $L^1$ is a divalent hydrocarbyl linker comprising 1 to 50 carbon atoms;
wherein x is independently 0, 1, 2 or 3; and
wherein n is independently 0, 1, 2 or 3.

**2.** The compound of Claim 1, wherein:

a) one or both benzene rings of the benzoxazines are substituted with R groups at the 3-position and/or the 5-position, relative to the oxygen of the benzoxazine ring;
b) both x values ≤ 2, preferably wherein both x values ≤ 1, and preferably wherein both x values are the same, and/or wherein both benzene rings of the benzoxazines are substituted with the same R group(s), more preferably wherein both x values = 0;
c) $L^1$ comprises 2 to 50 carbon atoms, 3 to 50 carbon atoms, 5 to 50 carbon atoms, 2 to 40 carbon atoms, 3 to 40 carbon atoms, 5 to 40 carbon atoms, 2 to 30 carbon atoms, 3 to 30 carbon atoms, 5 to 30 carbon atoms, 2 to 20 carbon atoms, 3 to 20 carbon atoms, or 5 to 20 carbon atoms;
d) $L^1$ is derived from the reaction of a diamine $NH_2$-$L^1$-$NH_2$, when $NH_2$-$L^1$-$NH_2$ is selected from: ethylene diamine, diethylene triamine, triethylene tetramine, hexamethylene diamine, 2,2,4-trimethyl hexamethylene diamine, 2,4,4-trimethyl hexamethylene diamine, a Jeffamine, metaxylylene diamine, 1,3-bis(aminomethyl) cyclohexane, benzene-1,4-diamine, isophorone diamine, diaminocyclohexane, 4-Cyclohexene-1,2-diamine, 3-Hexyne-1,6-diamine, bis-(p-aminocyclohexyl) methane or methylene dianiline; preferably when $NH_2$-$L^1$-$NH_2$ is selected from: ethylene diamine, hexamethylene diamine, 2,2,4-trimethyl hexamethylene diamine, 2,4,4-trimethyl hexamethylene diamine, a Jeffamine, metaxylylene diamine, 1,3-bis(aminomethyl) cyclohexane, benzene-1,4-diamine, isophorone diamine, diaminocyclohexane, bis-(p-aminocyclohexyl) methane and methylene dianiline;
e) the compound has the structure:

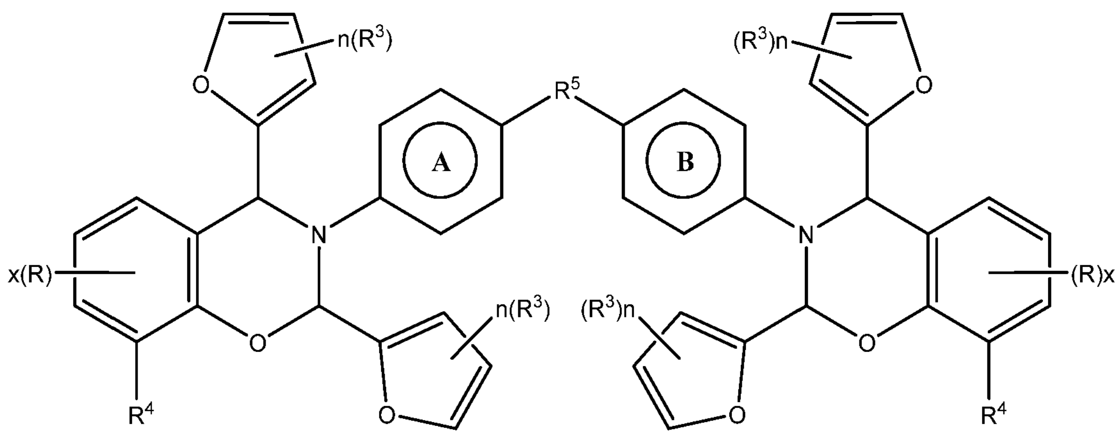

wherein ring A and ring B are independently either aromatic or saturated 6 membered carbon rings, preferably wherein rings A and B are both saturated rings; wherein $R^5$ is a divalent $C_1$ to $C_{10}$ alkyl group, preferably wherein $R^5$ is a methylene group; and wherein R, $R^3$, x, and n are independently as defined in Claim 1, preferably where both x values = 0 and/or wherein all n values = 0;
and/or
f) $L^1$ comprises at least one of:

i) a heteroatom selected from nitrogen, oxygen, sulphur and silicon;
ii) a double or triple carbon-carbon bond; or
iii) an aromatic ring, heteroaromatic ring, and/or non-aromatic ring.

**3.** A compound of formula (II):

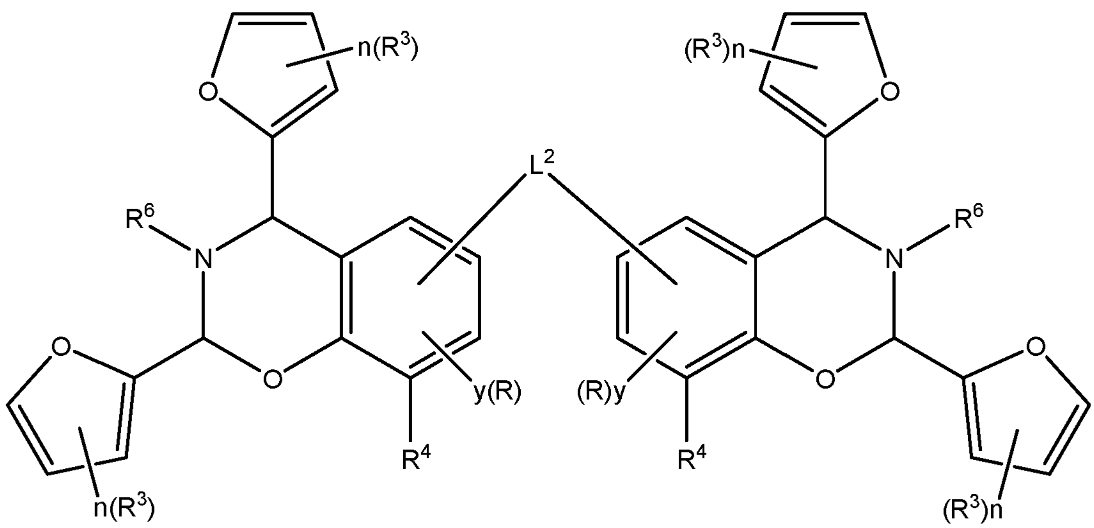

(II)

wherein each R group is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{20}$ cycloalkyl, $C_1$ to $C_{20}$ alkyl-$C_3$ to $C_{10}$ heterocycloalkyl, $C_1$ to $C_{20}$ alkyloxy, $C_1$ to $C_{20}$ alkylamino, -OH, -$OR^1$, -$NH_2$, -$NHR^1$, -$NR^1_2$, -C(O)OH, -C(O)$OR^1$, -C(O)$NH_2$, - C(O)$NHR^1$, -C(O)$NR^1_2$, -O(CO)H, -O(CO)$R^1$, -NH(CO)H, -NH(CO)$R^1$, -$NR^1$(CO)H, - $NR^1$(CO)$R^1$, -SH, -$SR^1$, -$SO_2H$, -$SO_2R^1$, -$SO_3R^1$, -$SO_3H$, -$SiR^1_3$, -$NO_2$, -CN, -F, -Cl, -Br, - I, $C_6$ to $C_{14}$ aryl optionally substituted with one or more $R^2$ group, and $C_2$ to $C_9$ heteroaryl optionally substituted with one or more $R^2$ group;

wherein each $R^1$ is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{10}$ cycloalkyl, $C_1$ to $C_{20}$ alkyl-$C_3$ to $C_{10}$ heterocycloalkyl, $C_1$ to $C_{20}$ alkoxy, $C_1$ to $C_{20}$ alkylamino, $C_6$ to $C_{14}$ aryl, and $C_2$ to $C_9$ heteroaryl;

wherein $R^2$ is independently selected from: $C_1$ to $C_{20}$ alkyl, $C_1$ to $C_{20}$ haloalkyl, $C_3$ to $C_{20}$ cycloalkyl, $C_2$ to $C_{20}$ alkenyl, $C_2$ to $C_{20}$ alkynyl, -OH, -$NH_2$, -$OC_1$ to $C_{20}$ alkyl, -$NHC_1$ to $C_{20}$ alkyl, -N($C_1$ to $C_{20}$ alkyl)$_2$, -$NO_2$, -CN, -F, -Cl, -Br, and -I;

wherein $R^3$ is independently selected from: $C_1$ to $C_6$ alkyl, $C_1$ to $C_6$ alkenyl, $C_1$ to $C_6$ alkoxy, $C_1$ to $C_6$ alkyloxy, -C(O)OH, -C(O)O-$C_1$ to $C_6$ alkyl, $C_1$ to $C_6$ alkyl-(O)O-H, $C_1$ to $C_6$ alkyl-(O)O-C, to $C_6$ alkyl, and -OH;

wherein $R^4$ is hydrogen;

wherein $R^6$ is independently a monovalent group selected from -H, -OH, or a hydrocarbyl group comprising 1 to 50 carbon atoms;

wherein $L^2$ is a direct bond, a divalent group selected from: C(O), O, S, S(O), S(O)$_2$, or a hydrocarbyl linker comprising 1 to 50 carbon atoms;

wherein y is independently 0, 1, or 2; and

wherein n is independently 0, 1, 2 or 3.

**4.** The compound of Claim 3, wherein $L^2$ is attached at the 4-position, relative to the benzoxazine oxygen; and/or wherein $L^2$ is selected from S(O)$_2$, $CH_2$, C($CH_3$)$_2$, or a divalent benzene ring; and/or
wherein both y values ≤ 1, and preferably wherein both y values are the same, and/or wherein both benzene rings of the benzoxazines are substituted with the same R group(s), more preferably wherein both y values = 0.

**5.** A compound of either formula (IIIa) or formula (IIIb):

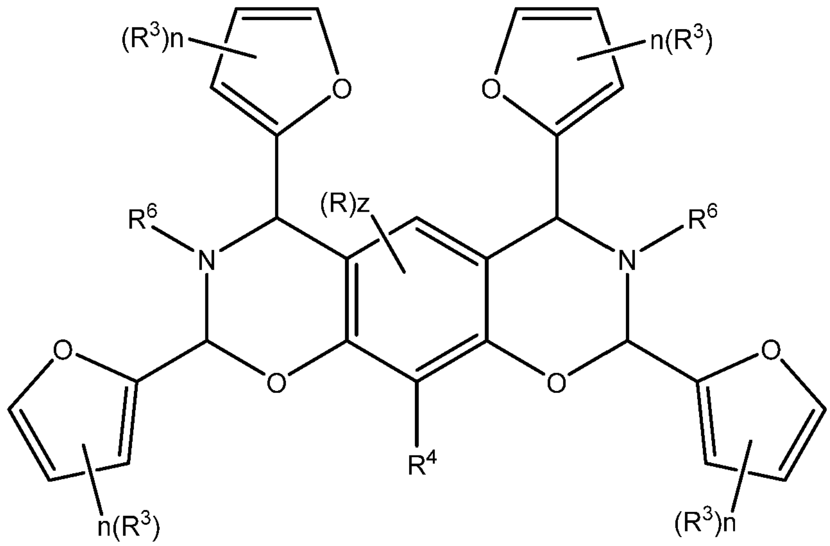

(IIIa)

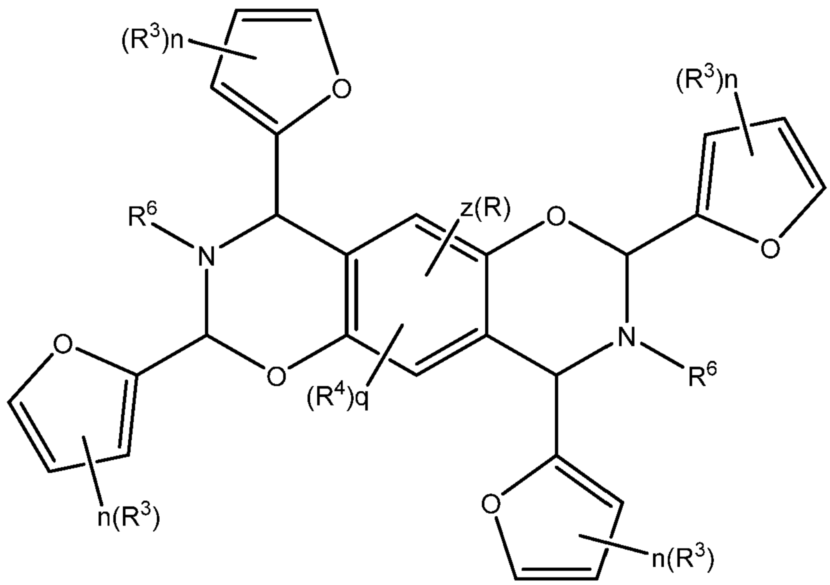

(IIIb)

wherein R is selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{10}$ cycloalkyl, $C_1$ to $C_{20}$ alkyl-$C_3$ to $C_{10}$ heterocycloalkyl, $C_1$ to $C_{20}$ alkyloxy, $C_1$ to $C_{20}$ alkylamino, -OH, -$OR^1$, -$NH_2$, -$NHR^1$, -$NR^1_2$, -C(O)OH, -C(O)$OR^1$, -C(O)$NH_2$, -C(O)$NHR^1$, -C(O)$NR^1_2$, - O(CO)H, -O(CO)$R^1$, -NH(CO)H, -NH(CO)$R^1$, -$NR^1$(CO)H, -$NR^1$(CO)$R^1$, -SH, -$SR^1$, - $SO_2H$, -$SO_2R^1$, -$SO_3R^1$, -$SO_3H$, -$SiR^1_3$, -$NO_2$, -CN, -F, -Cl, -Br, -I, $C_6$ to $C_{14}$ aryl optionally substituted with one or more $R^2$ group, and $C_2$ to $C_9$ heteroaryl optionally substituted with one or more $R^2$ group;

wherein each $R^1$ is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{10}$ cycloalkyl, $C_1$ to $C_{20}$ alkyl-$C_3$ to $C_{10}$ heterocycloalkyl, $C_1$ to $C_{20}$ alkoxy, $C_1$ to $C_{20}$ alkylamino, $C_6$ to $C_{14}$ aryl, and $C_2$ to $C_9$ heteroaryl;

wherein $R^2$ is independently selected from: $C_1$ to $C_{20}$ alkyl, $C_1$ to $C_{20}$ haloalkyl, $C_3$ to $C_{20}$ cycloalkyl, $C_2$ to $C_{20}$ alkenyl, $C_2$ to $C_{20}$ alkynyl, -OH, -$NH_2$, -$OC_1$ to $C_{20}$ alkyl, -$NHC_1$ to $C_{20}$ alkyl, -N($C_1$ to $C_{20}$ alkyl)$_2$, -$NO_2$, -CN, -F, -Cl, -Br, and -I;

wherein $R^3$ is independently selected from: $C_1$ to $C_6$ alkyl, $C_1$ to $C_6$ alkenyl, $C_1$ to $C_6$ alkoxy, $C_1$ to $C_6$ alkyloxy, -C(O)OH, -C(O)O-$C_1$ to $C_6$ alkyl, $C_1$ to $C_6$ alkyl-(O)O-H, $C_1$ to $C_6$ alkyl-(O)O-$C_1$ to $C_6$ alkyl, and -OH;

wherein $R^4$ is hydrogen;
wherein $R^6$ is independently a monovalent group selected from -H, -OH, or a hydrocarbyl group comprising 1 to 50 carbon atoms;
wherein z is 0 or 1;
wherein q = 2 - z; and
wherein n is independently 0, 1, 2 or 3.

**6.** The compound of any preceding claim, wherein:

1) each $R^3$ is independently selected from: $C_1$ to $C_6$ alkyl, $C_1$ to $C_6$ alkoxy, $C_1$ to $C_6$ alkyloxy, and -OH, preferably $-CH_3$, $-CH_2OH$, or $-CH_2OCH_3$;
2) all n values ≤ 1, and preferably wherein all n values are the same, and/or wherein all $R^3$ groups are the same, and/or wherein all $R^3$ groups are attached at the position ortho to the furan oxygen; more preferably wherein all n values = 0; and/or
3) each R group is independently selected from: $C_1$ to $C_{20}$ alkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{10}$ cycloalkyl, $C_1$ to $C_{20}$ alkyl-$C_3$ to $C_{10}$ heterocycloalkyl, $C_1$ to $C_{20}$ alkyloxy, $C_1$ to $C_{20}$ alkylamino, -OH, -$OR^1$, $-NH_2$, $-NHR^1$, $-NR^1_2$, -O(CO)H, -O(CO)$R^1$, -NH(CO)H, -NH(CO)$R^1$, -$NR^1$(CO)H, - $NR^1$(CO)$R^1$, -SH, -$SR^1$, -$SiR^1_3$, -F, -Cl, -Br, -I, $C_6$ to $C_{14}$ aryl optionally substituted with one or more $R^2$ group, and $C_2$ to $C_9$ heteroaryl optionally substituted with one or more $R^2$ group;
wherein $R^1$ is independently selected from: $C_1$ to $C_{20}$ alkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyl-$C_6$ to $C_{14}$ aryl, $C_1$ to $C_{20}$ alkyl-$C_2$ to $C_9$ heteroaryl, $C_1$ to $C_{20}$ alkyl-$C_3$ to $C_{20}$ cycloalkyl, $C_1$ to $C_{20}$ alkyl-$C_3$ to $C_{10}$, heterocycloalkyl, $C_1$ to $C_{20}$ alkoxy, $C_1$ to $C_{20}$ alkylamino, $C_6$ to $C_{14}$ aryl, and $C_2$ to $C_9$ heteroaryl; and
wherein $R^2$ is independently selected from: $C_1$ to $C_{20}$ alkyl, $C_3$ to $C_{20}$ cycloalkyl, $C_2$ to $C_{20}$ alkenyl, $C_2$ to $C_{20}$ alkynyl, -OH, $-NH_2$, -$OC_1$ to $C_{20}$ alkyl, -$NHC_1$ to $C_{20}$ alkyl, -N($C_1$ to $C_{20}$ alkyl$)_2$, -F, -Cl, -Br, or -I:

preferably wherein each R group is independently selected from: $C_1$ to $C_{20}$ alkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyloxy, $C_1$ to $C_{20}$ alkylamino, -OH, -$OR^1$, $-NH_2$, $-NHR^1$, $-NR^1_2$, $C_6$ to $C_{14}$ aryl optionally substituted with one or more $R^2$ group, or $C_2$ to $C_9$ heteroaryl optionally substituted with one or more $R^2$ group; more preferably -OH;
wherein $R^1$ is independently selected from: $C_1$ to $C_{20}$ alkyl, $C_3$ to $C_{20}$ cycloalkyl or $C_2$ to $C_{20}$ alkenyl;
wherein $R^2$ is independently selected from: $C_1$ to $C_{20}$ alkyl, -OH, $-NH_2$, -$OC_1$ to $C_{20}$ alkyl, -$NHC_1$ to $C_{20}$ alkyl or -N($C_1$ to $C_{20}$ alkyl$)_2$, more preferably -OH.

**7.** The compound of any one of Claims 3 to 6, wherein $R^6$ is independently selected from: -H, -OH, $C_1$ to $C_{20}$ alkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyloxy, $C_1$ to $C_{20}$ alkylamino, $C_6$ to $C_{14}$ aryl, and $C_2$ to $C_9$ heteroaryl; wherein $R^6$ is optionally substituted with one or more $R^7$ groups;
wherein $R^7$ is independently selected from: $C_1$ to $C_{20}$ alkyl, $C_1$ to $C_{20}$ haloalkyl, $C_3$ to $C_{20}$ cycloalkyl, $C_3$ to $C_{20}$ heterocycloalkyl, $C_2$ to $C_{20}$ alkenyl, $C_2$ to $C_{20}$ alkynyl, $C_1$ to $C_{20}$ alkyloxy, $C_1$ to $C_{20}$ alkylamino, -OH, -$OR^1$, $-NH_2$, $-NHR^1$, $-NR^1_2$, -C(O)OH, -C(O)$OR^1$, - C(O)$NH_2$, -C(O)$NHR^1$, -C(O)$NR^1_2$, -O(CO)H, -O(CO)$R^1$, -NH(CO)H, -NH(CO)$R^1$, - $NR^1$(CO)H, -$NR^1$(CO)$R^1$, -SH, -$SR^1$, -$SO_2H$, -$SO_2R^1$, -$SO_3R^1$, -$SO_3H$, -$SiR^1_3$, -$NO_2$, -CN, - F, -Cl, -Br, -I, $C_6$ to $C_{14}$ aryl, and $C_2$ to $C_9$ heteroaryl.

**8.** A process for preparing a compound of formula (I) according to Claim 1 or Claim 2, said method comprising:

i) providing a diamine $NH_2$-$L^1$-$NH_2$ wherein $L^1$ is as defined in Claim 1 or Claim 2;
ii) adding at least one furfuraldehyde substituted with n $R^3$ groups, and at least one phenolic compound substituted with x R groups, wherein R, $R^3$, n and x are as defined in Claim 1 or Claim 2 and Claim 6, with the proviso that both positions ortho to the phenolic OH group are unsubstituted on the at least one phenolic compound; and
iii) forming a compound of formula (I).

**9.** A process for preparing a compound of formula (II), (IIIa), or (IIIb) according to any one of Claims 3 to 7, said method comprising:

i) providing: A) a bisphenol compound comprising a linker $L^2$ and substituted with y R groups on each benzene ring of the bisphenol compound; B) a benzene-1,4-diol compound substituted with z R groups; or C) a benzene-1,3-diol optionally substituted with 1 R group at the 5-position; wherein y, z, R and $L^2$ are as defined in any one of Claims 3 to 7;
ii) contacting the compound from step i) with at least one furfuraldehyde substituted with n $R^3$ groups, and at least one primary amine substituted with one $R^6$ group, wherein n, $R^3$ and $R^6$ are as defined in any one of Claims 3 to 7; and
iii) forming a compound of formula (II), (IIIa), or (IIIb).

**10.** The process of Claim 8 or Claim 9, wherein the molar ratio of the diamine, bisphenol compound or benzenediol to the at least one furfuraldehyde is from 1:2 to 1:20, preferably from 1:3 to 1:10, more preferably from 1:4 to 1:8; and/or wherein the molar ratio of the bisphenol compound or benzenediol to the at least one primary amine, or the molar ratio of the diamine to the at least one phenolic compound, is from 1:1 to 1:5, preferably from 1:1.5 to 1:4, more preferably from 1:2 to 1:3.

**11.** The process of any one of Claims 8 to 10, wherein:

I) the diamine or at least one primary amine, is contacted with the at least one furfuraldehyde prior to the addition of either; the at least one phenolic compound, or the bisphenol compound or benzenediol, respectively;
II) the diamine or at least one primary amine, is contacted with a portion of the at least one furfuraldehyde prior to the addition of either; the at least one phenolic compound, or the bisphenol compound or benzenediol, respectively, wherein the remaining furfuraldehyde is added after and/or simultaneously to addition of either; the at least one phenolic compound, or the bisphenol compound or benzenediol, respectively; or
III) the diamine or at least one primary amine, is contacted with either; the at least one phenolic compound, or the bisphenol compound or benzenediol, respectively, and the at least one furfuraldehyde simultaneously;

and preferably wherein, in I), II) or III), two different phenolic compounds or two different primary amines are added separately; or wherein only one phenolic compound or only one primary amine is added.

**12.** A resin composition comprising the compound of any one of Claims 1 to 7.

**13.** Use of a compound according to any one of Claims 1 to 7 or a resin composition according to Claim 12 to reinforce a material, preferably a fibrous material, more preferably glass fibre, carbon fibre or flax; and optionally wherein a catalyst or co-reactant is used to reduce the activation temperature of a polymerisation reaction of the compound of any one of Claims 1 to 7, preferably wherein the catalyst is ferric chloride.

**14.** A method for preparing a reinforced material, said method comprising:

a) contacting a material to be reinforced with a compound as defined in any one of Claim 1 to 7, or with a resin composition as defined in Claim 12; and
b) allowing said compound to self-polymerise to form a reinforced material, preferably wherein the material to be reinforced is a fibrous material, more preferably glass fibre, carbon fibre or flax.

**15.** A reinforced material prepared, or preparable, by the method of Claim 14.

## Patentansprüche

**1.** Eine Verbindung der Formel (I):

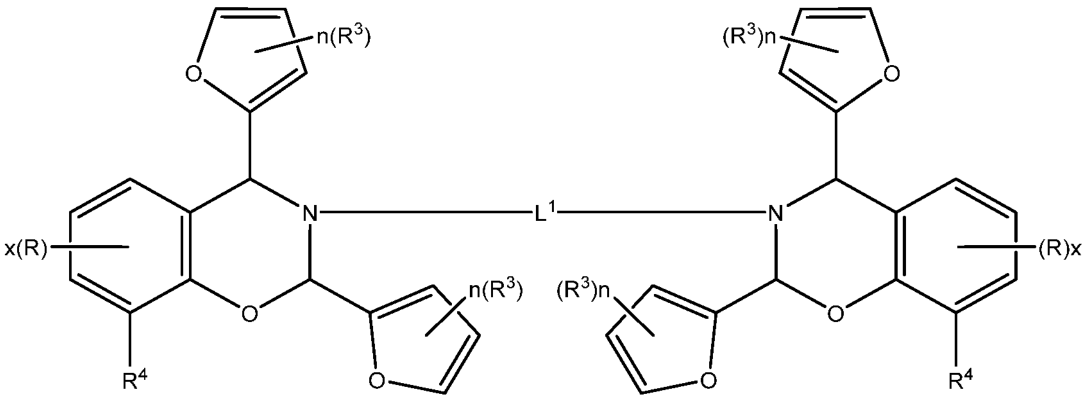

(I)

wobei jede R-Gruppe unabhängig aus den Folgenden ausgewählt ist: $C_1$- bis $C_{20}$-Alkyl, $C_1$- bis $C_{20}$-Halogenalkyl, $C_3$- bis $C_{20}$-Cycloalkyl, $C_3$- bis $C_{20}$-Heterocycloalkyl, $C_2$-bis $C_{20}$-Alkenyl, $C_2$- bis $C_{20}$-Alkinyl, $C_1$- bis $C_{20}$-Alkyl-$C_6$- bis $C_{14}$-Aryl, $C_1$- bis $C_{20}$-Alkyl-$C_2$-bis $C_9$-Heteroaryl, $C_1$- bis $C_{20}$-Alkyl-$C_3$- bis $C_{10}$-Cycloalkyl, $C_1$- bis $C_{20}$-Alkyl-$C_3$- bis $C_{10}$-Heterocycloalkyl, $C_1$- bis $C_{20}$-Alkyloxy, $C_1$- bis $C_{20}$-Alkylamino, -OH, $-OR^1$, $-NH_2$, $-NHR^1$, - $NR^1{}_2$, -C(O)OH, $-C(O)OR^1$, $-C(O)NH_2$, $-C(O)NHR^1$, $-C(O)NR^1{}_2$, -O(CO)H, $-O(CO)R^1$, - NH(CO)H, $-NH(CO)R^1$, $-NR^1(CO)H$, $-NR^1(CO)R^1$, -SH, $-SR^1$, $-SO_2H$, $-SO_2R^1$, $-SO_3R^1$, - $SO_3H$, $-SiR^1{}_3$, $-NO_2$, -CN, -F, -Cl, -Br, -I, $C_6$- bis $C_{14}$-Aryl, optional substituiert mit einer oder mehreren $R^2$-Gruppen, und $C_2$- bis $C_9$-Heteroaryl, optional substituiert mit einer oder mehreren $R^2$-Gruppen;

wobei jedes $R^1$ unabhängig aus den Folgenden ausgewählt ist: $C_1$- bis $C_{20}$-Alkyl, $C_1$- bis $C_{20}$-Halogenalkyl, $C_3$- bis $C_{20}$-Cycloalkyl, $C_3$- bis $C_{20}$-Heterocycloalkyl, $C_2$- bis $C_{20}$-Alkenyl, $C_2$- bis $C_{20}$-Alkinyl, $C_1$- bis $C_{20}$-Alkyl-$C_6$- bis $C_{14}$-Aryl, $C_1$- bis $C_{20}$-Alkyl-$C_2$- bis $C_9$-Heteroaryl, $C_1$- bis $C_{20}$-Alkyl-$C_3$- bis $C_{10}$-Cycloalkyl, $C_1$- bis $C_{20}$-Alkyl-$C_3$- bis $C_{10}$-Heterocycloalkyl, $C_1$- bis $C_{20}$-Alkoxy, $C_1$- bis $C_{20}$-Alkylamino, $C_6$- bis $C_{14}$-Aryl und $C_2$- bis $C_9$-Heteroaryl;

wobei $R^2$ unabhängig aus den Folgenden ausgewählt ist: $C_1$- bis $C_{20}$-Alkyl, $C_1$- bis $C_{20}$-Halogenalkyl, $C_3$- bis $C_{20}$-Cycloalkyl, $C_2$- bis $C_{20}$-Alkenyl, $C_2$- bis $C_{20}$-Alkinyl, -OH, - $NH_2$, $-OC_1$- bis $C_{20}$-Alkyl, $-NHC_1$- bis $C_{20}$-Alkyl, $-N(C_1$- bis $C_{20}$-Alkyl$)_2$, $-NO_2$, -CN, -F, -Cl, - Br und -I;

wobei $R^3$ unabhängig aus den Folgenden ausgewählt ist: $C_1$- bis $C_6$-Alkyl, $C_1$- bis $C_6$-Alkenyl, $C_1$- bis $C_6$-Alkoxy, $C_1$- bis $C_6$-Alkyloxy, -C(O)OH, -C(O)O-$C_1$- bis $C_6$-Alkyl, $C_1$-bis $C_6$-Alkyl-(O)O-H, $C_1$- bis $C_6$-Alkyl-(O)O-$C_1$- bis $C_6$-Alkyl und -OH;

wobei $R^4$ Wasserstoff ist;

wobei $L^1$ ein zweiwertiger Hydrocarbyl-Linker ist, der 1 bis 50 Kohlenstoffatome umfasst;

wobei x unabhängig 0, 1, 2 oder 3 ist; und

wobei n unabhängig 0, 1, 2 oder 3 ist.

**2.** Verbindung nach Anspruch 1, wobei:

a) ein oder beide Benzolringe der Benzoxazine an der 3-Position und/oder der 5-Position, relativ zu dem Sauerstoff des Benzoxazinrings, mit R-Gruppen substituiert sind;

b) beide x-Werte ≤ 2, wobei bevorzugt beide x-Werte ≤ 1, und wobei bevorzugt beide x-Werte gleich sind, und/oder wobei beide Benzolringe der Benzoxazine mit der (den) gleichen R-Gruppe(n) substituiert sind, wobei bevorzugter beide x-Werte = 0;

c) $L^1$ 2 bis 50 Kohlenstoffatome, 3 bis 50 Kohlenstoffatome, 5 bis 50 Kohlenstoffatome, 2 bis 40 Kohlenstoffatome, 3 bis 40 Kohlenstoffatome, 5 bis 40 Kohlenstoffatome, 2 bis 30 Kohlenstoffatome, 3 bis 30 Kohlenstoffatome, 5 bis 30 Kohlenstoffatome, 2 bis 20 Kohlenstoffatome, 3 bis 20 Kohlenstoffatome oder 5 bis 20 Kohlenstoffatome umfasst;

d) $L^1$ aus der Reaktion eines Diamins $NH_2$-$L^1$-$NH_2$ deriviert ist, wenn $NH_2$-$L^1$-$NH_2$ aus den Folgenden ausgewählt ist: Ethylendiamin, Diethylentriamin, Triethylentetramin, Hexamethylendiamin, 2,2,4-Trimethylhexamethylendiamin, 2,4,4-Trimethylhexamethylendiamin, einem Jeffamin, Metaxylylendiamin, 1,3-Bis-(aminomethyl)cyclohexan, Benzol-1,4-diamin, Isophorondiamin, Diaminocyclohexan, 4-Cyclohexen-1,2-diamin, 3-Hexin-1,6-diamin, Bis-(p-aminocyclohexyl)methan oder Methylendianilin; bevorzugt wenn $NH_2$-$L^1$-$NH_2$ aus den Folgenden ausgewählt ist: Ethylendiamin, Hexamethylendiamin, 2,2,4-Trimethylhexamethylendiamin, 2,4,4-Trimethylhexamethylendiamin, einem Jeffamin, Metaxylylendiamin, 1,3-Bis-(aminomethyl)cyclohexan, Benzol-1,4-diamin, Isophorondiamin, Diaminocyclohexan, Bis-(p-aminocyclohexyl)methan und Methylendianilin;

e) die Verbindung die folgende Struktur aufweist:

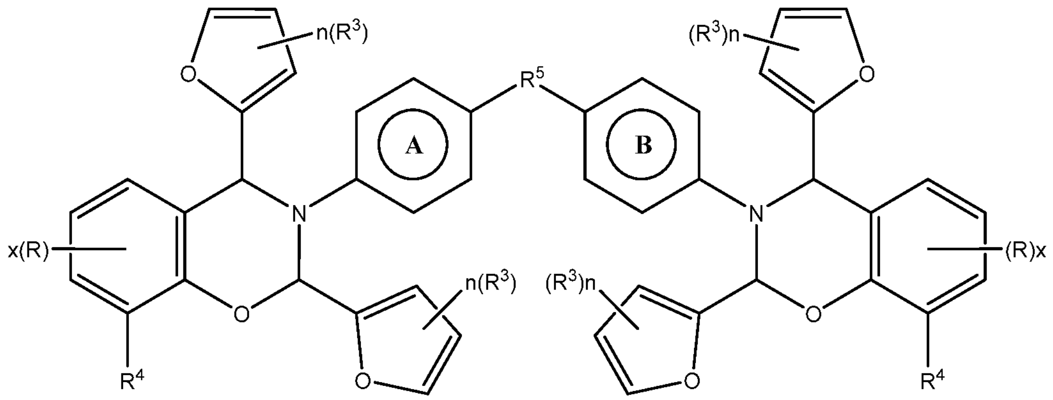

wobei Ring A und Ring B unabhängig entweder aromatische oder gesättigte 6-gliedrige Kohlenstoffringe sind, wobei Ringe A und B bevorzugt beide gesättigte Ringe sind; wobei $R^5$ eine zweiwertige $C_1$ bis $C_{10}$-Alkylgruppe ist, wobei $R^5$ bevorzugt eine Methylengruppe ist; und wobei R, $R^3$, x und n unabhängig wie in Anspruch 1 definiert sind, wobei bevorzugt beide x-Werte = 0 und/oder wobei alle n-Werte = 0;
und/oder
f) $L^1$ mindestens eines der Folgenden umfasst:

i) ein Heteroatom, ausgewählt aus Stickstoff, Sauerstoff, Schwefel und Silicium;
ii) eine Doppel- oder Dreifach-Kohlenstoff-Kohlenstoff-Bindung; oder
iii) einen aromatischen Ring, heteroaromatischen Ring und/oder nichtaromatischen Ring.

**3.** Eine Verbindung der Formel (II):

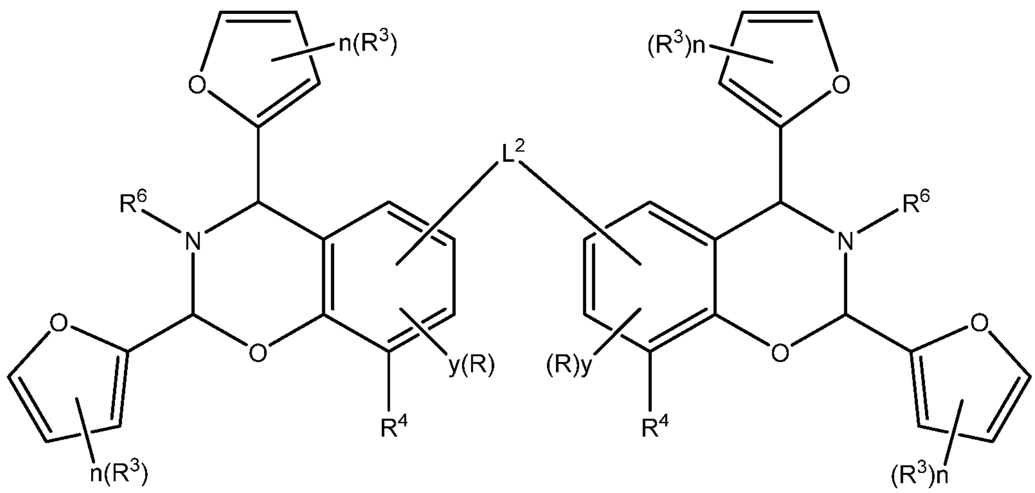

(II)

wobei jede R-Gruppe unabhängig aus den Folgenden ausgewählt ist: $C_1$- bis $C_{20}$-Alkyl, $C_1$- bis $C_{20}$-Halogenalkyl, $C_3$- bis $C_{20}$-Cycloalkyl, $C_3$- bis $C_{20}$-Heterocycloalkyl, $C_2$-bis $C_{20}$-Alkenyl, $C_2$- bis $C_{20}$-Alkinyl, $C_1$- bis $C_{20}$-Alkyl-$C_6$- bis $C_{14}$-Aryl, $C_1$- bis $C_{20}$-Alkyl-$C_2$-bis $C_9$-Heteroaryl, $C_1$- bis $C_{20}$-Alkyl-$C_3$- bis $C_{20}$-Cycloalkyl, $C_1$- bis $C_{20}$-Alkyl-$C_3$- bis $C_{10}$-Heterocycloalkyl, $C_1$- bis $C_{20}$-Alkyloxy, $C_1$- bis $C_{20}$-Alkylamino, -OH, $-OR^1$, $-NH_2$, $-NHR^1$, - $NR^1_2$, -C(O)OH, $-C(O)OR^1$, $-C(O)NH_2$, $-C(O)NHR^1$, $-C(O)NR^1_2$, -O(CO)H, $-O(CO)R^1$, - NH(CO)H, $-NH(CO)R^1$, $-NR^1(CO)H$, $-NR^1(CO)R^1$, -SH, $-SR^1$, $-SO_2H$, $-SO_2R^1$, $-SO_3R^1$, - $SO_3H$, $-SiR^1_3$, $-NO_2$, -CN, -F, -Cl, -Br, -I, $C_6$- bis $C_{14}$-Aryl, optional substituiert mit einer oder mehreren $R^2$-Gruppen, und $C_2$- bis $C_9$-Heteroaryl, optional substituiert mit einer oder mehreren $R^2$-Gruppen;
wobei jedes $R^1$ unabhängig aus den Folgenden ausgewählt ist: $C_1$- bis $C_{20}$-Alkyl, $C_1$- bis $C_{20}$-Halogenalkyl, $C_3$- bis $C_{20}$-Cycloalkyl, $C_3$- bis $C_{20}$-Heterocycloalkyl, $C_2$- bis $C_{20}$-Alkenyl, $C_2$- bis $C_{20}$-Alkinyl, $C_1$- bis $C_{20}$-Alkyl-$C_6$- bis $C_{14}$-Aryl, $C_1$- bis $C_{20}$-Alkyl-$C_2$- bis $C_9$-Heteroaryl, $C_1$- bis $C_{20}$-Alkyl-$C_3$- bis $C_{10}$-Cycloalkyl, $C_1$- bis $C_{20}$-Alkyl-$C_3$-

bis $C_{10}$-Heterocycloalkyl, $C_1$- bis $C_{20}$-Alkoxy, $C_1$- bis $C_{20}$-Alkylamino, $C_6$- bis $C_{14}$-Aryl und $C_2$- bis $C_9$-Heteroaryl;
wobei $R^2$ unabhängig aus den Folgenden ausgewählt ist: $C_1$- bis $C_{20}$-Alkyl, $C_1$- bis $C_{20}$-Halogenalkyl, $C_3$- bis $C_{20}$-Cycloalkyl, $C_2$- bis $C_{20}$-Alkenyl, $C_2$- bis $C_{20}$-Alkinyl, -OH, - $NH_2$, -$OC_1$- bis $C_{20}$-Alkyl, -$NHC_1$- bis $C_{20}$-Alkyl, -N($C_1$- bis $C_{20}$-Alkyl)$_2$, -$NO_2$, -CN, -F, -Cl, - Br und -I;
wobei $R^3$ unabhängig aus den Folgenden ausgewählt ist: $C_1$- bis $C_6$-Alkyl, $C_1$- bis $C_6$-Alkenyl, $C_1$- bis $C_6$-Alkoxy, $C_1$- bis $C_6$-Alkyloxy, -C(O)OH, -C(O)O-$C_1$- bis $C_6$-Alkyl, $C_1$-bis $C_6$-Alkyl-(O)O-H, $C_1$- bis $C_6$-Alkyl-(O)O-$C_1$- bis $C_6$-Alkyl und -OH;
wobei $R^4$ Wasserstoff ist;
wobei $R^6$ unabhängig eine einwertige Gruppe, ausgewählt aus -H, -OH, oder eine Hydrocarbylgruppe, die 1 bis 50 Kohlenstoffatome umfasst, ist;
wobei $L^2$ eine direkte Bindung, eine zweiwertige Gruppe, ausgewählt aus: C(O), O, S, S(O), S(O)$_2$, oder ein Hydrocarbyl-Linker, der 1 bis 50 Kohlenstoffatome umfasst, ist;
wobei y unabhängig 0, 1, oder 2 ist; und
wobei n unabhängig 0, 1, 2 oder 3 ist.

**4.** Verbindung nach Anspruch 3, wobei $L^2$ an der 4-Position gebunden ist, relativ zu dem Benzoxazinsauerstoff; und/oder wobei $L^2$ aus S(O)$_2$, $CH_2$, C($CH_3$)$_2$ ausgewählt oder ein zweiwertiger Benzolring ist; und/oder wobei beide y-Werte ≤ 1, und wobei bevorzugt beide y-Werte gleich sind, und/oder wobei beide Benzolringe der Benzoxazine mit der (den) gleichen R-Gruppe(n) substituiert sind, wobei bevorzugter beide y-Werte = 0.

**5.** Eine Verbindung entweder der Formel (IIIa) oder der Formel (IIIb):

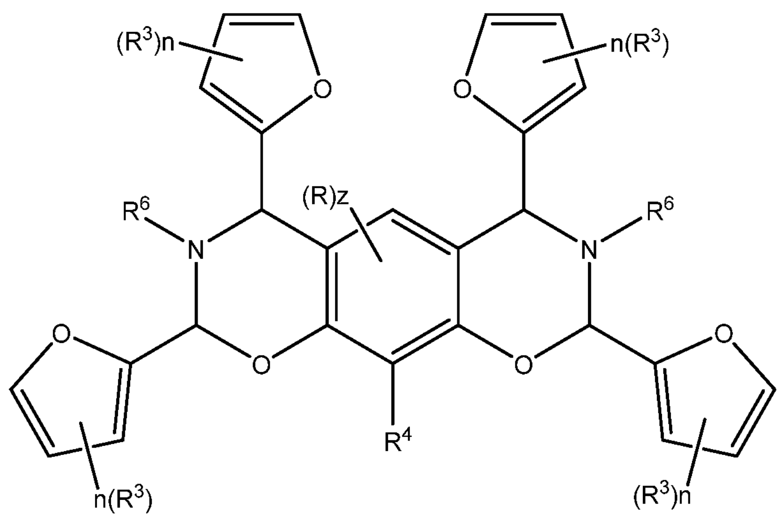

(IIIa)

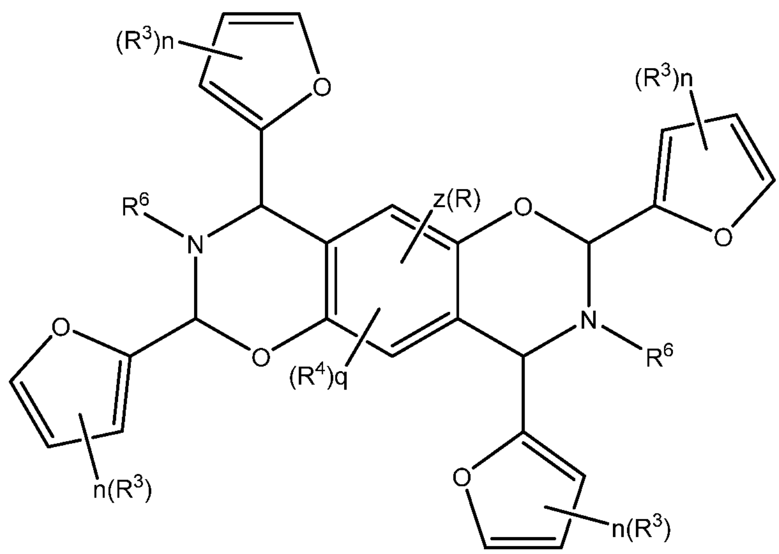

(IIIb)

wobei R aus den Folgenden ausgewählt ist: $C_1$- bis $C_{20}$-Alkyl, $C_1$- bis $C_{20}$-Halogenalkyl, $C_3$- bis $C_{20}$-Cycloalkyl, $C_3$- bis $C_{20}$-Heterocycloalkyl, $C_2$- bis $C_{20}$-Alkenyl, $C_2$-bis $C_{20}$-Alkinyl, $C_1$- bis $C_{20}$-Alkyl-$C_6$- bis $C_{14}$-Aryl, $C_1$- bis $C_{20}$-Alkyl-$C_2$- bis $C_9$-Heteroaryl, $C_1$- bis $C_{20}$-Alkyl-$C_3$- bis $C_{10}$-Cycloalkyl, $C_1$- bis $C_{20}$-Alkyl-$C_3$- bis $C_{10}$-Heterocycloalkyl, $C_1$-bis $C_{20}$-Alkyloxy, $C_1$- bis $C_{20}$-Alkylamino, -OH, $-OR^1$, $-NH_2$, $-NHR^1$, $-NR^1{}_2$, -C(O)OH, - $C(O)OR^1$, $-C(O)NH_2$, $-C(O)NHR^1$, $-C(O)NR^1{}_2$, -O(CO)H, $-O(CO)R^1$, -NH(CO)H, - $NH(CO)R^1$, $-NR^1(CO)H$, $-NR^1(CO)R^1$, -SH, $-SR^1$, $-SO_2H$, $-SO_2R^1$, $-SO_3R^1$, $-SO_3H$, $-SiR^1{}_3$, $-NO_2$, -CN, -F, -Cl, -Br, -I, $C_6$- bis $C_{14}$-Aryl, optional substituiert mit einer oder mehreren $R^2$-Gruppen, und $C_2$- bis $C_9$-Heteroaryl, optional substituiert mit einer oder mehreren $R^2$-Gruppen;
wobei jedes $R^1$ unabhängig aus den Folgenden ausgewählt ist: $C_1$- bis $C_{20}$-Alkyl, $C_1$- bis $C_{20}$-Halogenalkyl, $C_3$- bis $C_{20}$-Cycloalkyl, $C_3$- bis $C_{20}$-Heterocycloalkyl, $C_2$- bis $C_{20}$-Alkenyl, $C_2$- bis $C_{20}$-Alkinyl, $C_1$- bis $C_{20}$-Alkyl-$C_6$- bis $C_{14}$-Aryl, $C_1$- bis $C_{20}$-Alkyl-$C_2$- bis $C_9$-Heteroaryl, $C_1$- bis $C_{20}$-Alkyl-$C_3$- bis $C_{10}$-Cycloalkyl, $C_1$- bis $C_{20}$-Alkyl-$C_3$- bis $C_{10}$-Heterocycloalkyl, $C_1$- bis $C_{20}$-Alkoxy, $C_1$- bis $C_{20}$-Alkylamino, $C_6$- bis $C_{14}$-Aryl und $C_2$- bis $C_9$-Heteroaryl;
wobei $R^2$ unabhängig aus den Folgenden ausgewählt ist: $C_1$- bis $C_{20}$-Alkyl, $C_1$- bis $C_{20}$-Halogenalkyl, $C_3$- bis $C_{20}$-Cycloalkyl, $C_2$- bis $C_{20}$-Alkenyl, $C_2$- bis $C_{20}$-Alkinyl, -OH, - $NH_2$, $-OC_1$- bis $C_{20}$-Alkyl, $-NHC_1$- bis $C_{20}$-Alkyl, $-N(C_1$- bis $C_{20}$-Alkyl$)_2$, $-NO_2$, -CN, -F, -Cl, - Br und -I;
wobei $R^3$ unabhängig aus den Folgenden ausgewählt ist: $C_1$- bis $C_6$-Alkyl, $C_1$- bis $C_6$-Alkenyl, $C_1$- bis $C_6$-Alkoxy, $C_1$- bis $C_6$-Alkyloxy, -C(O)OH, -C(O)O-$C_1$- bis $C_6$-Alkyl, $C_1$-bis $C_6$-Alkyl-(O)O-H, $C_1$- bis $C_6$-Alkyl-(O)-O-$C_1$- bis $C_6$-Alkyl und -OH;
wobei $R^4$ Wasserstoff ist;
wobei $R^6$ unabhängig eine einwertige Gruppe, ausgewählt aus -H, -OH, oder eine Hydrocarbylgruppe, die 1 bis 50 Kohlenstoffatome umfasst, ist;
wobei z 0 oder 1 ist;
wobei q = 2 - z; und
wobei n unabhängig 0, 1, 2 oder 3 ist.

**6.** Verbindung nach einem der vorherigen Ansprüche, wobei:

1) jedes $R^3$ unabhängig aus den Folgenden ausgewählt ist: $C_1$- bis $C_6$-Alkyl, $C_1$- bis $C_6$-Alkoxy, $C_1$- bis $C_6$-Alkyloxy und -OH, bevorzugt-$CH_3$, $-CH_2OH$, oder $-CH_2OCH_3$;
2) alle n-Werte $\leq 1$, und wobei bevorzugt alle n-Werte gleich sind, und/oder wobei alle $R^3$-Gruppen gleich sind, und/oder wobei alle $R^3$-Gruppen an der Position in ortho-Stellung zu dem Furansauerstoff gebunden sind; wobei bevorzugter alle n-Werte = 0; und/oder
3) jede R-Gruppe unabhängig aus den Folgenden ausgewählt ist: $C_1$- bis $C_{20}$-Alkyl, $C_3$- bis $C_{20}$-Cycloalkyl, $C_3$- bis $C_{20}$-Heterocycloalkyl, $C_2$- bis $C_{20}$-Alkenyl, $C_2$- bis $C_{20}$-Alkinyl, $C_1$- bis $C_{20}$-Alkyl-$C_6$- bis $C_{14}$-Aryl, $C_1$- bis $C_{20}$-Alkyl-$C_2$- bis $C_9$-Heteroaryl, $C_1$- bis $C_{20}$-Alkyl-$C_3$- bis $C_{10}$-Cycloalkyl, $C_1$- bis $C_{20}$-Alkyl-$C_3$- bis $C_{10}$-Heterocycloalkyl, $C_1$- bis $C_{20}$-Alkyloxy, $C_1$- bis $C_{20}$-Alkylamino, -OH, $-OR^1$, $-NH_2$, $-NHR^1$, $-NR^1{}_2$, -O(CO)H, $-O(CO)R^1$, -NH(CO)H, $-NH(CO)R^1$, $-NR^1(CO)H$, $-NR^1(CO)R^1$, -SH, $-SR^1$, $-SiR^1{}_3$, -F, -Cl, -Br, -I, $C_6$- bis $C_{14}$-Aryl, optional

substituiert mit einer oder mehreren $R^2$-Gruppen, und $C_2$- bis $C_9$-Heteroaryl, optional substituiert mit einer oder mehreren $R^2$-Gruppen;
wobei $R^1$ unabhängig aus den Folgenden ausgewählt ist: $C_1$- bis $C_{20}$-Alkyl, $C_3$- bis $C_{20}$-Cycloalkyl, $C_3$- bis $C_{20}$-Heterocycloalkyl, $C_2$- bis $C_{20}$-Alkenyl, $C_2$- bis $C_{20}$-Alkinyl, $C_1$-bis $C_{20}$-Alkyl-$C_6$- bis $C_{14}$-Aryl, $C_1$- bis $C_{20}$-Alkyl-$C_2$- bis $C_9$-Heteroaryl, $C_1$- bis $C_{20}$-Alkyl-$C_3$-bis $C_{20}$-Cycloalkyl, $C_1$- bis $C_{20}$-Alkyl-$C_3$ bis $C_{10}$-Heterocycloalkyl, $C_1$- bis $C_{20}$-Alkoxy, $C_1$-bis $C_{20}$-Alkylamino, $C_6$- bis $C_{14}$-Aryl und $C_2$- bis $C_9$-Heteroaryl; und
wobei $R^2$ unabhängig aus den Folgenden ausgewählt ist: $C_1$- bis $C_{20}$-Alkyl, $C_3$- bis $C_{20}$-Cycloalkyl, $C_2$- bis $C_{20}$-Alkenyl, $C_2$- bis $C_{20}$-Alkinyl, -OH, $-NH_2$, $-OC_1$- bis $C_{20}$-Alkyl, - $NHC_1$- bis $C_{20}$-Alkyl, $-N(C_1$- bis $C_{20}$-Alkyl$)_2$, -F, -Cl, -Br oder -I:

wobei bevorzugt jede R-Gruppe unabhängig aus den Folgenden ausgewählt ist: $C_1$- bis $C_{20}$-Alkyl, $C_3$- bis $C_{20}$-Cycloalkyl, $C_3$- bis $C_{20}$-Heterocycloalkyl, $C_2$- bis $C_{20}$-Alkenyl, $C_2$- bis $C_{20}$-Alkinyl, $C_1$- bis $C_{20}$-Alkyloxy, $C_1$- bis $C_{20}$-Alkylamino, -OH, $-OR^1$, $-NH_2$, $-NHR^1$, $-NR^1{}_2$, $C_6$- bis $C_{14}$-Aryl, optional substituiert mit einer oder mehreren $R^2$-Gruppen, oder $C_2$-bis $C_9$-Heteroaryl, optional substituiert mit einer oder mehreren $R^2$-Gruppen; bevorzugter -OH;
wobei $R^1$ unabhängig aus den Folgenden ausgewählt ist: $C_1$- bis $C_{20}$-Alkyl, $C_3$- bis $C_{20}$-Cycloalkyl oder $C_2$- bis $C_{20}$-Alkenyl;
wobei $R^2$ unabhängig aus den Folgenden ausgewählt ist: $C_1$- bis $C_{20}$-Alkyl, -OH, - $NH_2$, $-OC_1$- bis $C_{20}$-Alkyl, $-NHC_1$- bis $C_{20}$-Alkyl oder $-N(C_1$- bis $C_{20}$-Alkyl$)_2$, bevorzugter - OH.

**7.** Verbindung nach einem der Ansprüche 3 bis 6, wobei $R^6$ unabhängig aus den Folgenden ausgewählt ist: -H, -OH, $C_1$- bis $C_{20}$-Alkyl, $C_3$- bis $C_{20}$-Cycloalkyl, $C_3$- bis $C_{20}$-Heterocycloalkyl, $C_2$- bis $C_{20}$-Alkenyl, $C_2$- bis $C_{20}$-Alkinyl, $C_1$- bis $C_{20}$-Alkyloxy, $C_1$- bis $C_{20}$-Alkylamino, $C_6$- bis $C_{14}$-Aryl, und $C_2$- bis $C_9$-Heteroaryl; wobei $R^6$ optional mit einer oder mehreren $R^7$-Gruppen substituiert ist;
wobei $R^7$ unabhängig aus den Folgenden ausgewählt ist: $C_1$- bis $C_{20}$-Alkyl, $C_1$- bis $C_{20}$-Halogenalkyl, $C_3$- bis $C_{20}$-Cycloalkyl, $C_3$- bis $C_{20}$-Heterocycloalkyl, $C_2$- bis $C_{20}$-Alkenyl, $C_2$- bis $C_{20}$-Alkinyl, $C_1$- bis $C_{20}$-Alkyloxy, $C_1$- bis $C_{20}$-Alkylamino, -OH, $-OR^1$, $-NH_2$, $-NHR^1$, $-NR^1{}_2$, -C(O)OH, $-C(O)OR^1$, $-C(O)NH_2$, $-C(O)NHR^1$, $-C(O)NR^1{}_2$, -O(CO) H, $-O(CO)R^1$, - NH(CO)H, $-NH(CO)R^1$, $-NR^1(CO)H$, $-NR^1(CO)R^1$, -SH, $-SR^1$, $-SO_2H$, $-SO_2R^1$, $-SO_3R^1$, - $SO_3H$, $-SiR^1{}_3$, $-NO_2$, -CN, -F, -Cl, -Br, -I, $C_6$- bis $C_{14}$-Aryl und $C_2$- bis $C_9$-Heteroaryl.

**8.** Ein Prozess zur Herstellung einer Verbindung der Formel (I) nach Anspruch 1 oder Anspruch 2, wobei das genannte Verfahren Folgendes umfasst:

i) Bereitstellen eines Diamins $NH_2$-$L^1$-$NH_2$, wobei $L^1$ wie in Anspruch 1 oder Anspruch 2 definiert ist;
ii) Hinzufügen mindestens eines Furfuraldehyds, substituiert mit n $R^3$-Gruppen, und mindestens einer phenolischen Verbindung, substituiert mit x R-Gruppen, wobei R, $R^3$, n und x wie in Anspruch 1 oder Anspruch 2 und Anspruch 6 definiert sind, mit der Maßgabe, dass beide Positionen in ortho-Stellung zu der phenolischen OH-Gruppe an der mindestens einen phenolischen Verbindung nicht substituiert sind; und
iii) Bilden einer Verbindung der Formel (I).

**9.** Ein Prozess zur Herstellung einer Verbindung der Formel (II), (IIIa) oder (IIIb) nach einem der Ansprüche 3 bis 7, wobei das genannte Verfahren Folgendes umfasst:

i) Bereitstellen: A) einer Bisphenolverbindung, die einen Linker $L^2$ umfasst und an jedem Benzolring der Bisphenolverbindung mit y R-Gruppen substituiert ist; B) einer Benzol-1,4-diolverbindung, die mit z R-Gruppen substituiert ist; oder C) eines Benzol-1,3-diols, das optional an der 5-Position mit 1 R-Gruppe substituiert ist; wobei y, z, R und $L^2$ wie in einem der Ansprüche 3 bis 7 definiert sind;
ii) In-Kontakt-Bringen der Verbindung aus Schritt i) mit mindestens einem Furfuraldehyd, das mit n $R^3$-Gruppen substituiert ist, und mindestens einem primären Amin, das mit einer $R^6$-Gruppe substituiert ist, wobei n, $R^3$ und $R^6$ wie in einem der Ansprüche 3 bis 7 definiert sind; und
iii) Bilden einer Verbindung der Formel (II), (IIIa) oder (IIIb).

**10.** Prozess nach Anspruch 8 oder Anspruch 9, wobei das Molverhältnis des Diamins, der Bisphenolverbindung oder des Benzoldiols zu dem mindestens einen Furfuraldehyd von 1:2 bis 1:20, bevorzugt von 1:3 bis 1:10, bevorzugter von 1:4 bis 1:8 beträgt; und/oder wobei das Molverhältnis der Bisphenolverbindung oder des Benzoldiols zu dem mindestens einen primären Amin oder das Molverhältnis des Diamins zu der mindestens einen phenolischen Verbindung von 1:1 bis 1:5, bevorzugt von 1:1,5 bis 1:4, bevorzugter von 1:2 bis 1:3 beträgt.

**11.** Prozess nach einem der Ansprüche 8 bis 10, wobei:

I) das Diamin oder mindestens eine primäre Amin mit dem mindestens einen Furfuraldehyd vor dem Hinzufügen entweder der mindestens einen phenolischen Verbindung oder der Bisphenolverbindung bzw. des Benzoldiols in Kontakt gebracht wird;
II) das Diamin oder mindestens eine primäre Amin mit einem Anteil des mindestens einen Furfuraldehyds vor dem Hinzufügen entweder der mindestens einen phenolischen Verbindung oder der Bisphenolverbindung bzw. des Benzoldiols in Kontakt gebracht wird, wobei das verbleibende Furfuraldehyd nach dem und/oder gleichzeitig mit dem Hinzufügen der mindestens einen phenolischen Verbindung oder der Bisphenolverbindung bzw. des Benzoldiols hinzugefügt wird; oder
III) das Diamin oder mindestens eine primäre Amin gleichzeitig entweder mit der mindestens einen phenolischen Verbindung oder der Bisphenolverbindung bzw. dem Benzoldiol und dem mindestens einen Furfuraldehyd in Kontakt gebracht wird;

und bevorzugt wobei, in I), II) oder III), zwei verschiedene phenolische Verbindungen oder zwei verschiedene primäre Amine separat hinzugefügt werden; oder wobei nur eine phenolische Verbindung oder nur ein primäres Amin hinzugefügt wird.

**12.** Eine Harzzusammensetzung, die die Verbindung nach einem der Ansprüche 1 bis 7 umfasst.

**13.** Verwendung einer Verbindung nach einem der Ansprüche 1 bis 7 oder einer Harzzusammensetzung nach Anspruch 12 zum Verstärken eines Materials, bevorzugt eines faserigen Materials, bevorzugter Glasfaser, Kohlenstofffaser oder Flachs; und wobei optional ein Katalysator oder Koreaktant verwendet wird, um die Aktivierungstemperatur einer Polymerisationsreaktion der Verbindung nach einem der Ansprüche 1 bis 7 zu reduzieren, wobei der Katalysator bevorzugt Eisen(III)-chlorid ist.

**14.** Ein Verfahren zur Herstellung eines verstärkten Materials, wobei das genannte Verfahren Folgendes umfasst:

a) In-Kontakt-Bringen eines zu verstärkenden Materials mit einer Verbindung wie in einem der Ansprüche 1 bis 7 definiert oder mit einer Harzzusammensetzung wie in Anspruch 12 definiert; und
b) Selbstpolymerisierenlassen der genannten Verbindung, um ein verstärktes Material zu bilden, wobei das zu verstärkende Material bevorzugt ein faseriges Material, bevorzugter Glasfaser, Kohlenstofffaser oder Flachs, ist.

**15.** Ein verstärktes Material, das durch das Verfahren nach Anspruch 14 hergestellt wird oder hergestellt werden kann.

## Revendications

**1.** Composé ayant la formule (I) :

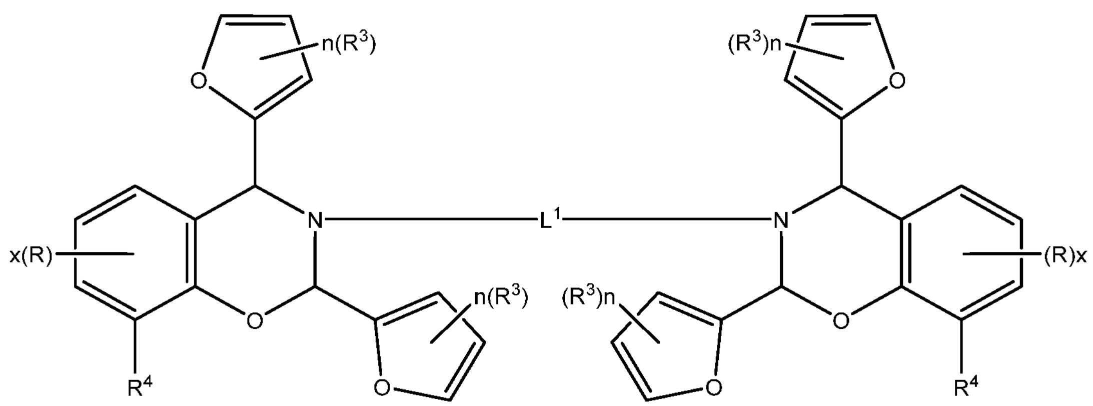

(I)

dans lequel chaque groupe R est sélectionné indépendamment parmi : des groupes alkyle $C_1$ à $C_{20}$, haloalkyle $C_1$ à $C_{20}$, cycloalkyle $C_3$ à $C_{20}$, hétérocycloalkyle $C_3$ à $C_{20}$, alcényle $C_2$ à $C_{20}$, alcynyle $C_2$ à $C_{20}$, alkyle $C_1$ à $C_{20}$-

aryle $C_6$ à $C_{14}$, alkyle $C_1$ à $C_{20}$-hétéroaryle $C_2$ à $C_9$, alkyle $C_1$ à $C_{20}$-cycloalkyle $C_3$ à $C_{10}$, alkyle $C_1$ à $C_{20}$ -hétérocycloalkyle $C_3$ à $C_{10}$, alkyloxy $C_1$ à $C_{20}$, alkylamino $C_4$ à $C_{20}$, -OH, -$OR^1$, -$NH_2$, -$NHR^1$, -$NR^1_2$, -C(O)OH, -C(O)$OR^1$, -C(O)$NH_2$, -C(O)NHR', -C(O)$NR^1_2$, -O(CO)H, -O(CO)$R^1$, -NH(CO)H, - NH(CO)$R^1$, -$NR^1$(CO)H, -$NR^1$(CO)$R^1$, -SH, -$SR^1$, -$SO_2H$, -$SO_2R^1$, -$SO_3R^1$, -$SO_3H$, -$SiR^1_3$, -$NO_2$, -CN, -F, -Cl, -Br, -I, aryle $C_6$ à $C_{14}$ optionnellement substitué par un ou plusieurs groupes $R^2$, et hétéroaryle $C_2$ à $C_9$ optionnellement substitué par un ou plusieurs groupes $R^2$ ;

dans lequel chaque groupe $R^1$ est sélectionné indépendamment parmi : des groupes alkyle $C_1$ à $C_{20}$, haloalkyle $C_1$ à $C_{20}$, cycloalkyle $C_3$ à $C_{20}$, hétérocycloalkyle $C_3$ à $C_{20}$, alcényle $C_2$ à $C_{20}$, alcynyle $C_2$ à $C_{20}$, alkyle $C_1$ à $C_{20}$-aryle $C_6$ à $C_{14}$, alkyle $C_1$ à $C_{20}$-hétéroaryle $C_2$ à $C_9$, alkyle $C_1$ à $C_{20}$-cycloalkyle $C_3$ à $C_{10}$, alkyle $C_1$ à $C_{20}$-hétérocycloalkyle $C_3$ à $C_{10}$, alcoxy $C_1$ à $C_{20}$, alkylamino $C_4$ à $C_{20}$, aryle $C_6$ à $C_{14}$, et hétéroaryle $C_2$ à $C_9$ ;

dans lequel le groupe $R^2$ est sélectionné indépendamment parmi : des groupes alkyle $C_1$ à $C_{20}$, haloalkyle $C_1$ à $C_{20}$, cycloalkyle $C_3$ à $C_{20}$, alcényle $C_2$ à $C_{20}$, alcynyle $C_2$ à $C_{20}$, -OH, -$NH_2$, -O-alkyle $C_1$ à $C_{20}$, -NH-alkyle $C_1$ à $C_{20}$, -N(alkyle $C_1$ à $C_{20})_2$, -$NO_2$, -CN, - F, -Cl, -Br, et -I ;

dans lequel le groupe $R^3$ est sélectionné indépendamment parmi : des groupes alkyle $C_1$ à $C_6$, alcényle $C_1$ à $C_6$, alcoxy $C_1$ à $C_6$, alkyloxy $C_1$ à $C_6$, -C(O)OH, -C(O)O-alkyle $C_1$ à $C_6$, alkyle $C_1$ à $C_6$-(O)O-H, alkyle $C_1$ à $C_6$-(O)O-alkyle $C_1$ à $C_6$, et -OH ;

dans lequel le groupe $R^4$ est un atome d'hydrogène ;

dans lequel le groupe $L^1$ est un lieur hydrocarbyle divalent comprenant 1 à 50 atomes de carbone ;

dans lequel x vaut indépendamment 0, 1, 2 ou 3 ; et

dans lequel n vaut indépendamment 0, 1, 2 ou 3.

**2.** Composé selon la revendication 1, dans lequel :

a) un cycle benzénique ou les deux cycles benzéniques des benzoxazines sont substitués par des groupes R en position 3 et/ou en position 5, par rapport à l'atome d'oxygène du cycle benzoxazine ;

b) les deux valeurs x sont ≤ 2, préférablement dans lequel les deux valeurs x sont ≤ 1, et préférablement dans lequel les deux valeurs x sont identiques, et/ou dans lequel les deux cycles benzéniques des benzoxazines sont substitués par le(s) même(s) groupe(s) R), plus préférablement dans lequel les deux valeurs x = 0 ;

c) le groupe $L^1$ comprend 2 à 50 atomes de carbone, 3 à 50 atomes de carbone, 5 à 50 atomes de carbone, 2 à 40 atomes de carbone, 3 à 40 atomes de carbone, 5 à 40 atomes de carbone, 2 à 30 atomes de carbone, 3 à 30 atomes de carbone, 5 à 30 atomes de carbone, 2 à 20 atomes de carbone, 3 à 20 atomes de carbone, ou 5 à 20 atomes de carbone ;

d) le groupe $L^1$ est dérivé de la réaction d'une diamine $NH_2$-$L^1$-$NH_2$, lorsque $NH_2$-$L^1$-$NH_2$ est sélectionnée parmi : l'éthylènediamine, la diéthylènetriamine, la triéthylènetétramine, l'hexaméthylènediamine, la 2,2,4-triméthylhexaméthylènediamine, la 2,4,4-triméthylhexaméthylènediamine, une Jeffamine, la méthaxylylènediamine, le 1,3-bis(aminométhyl)cyclohexane, la benzène-1,4-diamine, l'isophoronediamine, le diaminocyclohexane, la 4-cyclohexène-1,2-diamine, la 3-hexyne-1,6-diamine, le bis-(p-aminocyclohexyl)méthane ou la méthylènedianiline ; préférablement lorsque $NH_2$-$L^1$-$NH_2$ est sélectionnée parmi l'éthylènediamine, l'hexaméthylènediamine, la 2,2,4-triméthylhexaméthylènediamine, la 2,4,4-triméthylhexaméthylènediamine, une Jeffamine, la méthanylylènediamine, le 1,3-bis(aminométhyl)cyclohexane, la benzène-1,4-diamine, l'isophoronediamine, le diaminocyclohexane, le bis-(p-aminocyclohexyl)méthane et la méthylènedianiline ;

e) le composé a la structure :

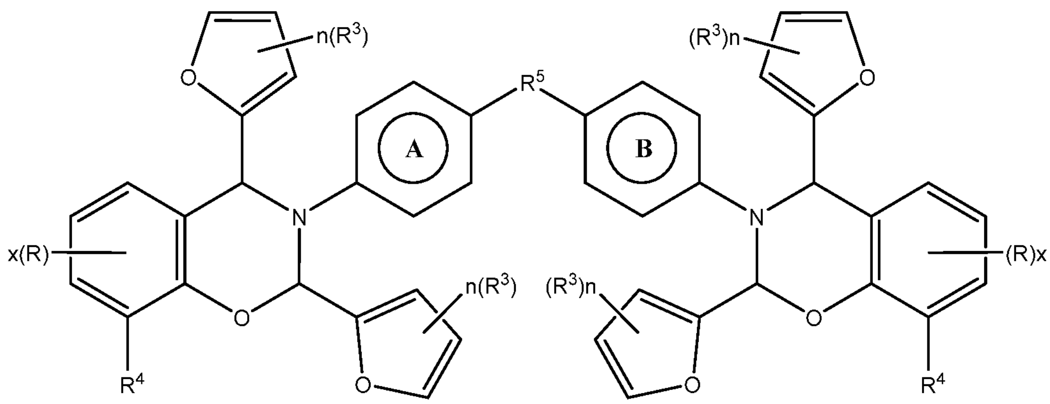

dans laquelle le cycle A et le cycle B sont indépendamment des cycles carbonés à 6 chaînons soit aromatiques,

soit saturés, préférablement dans laquelle les cycles A et B sont tous deux des cycles saturés ; dans laquelle le groupe $R^5$ est un groupe alkyle $C_1$ à $C_{10}$ divalent, préférablement dans laquelle le groupe $R^5$ est un groupe méthylène ; et dans laquelle le groupe R, le groupe $R^3$, x et n sont indépendamment tels que définis dans la revendication 1, préférablement dans laquelle les deux valeurs x = 0 et/ou dans laquelle toutes les valeurs n = 0 ; et/ou

f) le groupe $L^1$ comprend au moins l'un de :

i) un hétéroatome sélectionné parmi un hétéroatome d'azote, d'oxygène, de soufre et de silicium ;
ii) une double ou triple liaison carbone-carbone ; ou
iii) un cycle aromatique, un cycle hétéroaromatique et/ou un cycle non aromatique.

**3.** Composé ayant la formule (II) :

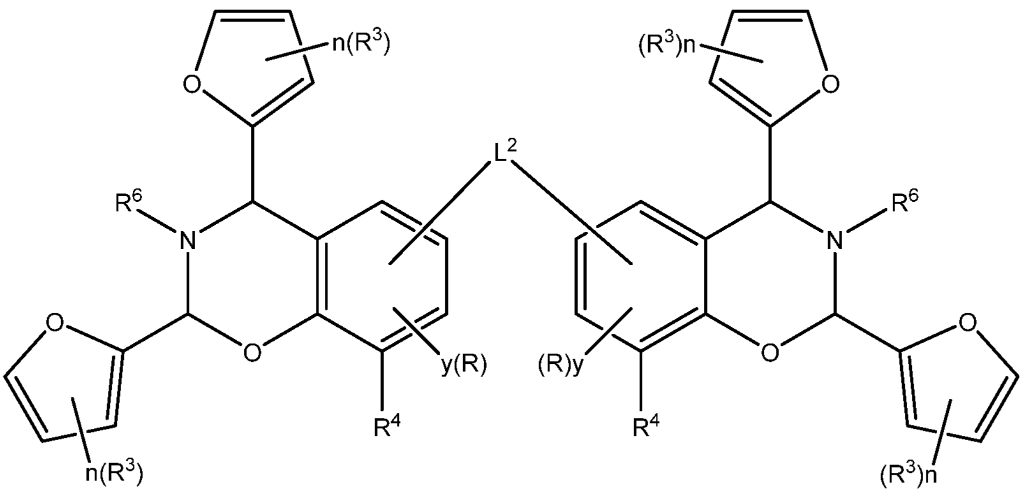

(II)

dans lequel chaque groupe R est sélectionné indépendamment parmi : des groupes alkyle $C_1$ à $C_{20}$, haloalkyle $C_1$ à $C_{20}$, cycloalkyle $C_3$ à $C_{20}$, hétérocycloalkyle $C_3$ à $C_{20}$, alcényle $C_2$ à $C_{20}$, alcynyle $C_2$ à $C_{20}$, alkyle $C_1$ à $C_{20}$-aryle $C_6$ à $C_{14}$, alkyle $C_1$ à $C_{20}$-hétéroaryle $C_2$ à $C_9$, alkyle $C_1$ à $C_{20}$-cycloalkyle $C_3$ à $C_{20}$, alkyle $C_1$ à $C_{20}$-hétérocycloalkyle $C_3$ à $C_{10}$, alkyloxy $C_1$ à $C_{20}$, alkylamino $C_4$ à $C_{20}$, -OH, $-OR^1$, $-NH_2$, $-NHR^1$, $-NR^1_2$, -C(O)OH, $-C(O)OR^1$, $-C(O)NH_2$, -C(O)NHR', $-C(O)NR^1_2$, -O(CO)H, $-O(CO)R^1$, -NH(CO)H, - $NH(CO)R^1$, $-NR^1(CO)H$, $-NR^1(CO)R^1$, -SH, $-SR^1$, $-SO_2H$, $-SO_2R^1$, $-SO_3R^1$, $-SO_3H$, $-SiR^1_3$, $-NO_2$, -CN, -F, -Cl, -Br, -I, aryle $C_6$ à $C_{14}$ optionnellement substitué par un ou plusieurs groupes $R^2$, et hétéroaryle $C_2$ à $C_9$ optionnellement substitué par un ou plusieurs groupes $R^2$ ;
dans lequel chaque groupe $R^1$ est sélectionné indépendamment parmi : des groupes alkyle $C_1$ à $C_{20}$, haloalkyle $C_1$ à $C_{20}$, cycloalkyle $C_3$ à $C_{20}$, hétérocycloalkyle $C_3$ à $C_{20}$, alcényle $C_2$ à $C_{20}$, alcynyle $C_2$ à $C_{20}$, alkyle $C_1$ à $C_{20}$-aryle $C_6$ à $C_{14}$, alkyle $C_1$ à $C_{20}$-hétéroaryle $C_2$ à $C_9$, alkyle $C_1$ à $C_{20}$-cycloalkyle $C_3$ à $C_{10}$, alkyle $C_1$ à $C_{20}$-hétérocycloalkyle $C_3$ à $C_{10}$, alcoxy $C_1$ à $C_{20}$, alkylamino $C_4$ à $C_{20}$, aryle $C_6$ à $C_{14}$, et hétéroaryle $C_2$ à $C_9$ ;
dans lequel le groupe $R^2$ est sélectionné indépendamment parmi : des groupes alkyle $C_1$ à $C_{20}$, haloalkyle $C_1$ à $C_{20}$, cycloalkyle $C_3$ à $C_{20}$, alcényle $C_2$ à $C_{20}$, alcynyle $C_2$ à $C_{20}$, -OH, $-NH_2$, -O-alkyle $C_1$ à $C_{20}$, -NH-alkyle $C_1$ à $C_{20}$, -N(alkyle $C_1$ à $C_{20})_2$, $-NO_2$, -CN, - F, -Cl, -Br, et -I ;
dans lequel le groupe $R^3$ est sélectionné indépendamment parmi : des groupes alkyle $C_1$ à $C_6$, alcényle $C_1$ à $C_6$, alcoxy $C_1$ à $C_6$, alkyloxy $C_1$ à $C_6$, -C(O)OH, -C(O)O-alkyle $C_1$ à $C_6$, alkyle $C_1$ à $C_6$-(O)O-H, alkyle $C_1$ à $C_6$-(O)O-alkyle $C_1$ à $C_6$, et -OH ;
dans lequel le groupe $R^4$ est un atome d'hydrogène ;
dans lequel le groupe $R^6$ est indépendamment un groupe monovalent sélectionné parmi -H, -OH, ou un groupe hydrocarbyle comprenant 1 à 50 atomes de carbone ;
dans lequel le groupe $L^2$ est une liaison directe, un groupe divalent sélectionné parmi : C(O), O, S, S(O), $S(O)_2$, ou un lieur hydrocarbyle comprenant 1 à 50 atomes de carbone ;
dans lequel y vaut indépendamment 0, 1 ou 2 ; et
dans lequel n vaut indépendamment 0, 1, 2 ou 3.

**4.** Composé selon la revendication 3, dans lequel le groupe $L^2$ est fixé en position 4, par rapport à l'atome d'oxygène de la benzoxazine ; et/ou dans lequel le groupe $L^2$ est sélectionné parmi $S(O)_2$, $CH_2$, $C(CH_3)_2$ ou un cycle benzénique divalent ; et/ou
dans lequel les deux valeurs y sont ≤ 1, et préférablement dans lequel les deux valeurs y sont identiques, et/ou dans lequel les deux cycles benzéniques des benzoxazines sont substitués par le(s) même(s) groupe(s) R, plus préférablement dans lequel les deux valeurs y = 0.

**5.** Composé ayant soit la formule (IIIa), soit la formule (IIIb) :

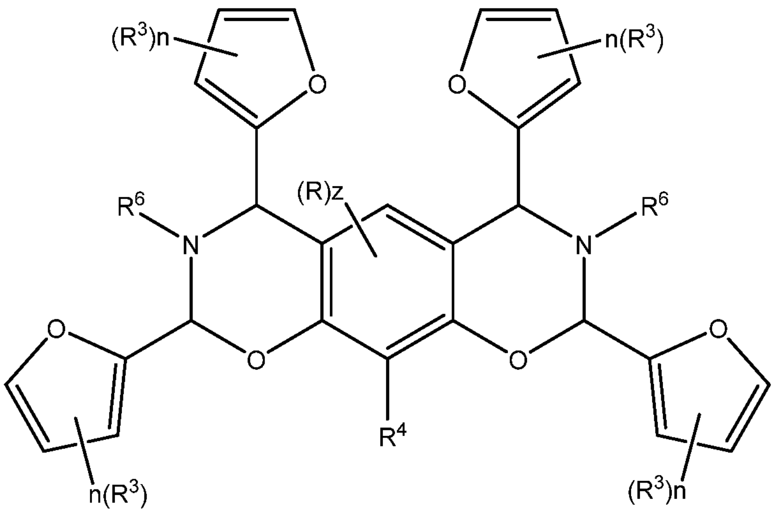

(IIIa)

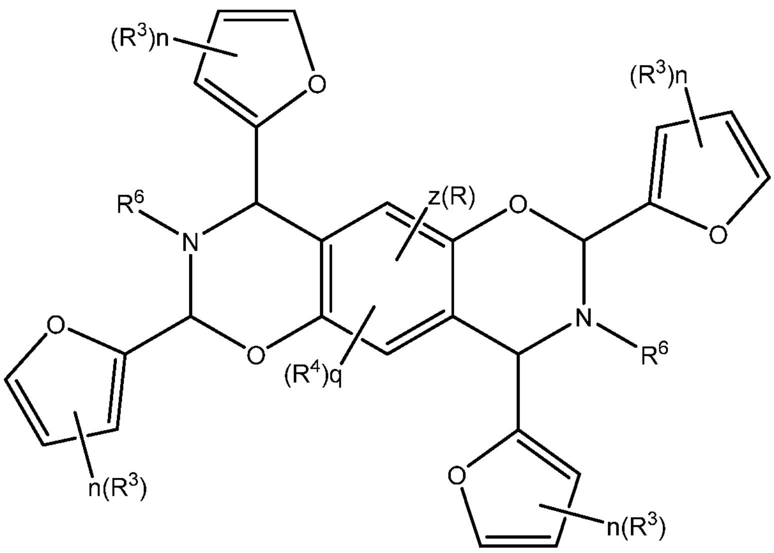

(IIIb)

dans lequel R est sélectionné parmi : des groupes alkyle $C_1$ à $C_{20}$, haloalkyle $C_1$ à $C_{20}$, cycloalkyle $C_3$ à $C_{20}$, hétérocycloalkyle $C_3$ à $C_{20}$, alcényle $C_2$ à $C_{20}$, alcynyle $C_2$ à $C_{20}$, alkyle $C_1$ à $C_{20}$-aryle $C_6$ à $C_{14}$, alkyle $C_1$ à $C_{20}$-hétéroaryle $C_2$ à $C_9$, alkyle $C_1$ à $C_{20}$-cycloalkyle $C_3$ à $C_{10}$, alkyle $C_1$ à $C_{20}$-hétérocycloalkyle $C_3$ à $C_{10}$, alkyloxy $C_1$ à $C_{20}$, alkylamino $C_4$ à $C_{20}$, -OH, $-OR^1$, $-NH_2$, $-NHR^1$, $-NR^1_2$, -C(O)OH, $-C(O)OR^1$, $-C(O)NH_2$, - C(O)NHR', $-C(O)NR^1_2$, -O(CO)H, $-O(CO)R^1$, -NH(CO)H, $-NH(CO)R^1$, $-NR^1(CO)H$, - $NR^1(CO)R^1$, -SH, $-SR^1$, $-SO_2H$, $-SO_2R^1$, $-SO_3R^1$, $-SO_3H$, $-SiR^1_3$, $-NO_2$, -CN, -F, -Cl, -Br, -I, aryle $C_6$ à $C_{14}$ optionnellement substitué par un ou plusieurs groupes $R^2$, et hétéroaryle $C_2$ à $C_9$ optionnellement substitué par un ou plusieurs groupes $R^2$ ;
dans lequel chaque groupe $R^1$ est sélectionné indépendamment parmi : des groupes alkyle $C_1$ à $C_{20}$, haloalkyle $C_1$ à $C_{20}$, cycloalkyle $C_3$ à $C_{20}$, hétérocycloalkyle $C_3$ à $C_{20}$, alcényle $C_2$ à $C_{20}$, alcynyle $C_2$ à $C_{20}$, alkyle $C_1$ à $C_{20}$-

aryle $C_6$ à $C_{14}$, alkyle $C_1$ à $C_{20}$-hétéroaryle $C_2$ à $C_9$, alkyle $C_1$ à $C_{20}$-cycloalkyle $C_3$ à $C_{10}$, alkyle $C_1$ à $C_{20}$-hétérocycloalkyle $C_3$ à $C_{10}$, alcoxy $C_1$ à $C_{20}$, alkylamino $C_4$ à $C_{20}$, aryle $C_6$ à $C_{14}$, et hétéroaryle $C_2$ à $C_9$ ;
dans lequel le groupe $R^2$ est sélectionné indépendamment parmi : des groupes alkyle $C_1$ à $C_{20}$, haloalkyle $C_1$ à $C_{20}$, cycloalkyle $C_3$ à $C_{20}$, alcényle $C_2$ à $C_{20}$, alcynyle $C_2$ à $C_{20}$, -OH, $-NH_2$, -O-alkyle $C_1$ à $C_{20}$, -NH-alkyle $C_1$ à $C_{20}$, -N(alkyle $C_1$ à $C_{20})_2$, $-NO_2$, -CN, - F, -Cl, -Br, et -I ;
dans lequel le groupe $R^3$ est sélectionné indépendamment parmi : des groupes alkyle $C_1$ à $C_6$, alcényle $C_1$ à $C_6$, alcoxy $C_1$ à $C_6$, alkyloxy $C_1$ à $C_6$, -C(O)OH, -C(O)O-alkyle $C_1$ à $C_6$, alkyle $C_1$ à $C_6$-(O)O-H, alkyle $C_1$ à $C_6$-(O)O-alkyle $C_1$ à $C_6$, et -OH ;
dans lequel le groupe $R^4$ est un atome d'hydrogène ;
dans lequel le groupe $R^6$ est indépendamment un groupe monovalent sélectionné parmi -H, -OH, ou un groupe hydrocarbyle comprenant 1 à 50 atomes de carbone ;
dans lequel z vaut 0 ou 1 ;
dans lequel q = 2 - z ; et
dans lequel n vaut indépendamment 0, 1, 2 ou 3.

**6.** Composé selon l'une quelconque des revendications précédentes, dans lequel :

1) chaque groupe $R^3$ est sélectionné indépendamment parmi : des groupes alkyle $C_1$ à $C_6$, alcoxy $C_1$ à $C_6$, alkyloxy $C_1$ à $C_6$, et -OH, préférablement $-CH_3$, $-CH_2OH$, ou $-CH_2OCH_3$ ;
2) toutes les valeurs n sont ≤ 1, et préférablement dans lequel toutes les valeurs n sont identiques, et/ou dans lequel tous les groupes $R^3$ sont identiques, et/ou dans lequel tous les groupes $R^3$ sont fixés en position ortho par rapport à l'atome d'oxygène du furane ; plus préférablement dans lequel toutes les valeurs n = 0 ; et/ou
3) chaque groupe R est sélectionné indépendamment parmi : des groupes alkyle $C_1$ à $C_{20}$, cycloalkyle $C_3$ à $C_{20}$, hétérocycloalkyle $C_3$ à $C_{20}$, alcényle $C_2$ à $C_{20}$, alcynyle $C_2$ à $C_{20}$, alkyle $C_1$ à $C_{20}$-aryle $C_6$ à $C_{14}$, alkyle $C_1$ à $C_{20}$-hétéroaryle $C_2$ à $C_9$, alkyle $C_1$ à $C_{20}$-cycloalkyle $C_3$ à $C_{10}$, alkyle $C_1$ à $C_{20}$-hétérocycloalkyle $C_3$ à $C_{10}$, alkyloxy $C_1$ à $C_{20}$, alkylamino $C_4$ à $C_{20}$, -OH, $-OR^1$, $-NH_2$, $-NHR^1$, $-NR^1_2$, -O(CO)H, $-O(CO)R^1$, -NH(CO)H, - $NH(CO)R^1$, $-NR^1(CO)H$, $-NR^1(CO)R^1$, -SH, $-SR^1$, $-SiR^1_3$, -F, -Cl, -Br, -I, aryle $C_6$ à $C_{14}$ optionnellement substitué par un ou plusieurs groupes $R^2$, et hétéroaryle $C_2$ à $C_9$ optionnellement substitué par un ou plusieurs groupes $R^2$ ;
dans lequel le groupe $R^1$ est sélectionné indépendamment parmi : des groupes alkyle $C_1$ à $C_{20}$, cycloalkyle $C_3$ à $C_{20}$, hétérocycloalkyle $C_3$ à $C_{20}$, alcényle $C_2$ à $C_{20}$, alcynyle $C_2$ à $C_{20}$, alkyle $C_1$ à $C_{20}$-aryle $C_6$ à $C_{14}$, alkyle $C_1$ à $C_{20}$-hétéroaryle $C_2$ à $C_9$, alkyle $C_1$ à $C_{20}$-cycloalkyle $C_3$ à $C_{20}$, alkyle $C_1$ à $C_{20}$-hétérocycloalkyle $C_3$ à $C_{10}$, alcoxy $C_1$ à $C_{20}$, alkylamino $C_4$ à $C_{20}$, aryle $C_6$ à $C_{14}$, et hétéroaryle $C_2$ à $C_9$ ; et
dans lequel le groupe $R^2$ est sélectionné indépendamment parmi : des groupes alkyle $C_1$ à $C_{20}$, cycloalkyle $C_3$ à $C_{20}$, alcényle $C_2$ à $C_{20}$, alcynyle $C_2$ à $C_{20}$, -OH, $-NH_2$, -O-alkyle $C_1$ à $C_{20}$, -NH-alkyle $C_1$ à $C_{20}$, -N(alkyle $C_1$ à $C_{20})_2$, -F, -Cl, -Br, ou -I ;
préférablement dans lequel chaque groupe R est sélectionné indépendamment parmi : des groupes alkyle $C_1$ à $C_{20}$, cycloalkyle $C_3$ à $C_{20}$, hétérocycloalkyle $C_3$ à $C_{20}$, alcényle $C_2$ à $C_{20}$, alcynyle $C_2$ à $C_{20}$, alkyloxy $C_1$ à $C_{20}$, alkylamino $C_4$ à $C_{20}$, -OH, $-OR^1$, - $NH_2$, $-NHR^1$, $-NR^1_2$, aryle $C_6$ à $C_{14}$ optionnellement substitué par un ou plusieurs groupes $R^2$, ou hétéroaryle $C_2$ à $C_9$ optionnellement substitué par un ou plusieurs groupes $R^2$ ; plus préférablement -OH ;
dans lequel le groupe $R^1$ est sélectionné indépendamment parmi : des groupes alkyle $C_1$ à $C_{20}$, cycloalkyle $C_3$ à $C_{20}$, ou alcényle $C_2$ à $C_{20}$,
dans lequel le groupe $R^2$ est sélectionné indépendamment parmi : des groupes alkyle $C_1$ à $C_{20}$, -OH, $-NH_2$, -O-alkyle $C_1$ à $C_{20}$, -NH-alkyle $C_1$ à $C_{20}$, ou -N(alkyle $C_1$ à $C_{20})_2$, plus préférablement -OH.

**7.** Composé selon l'une quelconque des revendications 3 à 6, dans lequel le groupe $R^6$ est sélectionné indépendamment parmi : des groupes -H, -OH, alkyle $C_1$ à $C_{20}$, cycloalkyle $C_3$ à $C_{20}$, hétérocycloalkyle $C_3$ à $C_{20}$, alcényle $C_2$ à $C_{20}$, alcynyle $C_2$ à $C_{20}$, alkyloxy $C_1$ à $C_{20}$, alkylamino $C_4$ à $C_{20}$, aryle $C_6$ à $C_{14}$, et hétéroaryle $C_2$ à $C_9$ ; dans lequel le groupe $R^6$ est optionnellement substitué par un ou plusieurs groupes $R^7$ ;
dans lequel le groupe $R^7$ est sélectionné indépendamment parmi : des groupes alkyle $C_1$ à $C_{20}$, haloalkyle $C_1$ à $C_{20}$, cycloalkyle $C_3$ à $C_{20}$, hétérocycloalkyle $C_3$ à $C_{20}$, alcényle $C_2$ à $C_{20}$, alcynyle $C_2$ à $C_{20}$, alkyloxy $C_1$ à $C_{20}$, alkylamino $C_4$ à $C_{20}$, -OH, $-OR^1$, - $NH_2$, $-NHR^1$, $-NR^1_2$, -C(O)OH, $-C(O)OR^1$, $-C(O)NH_2$, $-C(O)NHR^1$, $-C(O)NR^1_2$, -O(CO)H, - $O(CO)R^1$, -NH(CO)H, $-NH(CO)R^1$, $-NR^1(CO)H$, $-NR^1(CO)R^1$, -SH, $-SR^1$, $-SO_2H$, $-SO_2R^1$, - $SO_3R^1$, $-SO_3H$, $-SiR^1_3$, $-NO_2$, -CN, -F, -Cl, -Br, -I, aryle $C_6$ à $C_{14}$, et hétéroaryle $C_2$ à $C_9$.

**8.** Procédé de préparation d'un composé ayant la formule (I) selon la revendication 1 ou la revendication 2, ledit procédé comprenant :

i) la fourniture d'une diamine $NH_2-L^1-NH_2$ dans laquelle le groupe $L^1$ est tel que défini dans la revendication 1 ou la revendication 2 ;
ii) l'ajout d'au moins un furfuraldéhyde substitué par n groupes $R^3$, et d'au moins un composé phénolique substitué par x groupes R, R, $R^3$, n et x étant tels que définis dans la revendication 1 ou la revendication 2 et la revendication 6, à condition que les deux positions ortho par rapport au groupe OH phénolique soient non substituées sur l'au moins un composé phénolique ; et
iii) la formation d'un composé ayant la formule (I).

**9.** Procédé de préparation d'un composé ayant la formule (II), (IIIa) ou (IIIb) selon l'une quelconque des revendications 3 à 7, ledit procédé comprenant :

i) la fourniture : A) d'un composé de bisphénol comprenant un lieur $L^2$ et substitué par y groupes R sur chaque cycle benzénique du composé de bisphénol ; B) d'un composé de benzène-1,4-diol substitué par z groupes R ; ou C) d'un benzène-1,3-diol optionnellement substitué par 1 groupe R en position 5 ; y, z, R et $L^2$ étant tels que définis dans l'une quelconque des revendications 3 à 7 ;
ii) la mise en contact du composé issu de l'étape i) avec au moins un furfuraldéhyde substitué par n groupes $R^3$, et au moins une amine primaire substituée par un groupe $R^6$, n, $R^3$ et $R^6$ étant tels que définis dans l'une quelconque des revendications 3 à 7 ; et
iii) la formation d'un composé ayant la formule (II), (IIIa) ou (IIIb).

**10.** Procédé selon la revendication 8 ou la revendication 9, dans lequel le rapport molaire de la diamine, du composé de bisphénol ou du benzènediol à l'au moins un furfuraldéhyde est de 1:2 à 1:20, préférablement de 1:3 à 1:10, plus préférablement de 1:4 à 1:8 ; et/ou dans lequel le rapport molaire du composé de bisphénol ou du benzènediol à l'au moins une amine primaire, ou le rapport molaire de la diamine à l'au moins un composé phénolique, est de 1:1 à 1:5, préférablement de 1:1,5 à 1:4, plus préférablement de 1:2 à 1:3.

**11.** Procédé selon l'une quelconque des revendications 8 à 10, dans lequel :

I) la diamine ou l'au moins une amine primaire est mise en contact avec l'au moins un furfuraldéhyde avant l'ajout de l'un ou l'autre de l'au moins un composé phénolique, ou du composé de bisphénol ou du benzènediol, respectivement ;
II) la diamine ou l'au moins une amine primaire est mise en contact avec une partie de l'au moins un furfuraldéhyde avant l'ajout de l'un ou l'autre de l'au moins un composé phénolique, ou du composé de bisphénol ou du benzènediol, respectivement, le furfuraldéhyde restant étant ajouté après et/ou simultanément à l'ajout de l'un ou l'autre de l'au moins un composé phénolique, ou du composé de bisphénol ou du benzènediol, respectivement ; ou
III) la diamine ou l'au moins une amine primaire est mise en contact avec l'un ou l'autre de l'au moins un composé phénolique, ou du composé de bisphénol ou du benzènediol, respectivement, et l'au moins un furfuraldéhyde simultanément ;

et préférablement dans lequel, dans I), II) ou III), deux différents composés phénoliques ou deux différentes amines primaires sont ajouté(e)s séparément ; ou dans lequel un seul composé phénolique ou une seule amine primaire est ajouté(e).

**12.** Composition de résine comprenant le composé selon l'une quelconque des revendications 1 à 7.

**13.** Utilisation d'un composé selon l'une quelconque des revendications 1 à 7 ou d'une composition de résine selon la revendication 12 pour renforcer un matériau, préférablement un matériau fibreux, plus préférablement de la fibre de verre, de la fibre de carbone ou du lin ; et optionnellement dans laquelle un catalyseur ou un coréactif est utilisé pour réduire la température d'activation d'une réaction de polymérisation du composé selon l'une quelconque des revendications 1 à 7, préférablement dans laquelle le catalyseur est du chlorure ferrique.

**14.** Procédé de préparation d'un matériau renforcé, ledit procédé comprenant :

a) la mise en contact d'un matériau à renforcer avec un composé tel que défini dans l'une quelconque des revendications 1 à 7, ou avec une composition de résine telle que définie dans la revendication 12 ; et
b) le fait de laisser ledit composé s'autopolymériser pour former un matériau renforcé, préférablement dans lequel le matériau à renforcer est un matériau fibreux, plus préférablement de la fibre de verre, de la fibre de

carbone ou du lin.

15. Matériau renforcé préparé, ou pouvant être préparé, par le procédé selon la revendication 14.

Figure 1

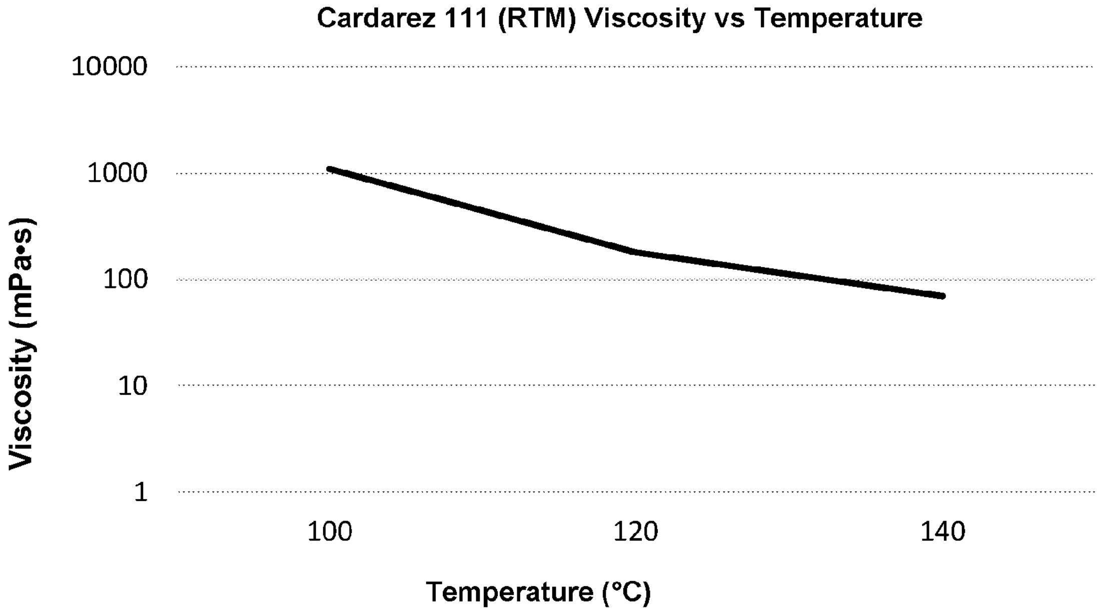


Figure 2

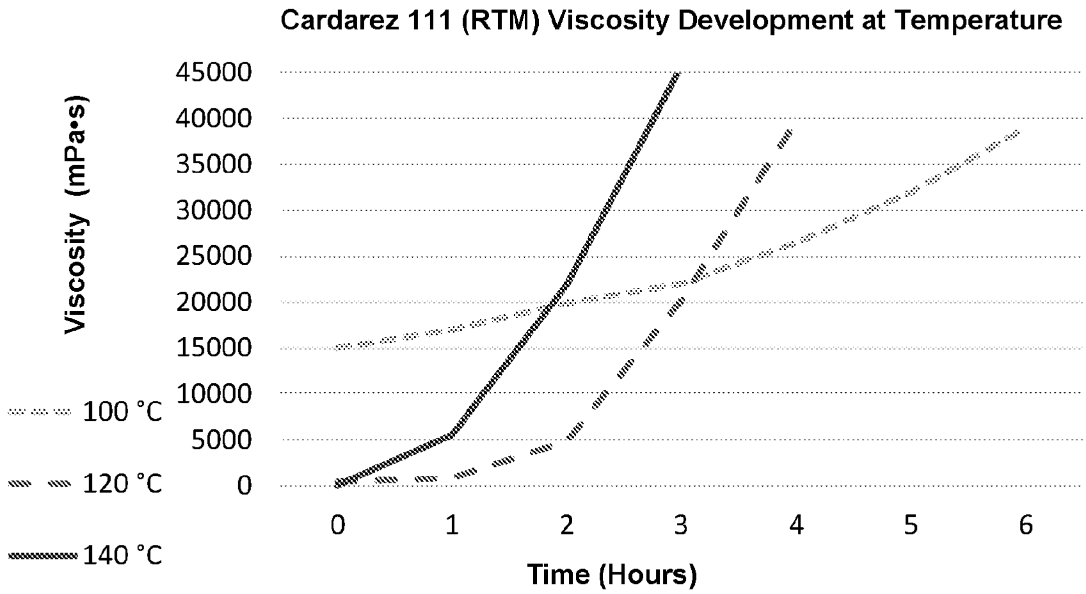

Figure 3

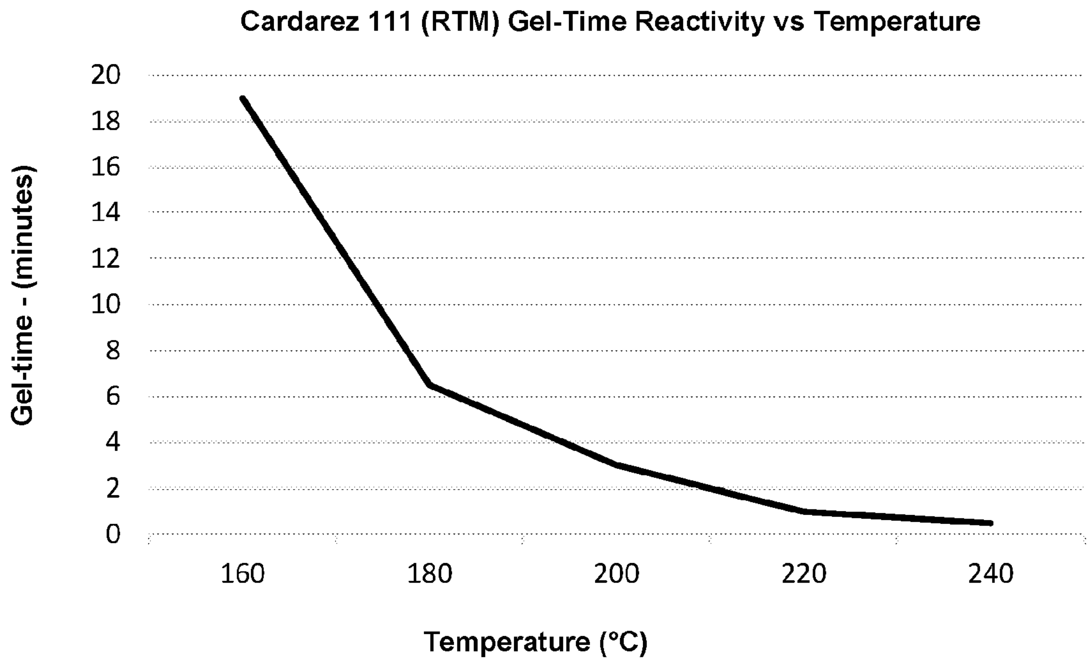


Figure 4

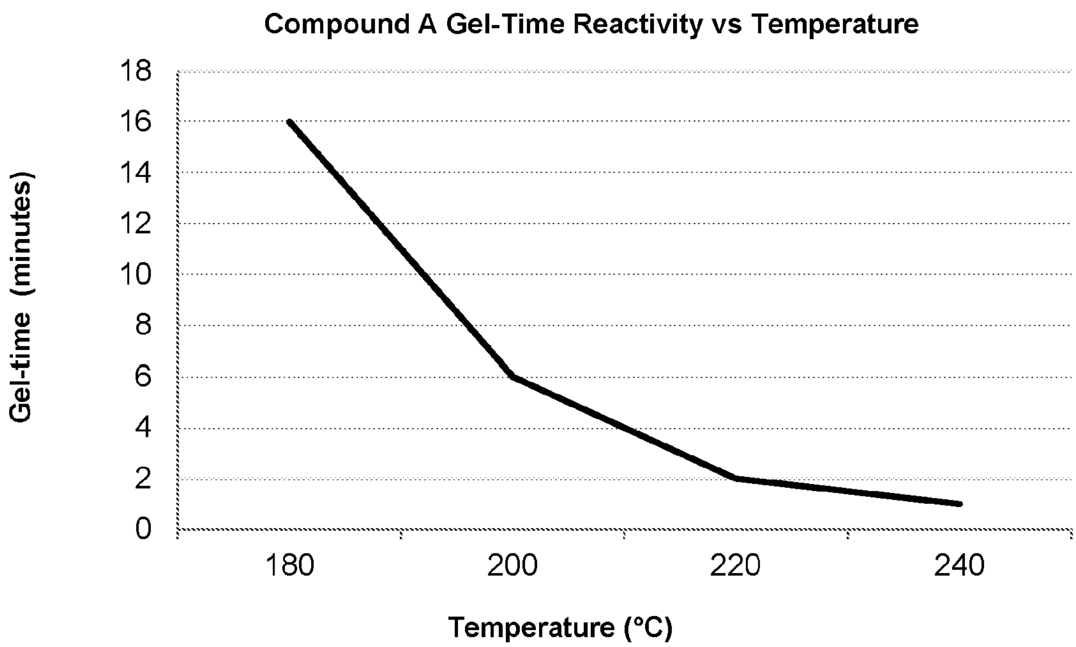

Figure 5

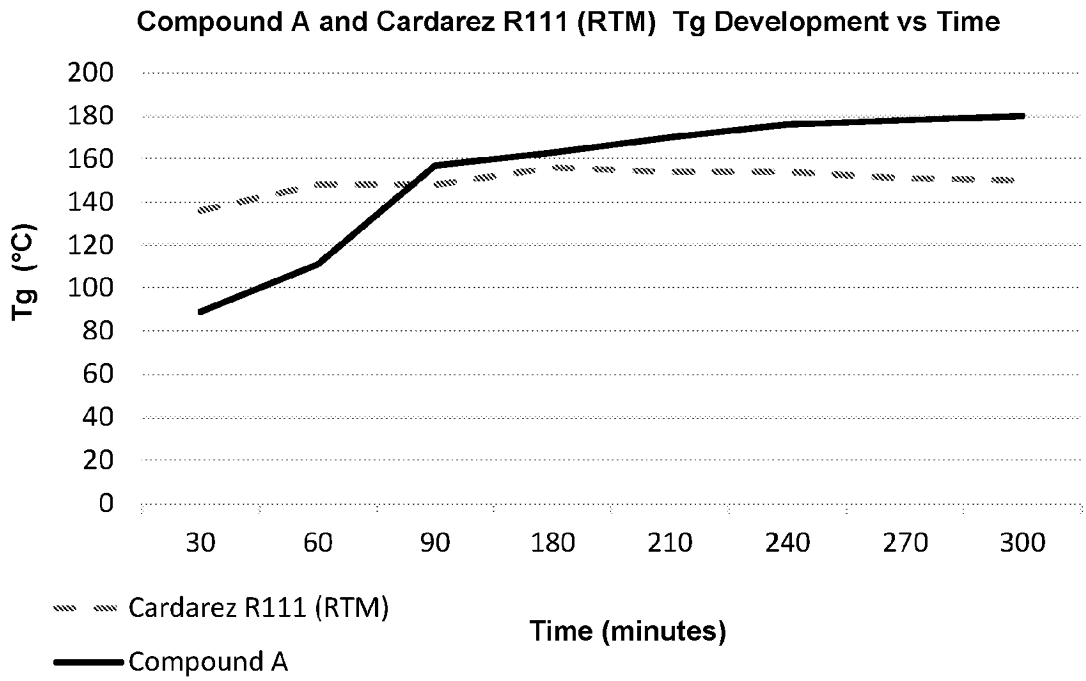


Figure 6

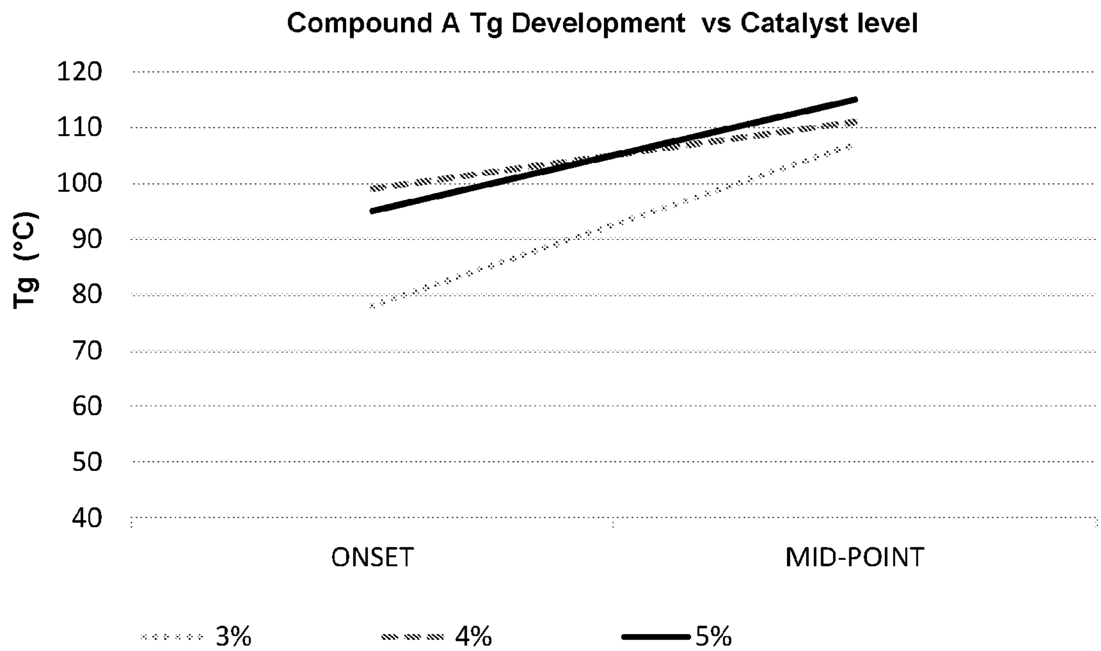

Figure 7

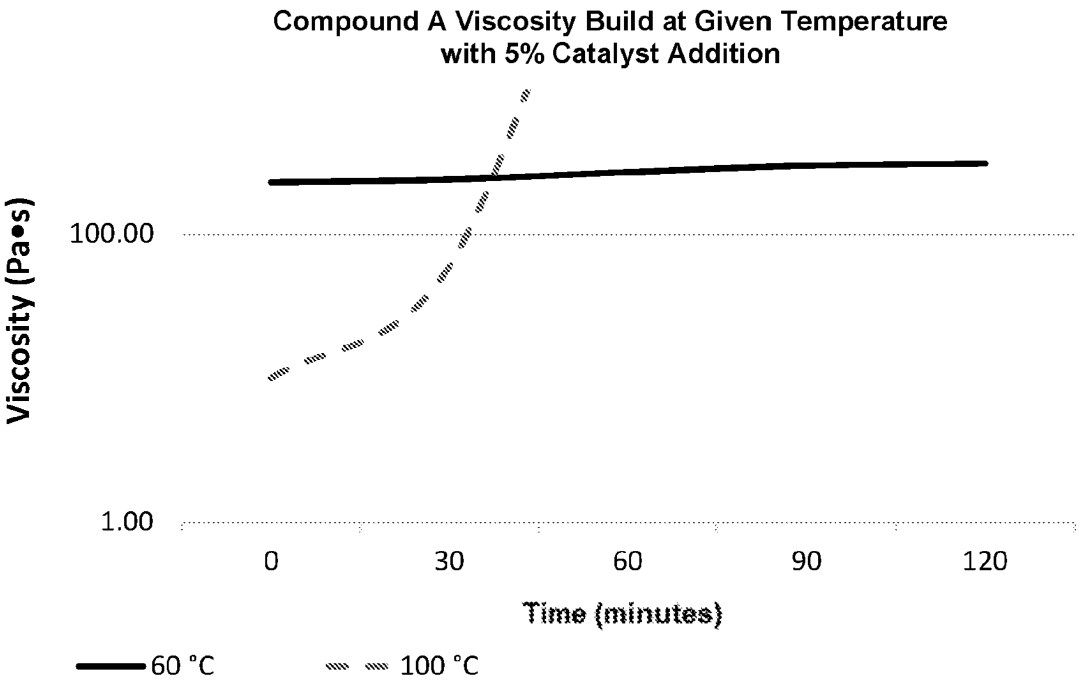


Figure 8

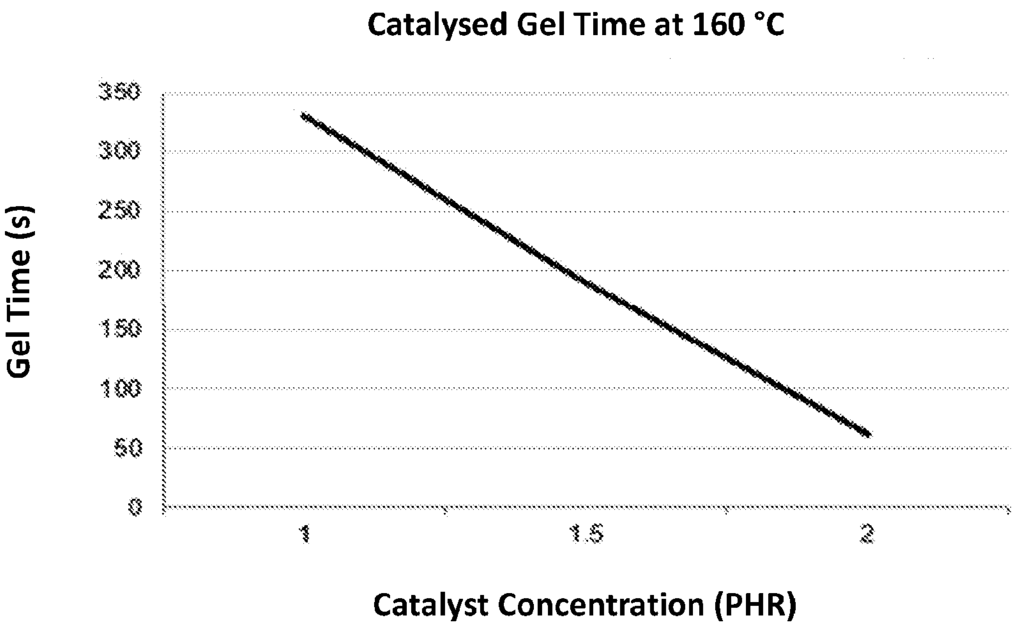

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019040407 A
- CN 106674214


### Non-patent literature cited in the description


- **M. S. AL AJELY** ; **A M NOORI**. Synthesis of New Oxazin Compounds Derived from Furfural. Chalcons and Schiff base. *LOJ pharmacol.*, 2019, vol. 1 (3), 66-71
- **S. A. OZTURKCAN** ; **T ZUHAL**. Synthesis and Charecterizations of New 1,3-Oxazine Derivatives. *J. Chem. Soc. Pak.*, 2011, vol. 33 (6), 939-944
- **D. R. DE OLIVEIRA et al.** ynthesis and Polymerization of Naphthoxazines Containing Furan Groups: An Approach to Novel Biobased and Flame-Resistant Thermosets,. *Int. J. Polym. Sci.*, 2018, vol. 2018, 1-13
- **HANSCH. C** ; **LEO. A**. Substituent constants for correlation analysis in chemistry and biology,. Wiley, 1979
- **HAMMETT. P**. The Effect of Structure upon the Reactions of Organic Compounds. Benzene Derivatives. *J. Am. Chem. Soc.*, 1937, vol. 59 (1), 96-103
- **HANSCH. C et al.** A Survey of Hammett Substituent Constants and Resonance and Field Parameters,. *Chem. Rev.*, 1991, vol. 91, 165-195
- **HANSCH. C et al.** A Survey of Hammett Substituent Constants and Resonance and Field Parameters. *Chem. Rev.*, 1991, vol. 91, 165-195
- **HANSCH. C** ; **LEO. A**. Substituent constants for correlation analysis in chemistry and biology. Wiley, 1979
- *ASTM D3056-14*, 2018